# EUROPEAN PATENT SPECIFICATION

(11) **EP 4 536 684 B1**
(45) Date of publication and mention of the grant of the patent: **02.12.2026**
(21) Application number: 23744305.6
(22) Date of filing: 13.06.2023
(51) Int. Cl.: C07K 7/64, A61P 17/00, A61P 37/00, A61K 38/00

(54) **INTERLEUKIN-13 BINDING CYCLIC OLIGOPEPTIDES AND METHODS OF USE THEREOF**
INTERLEUKIN-13-BINDENDE CYCLISCHE OLIGOPEPTIDE UND VERFAHREN ZUR VERWENDUNG DAVON
OLIGOPEPTIDES CYCLIQUES SE LIANT À L'INTERLEUKINE-13 ET LEURS PROCÉDÉS D'UTILISATION

(30) Priority: 13.06.2022 US 202263351712 P; 24.10.2022 US 202263418715 P
(43) Date of publication of application: 16.04.2025
(73) Proprietor: B.A.I. Laboratories, LLC, Cambridge, MA 02142 (US)
(72) Inventor: BAI, Xuefei, Cambridge, Massachusetts 02142 (US); LIN, Sheng, Beijing 100190 (CN); LI, Le, Beijing 100190 (CN)
(74) Representative: Cohausz & Florack
(86) International application number: PCT/US2023/068376
(87) International publication number: WO 2023/245016

(56) References cited:
- WO-A1-2005/014646
- WO-A1-2010/057275
- JP-A- 2018 172 296
- US-A1- 2021 301 010
- DONG NA ET AL: "Simplified Head-to-Tail Cyclic Polypeptides as Biomaterial-Associated Antimicrobials with Endotoxin Neutralizing and Anti-Inflammatory Capabilities", INTERNATIONAL JOURNAL OF MOLECULAR SCIENCES, vol. 20, no. 23, 25 November 2019 (2019-11-25), pages 5904, XP093077947, Retrieved from the Internet <URL:https://www.ncbi.nlm.nih.gov/pmc/articles/PMC6928678/pdf/ijms-20-05904.pdf> DOI: 10.3390/ijms20235904

## Description

### BACKGROUND OF THE DISCLOSURE

Interleukin-13 (IL-13) is one of the key cytokines involved in the type 2 (T2) immune response. A recent study has shown that it plays an important role driving inflammation in the periphery. IL-13 is secreted by T lymphocytes and mast cells and has been identified as the primary cytokine involved in atopic dermatitis (AD) inflammation, as evidenced by local overexpression of IL-13. IL-13 impacts skin biology through the recruitment of inflammatory cells, the alteration of the skin microbiome and the decrease in the epidermal barrier function. Alopecia Areata (AA) is an autoimmune disorder that shares phenotypic similarities with AD, such as pruritus, elevated IgE levels, and filaggrin mutations. A genomic study in patients with AA identified IL-13 gene susceptibility. In addition, lesional scalps from AA patients demonstrated increased IL-13 mRNA as compared to nonlesional scalps. Moreover, AA patients demonstrated significantly elevated IL-13 levels in serum as compared with healthy control group.

IL-13's significant impact on the skin immune response and barrier function is partially through its ability to activate of STAT6, resulting in decreased expression of key structural proteins, such as filaggrin, filaggrin 2, loricrin, involucrin, keratin 1, keratin 10, hornerin, desmoglein, and desmocollin1, as well as the lipid compositions of skin. These changes contribute to the increased transepidermal water loss (TEWL), which is a hallmark of AD. IL-13 is also reported to decrease the production of AMP by keratinocytes and thereby play a role in the dysbiosis of the skin, characterized by a strong colonization with *Staphylococcus aureus,* which has been shown to precede the appearance of AD lesions.

In view of IL-13's role in the generation and maintenance of the inflammatory reaction and its significant impact on epidermal barrier function, pharmacological approaches targeting IL-13-induced pathways are in development. Such approaches include small molecules interfering with interactions between IL-13 and its receptor; small molecules interfering with intracellular signaling pathways, such as kinase inhibitors; and biologics, such as monoclonal antibodies, blocking the binding of IL-13 to its receptor sites. For example, tralokinumab is a fully human IgG4 antibody that binds to IL-13 at an epitope that overlaps with the binding site of the IL-13Rα receptors, preventing IL-13 from binding to both IL-13Rα1 and IL-13Rα2.

However, each of these approaches has significant limitations in their application to treat skin inflammatory conditions and diseases, such as AD and AA. For example, current treatment of atopic dermatitis with topical corticosteroid may result in undesired side effects such atrophy or thinning of skin, stria or stretch marks, easy bruising, and telangiectasia. Continuous treatment with topical steroid may also result in withdrawal or rebound effects once the treatment ceases, symptoms include, for example, skin burning, itching, and peeling. In addition, existing antibody treatment for AD, such as tralokinumab, is administered by injection. DONG NA ET AL: "Simplified Head-to-Tail Cyclic Polypeptides as Biomaterial-Associated Antimicrobials with Endotoxin Neutralizing and Anti-Inflammatory Capabilities", INTERNATIONAL JOURNAL OF MOLECULAR SCIENCES, vol. 20, no. 23 25. November 2019, page 5904, disclose head-to-tail cyclic D-proline antimicrobial peptides using the template (IR)ₙP(IR)ₙP (n= 1, 2 and 3). An LPS-induced inflammation assay showed that cyclic tetradecapeptide OIR3 (cyclo-(IR)₃P(IR)₃P) inhibited the pro-inflammatory factor TNF-a in RAW264.7 cells. OIR3 was able to reduce oxazolone-induced skin inflammation in allergic dermatitis mause model via the inhibition of TNF-a, IL-1β and IL-6 mRNA expression. JP 2018 172296 A discloses the cyclic tetrapeptides Tyr-Pro-Pro-Glu and Glu-Pro-Pro-Tyr, which are stated to be useful in pharmaceutical compositions and for the treatment of atopic dermatitis. WO 2010/057275 A1 discloses cyclic peptides, which are stated to be useful in pharmaceutical and ointment compositions for the treatment of skin diseases such as dermatitis, eczema or urticaria. Accordingly, there remains a need for therapeutics targeting IL-13 for treating skin diseases and conditions, especially those that are suitable for topical application.

### SUMMARY OF THE DISCLOSURE

The present disclosure provides cyclic oligopeptides that bind IL-13 and methods of using such cyclic oligopeptides to treat IL-13-associated skin disorders or conditions.

A first aspect of the present disclosure provides a cyclic oligopeptide that binds to IL-13. In some embodiments, the cyclic oligopeptide comprises between two and twenty-two amino acid residues. In some embodiments, the cyclic oligopeptide comprises between six and eight amino acid residues. In some embodiments, the cyclic oligopeptide comprises a L-proline residue or a D-proline residue.

According to the invention, the cyclic oligopeptide comprises an amino acid sequence selected from the group consisting of SEQ ID NOs:1-11. In some embodiments, the cyclic oligopeptide comprises the amino acid sequence of D-ARG D-PHE D-VAL TYR GLU PRO (SEQ ID NO:1). Optionally, the cyclic oligopeptide comprises the amino acid sequence of ARG THR D-VAL GLU D-PHE D-PRO (SEQ ID NO:2). The cyclic oligopeptide may comprise the amino acid sequence of GLU D-THR D-VAL TRP D-PRO D-PRO (SEQ ID NO:3). In some embodiments, the cyclic oligopeptide comprises the amino acid sequence of ARG GLU D-VAL TRP D-PRO D-PRO (SEQ ID NO:4). Optionally, the cyclic oligopeptide comprises the amino acid sequence of TRP VAL ARG GLU D-PRO D-PRO (SEQ ID NO:5). The cyclic oligopeptide may comprise the amino acid sequence of TYR ARG GLU D-THR D-VAL D-PRO (SEQ ID NO:6). In some embodiments, the cyclic oligopeptide comprises the amino acid sequence of THR ARG D-PHE PRO D-LEU D-PRO (SEQ ID NO:7). Optionally, the cyclic oligopeptide comprises the amino acid sequence of D-LEU ARG GLU PRO TRP MET PRO (SEQ ID NO:8). The cyclic oligopeptide may comprise the amino acid sequence of D-ARG LEU D-TRP TRP D-THR GLU PRO (SEQ ID NO:9). In some embodiments, the cyclic oligopeptide comprises the amino acid sequence of ARG ASP TYR CYS D-PRO D-TRP D-PRO (SEQ ID NO:10). Optionally, the cyclic oligopeptide comprises the amino acid sequence of VAL PRO D-LEU D-TRP D-VAL LEU ARG PRO (SEQ ID NO:11).

In some embodiments, the cyclic oligopeptide has the structure of any one of Compounds 1-11, as defined herein. In some embodiments, the cyclic oligopeptide has the structure of Compound 1, as defined herein. Optionally, the cyclic oligopeptide has the structure of Compound 2, as defined herein. The cyclic oligopeptide may have the structure of Compound 3, as defined herein. In some embodiments, the cyclic oligopeptide has the structure of Compound 4, as defined herein. Optionally, the cyclic oligopeptide has the structure of Compound 5, as defined herein. The cyclic oligopeptide may have the structure of Compound 6, as defined herein. In some embodiments, the cyclic oligopeptide has the structure of Compound 7, as defined herein. Optionally, the cyclic oligopeptide has the structure of Compound 8, as defined herein. The cyclic oligopeptide may have the structure of Compound 9, as defined herein. In some embodiments, the cyclic oligopeptide has the structure of Compound 10, as defined herein. Optionally, the cyclic oligopeptide has the structure of Compound 11, as defined herein.

In some embodiments, the cyclic oligopeptide is lipidated. In some embodiments, the cyclic oligopeptide is PEGylated.

A second aspect of the present disclosure provides a pharmaceutical formulation comprising a cyclic oligopeptide disclosed herein and a pharmaceutically acceptable carrier.

The references to methods of treatment in the subsequent paragraphs of this description are to be interpreted as references to the compounds, pharmaceutical compositions and medicaments of the present invention for use in a method for treatment of the human (or animal) body by therapy. A third aspect of the present disclosure provides a method of treating or preventing an IL-13-associated skin disorder or condition in a subject in need thereof. In some embodiments, the method comprises administering to the subject a therapeutically effective amount of any one of the cyclic oligopeptides disclosed herein or a combination thereof. In some embodiments, the subject is a human.

In some embodiments, the IL 13 associated skin disorder or condition is an inflammatory, an allergic, or an autoimmune disorder or condition. In some embodiments, the IL 13 associated skin disorder or condition is selected from the group consisting of: atopic dermatitis, allergic contact dermatitis, urticaria, eczema, chronic hand eczema, bullous diseases (bullous pemphigoid), alopecia areata, prurigo and molluscum contagiosum. Optionally, the IL-13-associated skin disorder or condition is atopic dermatitis. The IL-13-associated skin disorder or condition may be allergic contact dermatitis. In some embodiments, the IL-13-associated skin disorder or condition is urticaria. Optionally, the IL-13-associated skin disorder or condition is eczema. The IL-13-associated skin disorder or condition may be chronic hand eczema. In some embodiments, the IL-13-associated skin disorder or condition is bullous diseases (bullous pemphigoid). Optionally, the IL-13-associated skin disorder or condition is alopecia areata. The IL-13-associated skin disorder or condition may be prurigo. In some embodiments, the IL-13-associated skin disorder or condition is molluscum contagiosum.

In some embodiments, the cyclic oligopeptide or combination thereof binds to IL-13 and reduces biological activities of IL-13 and/or an IL-13 receptor (IL-13R). In some embodiments, the cyclic oligopeptide or combination thereof binds to IL-13. In some embodiments, the cyclic oligopeptide or combination thereof reduces biological activities of IL-13 and/or an IL-13 receptor (IL-13R). In some embodiments, the cyclic oligopeptide or combination thereof reduces biological activities of IL-13. In some embodiments, the cyclic oligopeptide or combination thereof reduces biological activities of an IL-13 receptor (IL-13R). In some embodiments, the cyclic oligopeptide or combination thereof reduces biological activities of IL-13 and an IL-13 receptor (IL-13R).

In some embodiments, the cyclic oligopeptide or the combination thereof is administered topically. Optionally, the cyclic oligopeptide or the combination thereof is administered transdermally. In some embodiments, the cyclic oligopeptide or the combination thereof is administered subcutaneously. Optionally, the cyclic oligopeptide or the combination thereof is administered intravenously. In some embodiments, the cyclic oligopeptide or the combination thereof is administered orally.

A fourth aspect of the present disclosure provides a method of producing a cyclic oligopeptide binds to IL-13. In some embodiments, the method comprises the steps of: (1) contacting FMOC (9-fluorenylmethyloxycarbonyl)-D-Pro-2cl-resin or FMOC-Pro-2cl-resin with a first amino acid residue under conditions suitable to form a peptide bond between the first amino acid residue and the resin-bound D-Pro or Pro to form a resin-bound oligopeptide; (2) washing the resin-bound oligopeptide to remove any unbound amino acid residue; (3) contacting the resin-bound oligopeptide with an additional amino acid residue under conditions suitable to form a peptide bond between the additional amino acid residue and the resin-bound oligopeptide; (4) washing the resin-bound oligopeptide to remove any unbound amino acid residue; (5) removing the resin from the oligopeptide; and (6) cyclizing the oligopeptide.

In some embodiments, the conditions suitable to form a peptide bond in steps (1) and (3) include the addition of O-(Benzotriazol-1-yl)-N, N, N', N'-tetramethyluronium tetrafluoroborate (TBTU). Optionally, the conditions suitable to form a peptide bond in steps (1) and (3) further include the addition of N,N-Dimethylformamide (DMF) and N,N-Diisopropylethylamine (DIEA).

In some embodiments, steps (3) and (4) are repeated between 1 and 20 times before the resin is removed from the oligopeptide.

In some embodiments, the cyclizing step comprises the carboxyl group of the D-Pro or Pro residue forming a covalent bond with one or more amino groups of the linear oligopeptide. In some embodiments, the cyclizing step comprises the addition of DMF, PyBOP (Benzotriazol-1-yl-oxytripyrrolidinophosphonium hexafluorophosphate), HOBT (1-Hydroxybenzotriazole), and DIEA to the linear oligopeptide and incubation of the mixture thereof at room temperature.

### BRIEF DESCRIPTION OF THE DRAWINGS

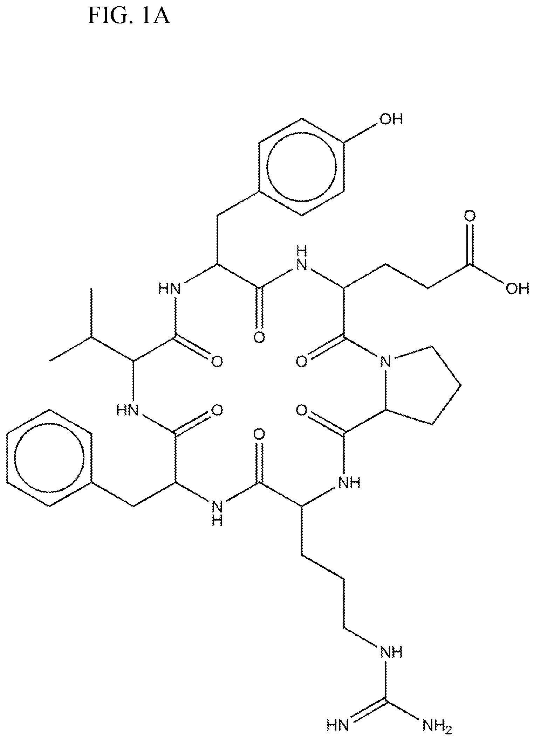

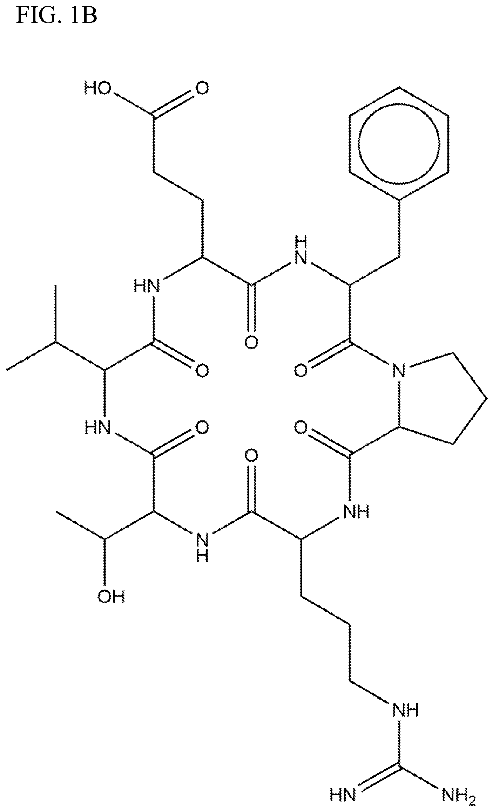

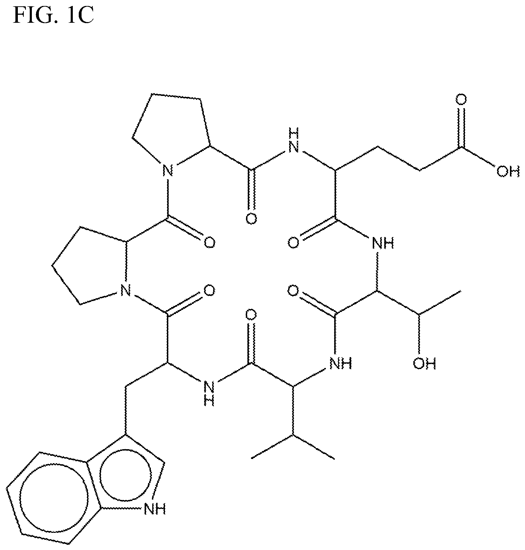

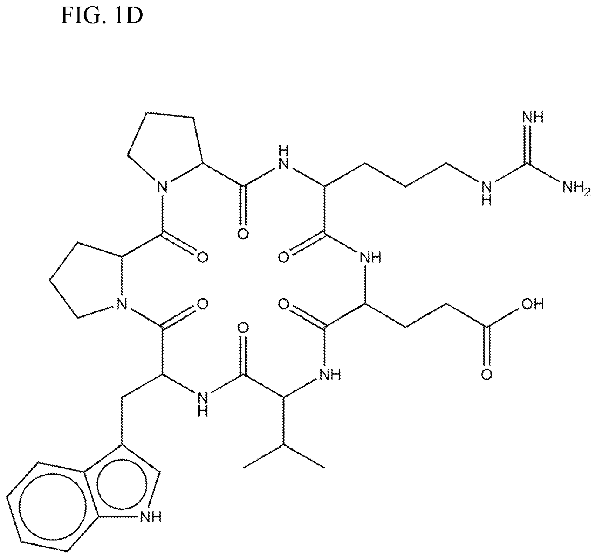

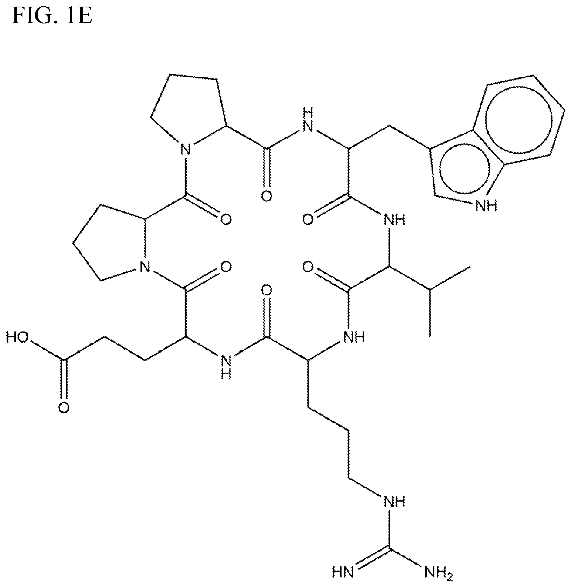

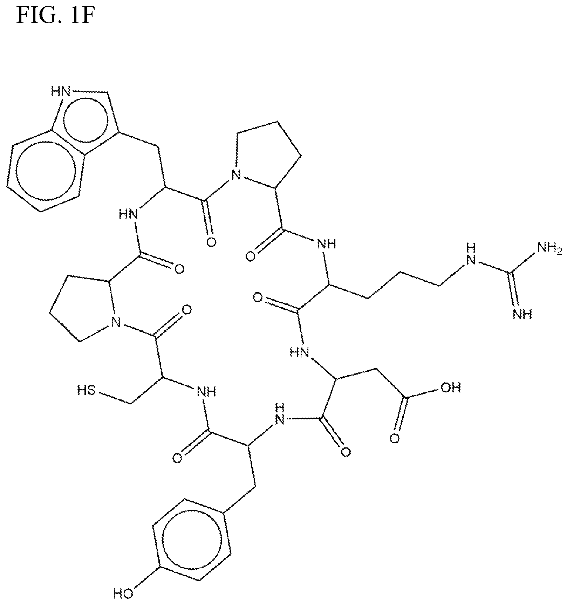

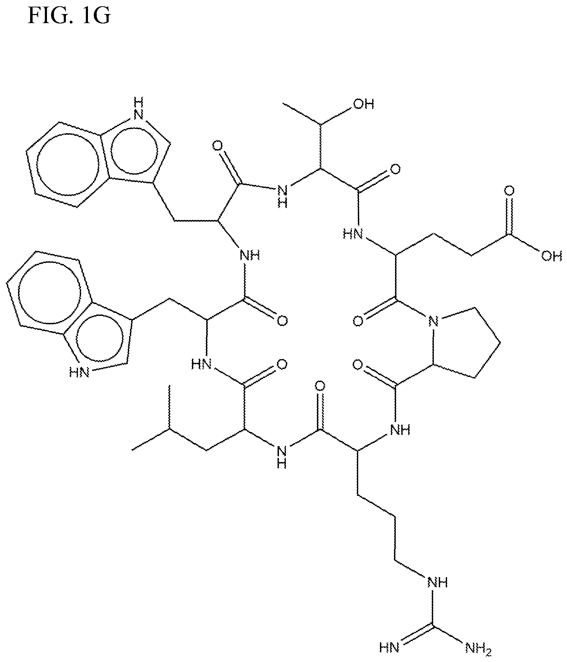

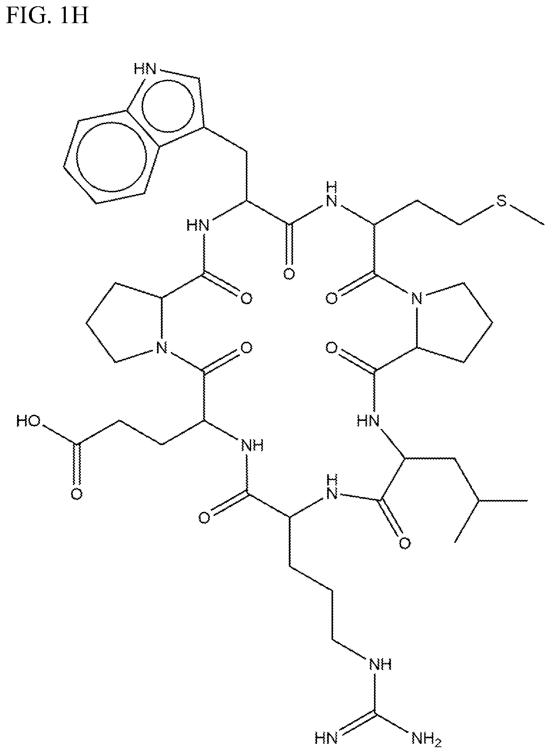

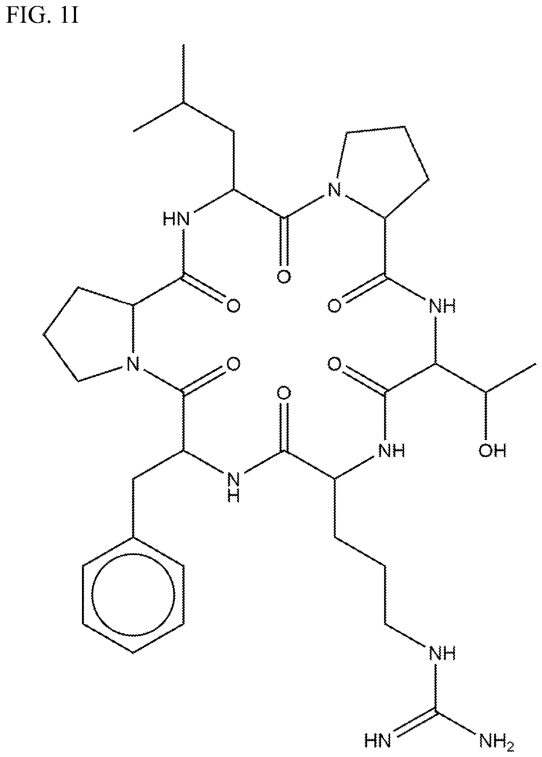

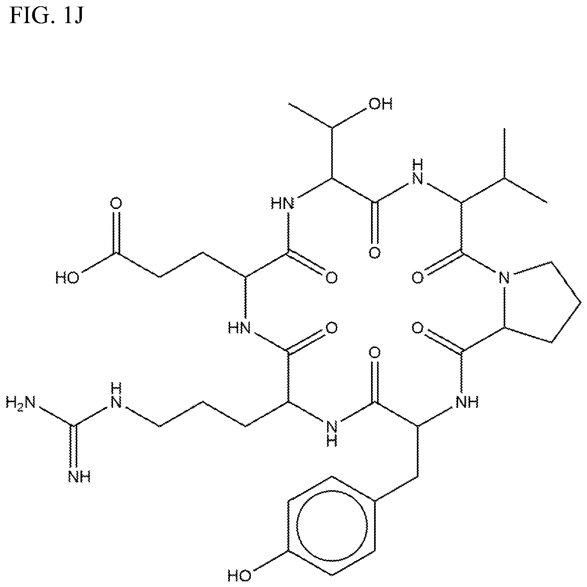

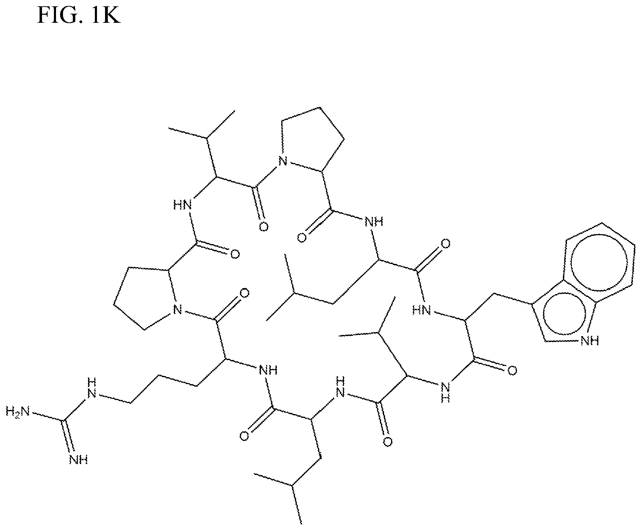

FIG.1A-1K depict chemical structures of exemplary cyclic oligopeptides of this disclosure, Compounds 1-11, respectively.

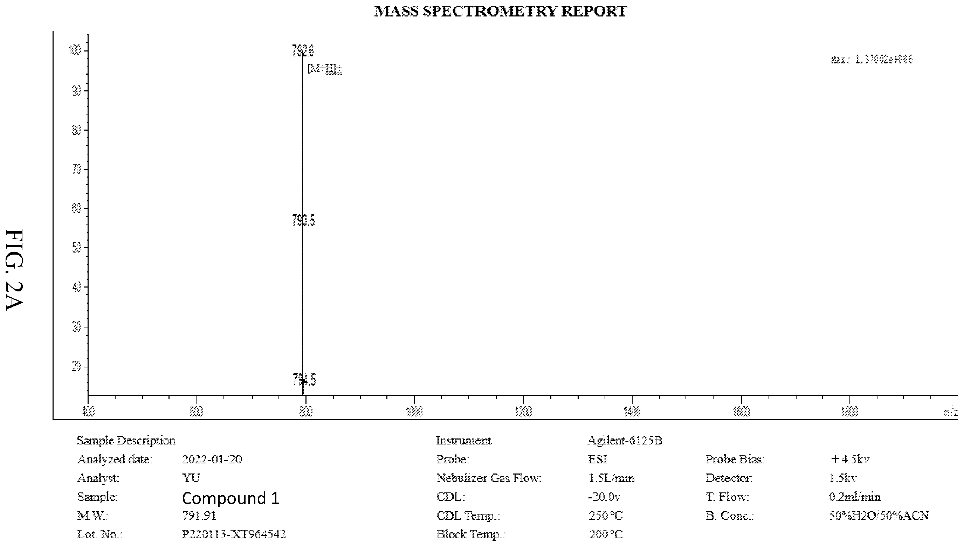

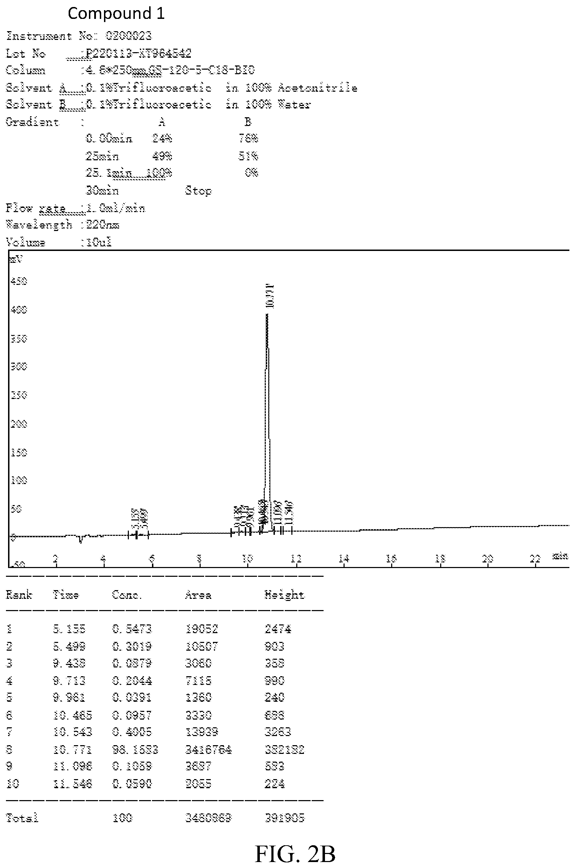

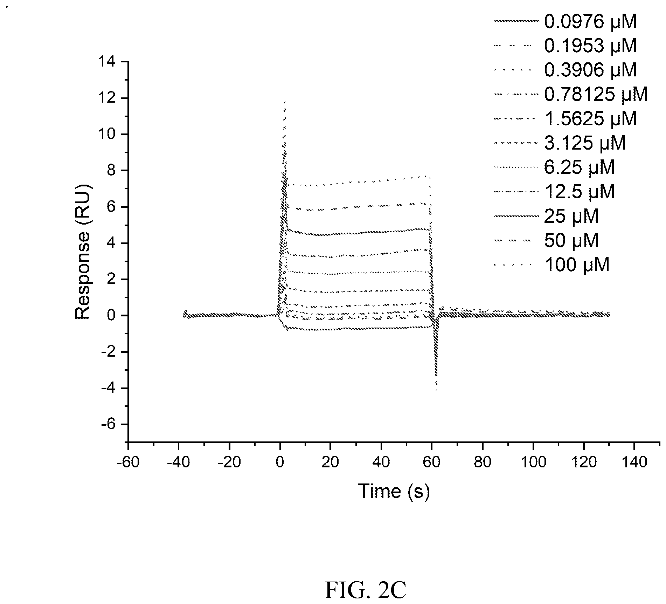

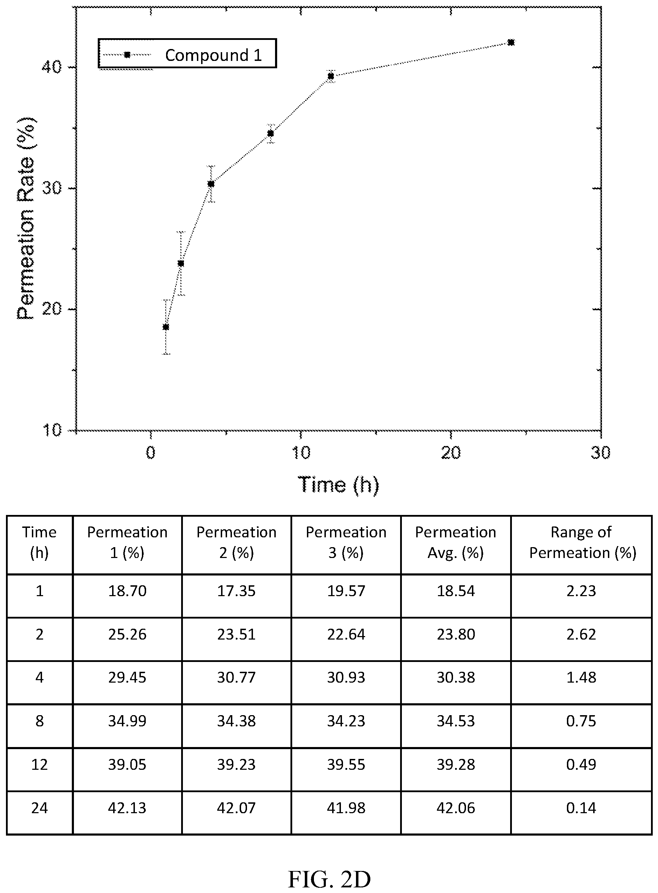

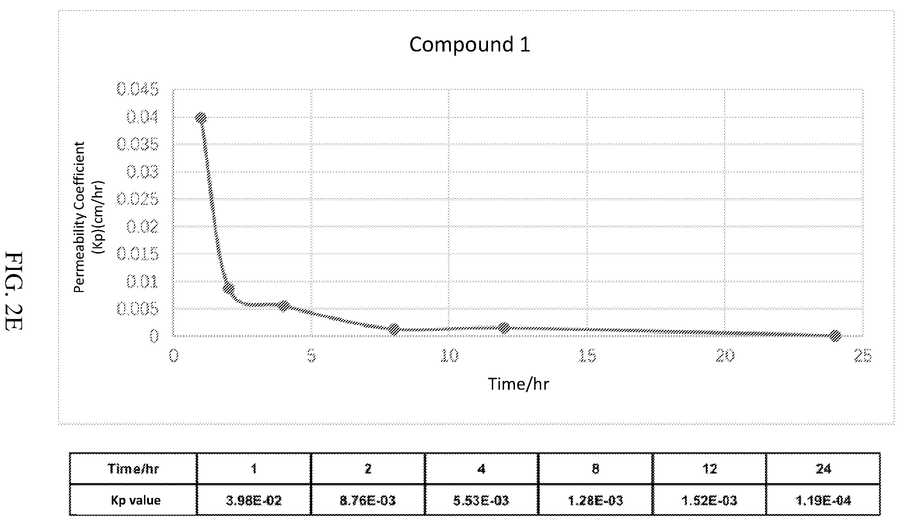

FIG. 2A provides the mass spectrometry (MS) spectrum for Compound 1. FIG. 2B provides the high-performance liquid chromatography (HPLC) chromatogram for Compound 1. FIG. 2C provides surface plasmon resonance (SPR) assay curves showing the binding affinity of Compound 1 for IL-13, the x-axis represents time of reaction and y-axis represents altitude of response (Response Units). FIG. 2D illustrates skin permeation rate of Compound 1 for up to 24 hours. FIG. 2E illustrates average permeability coefficient (Kp) of Compound 1 for 1, 2, 4, 8, 12 and 24 hours.

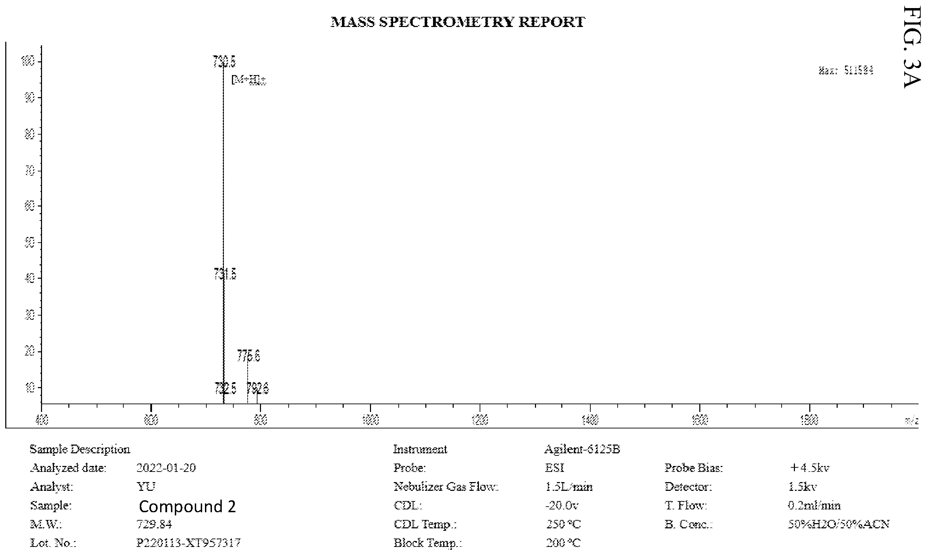

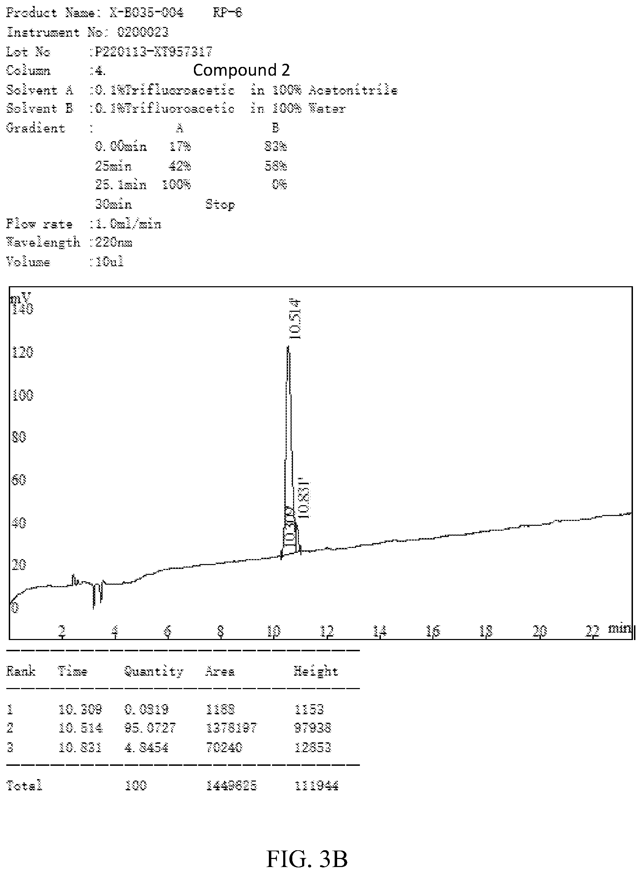

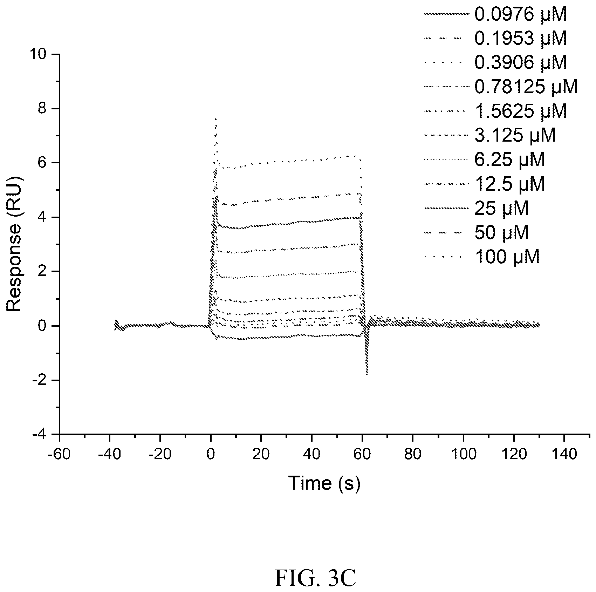

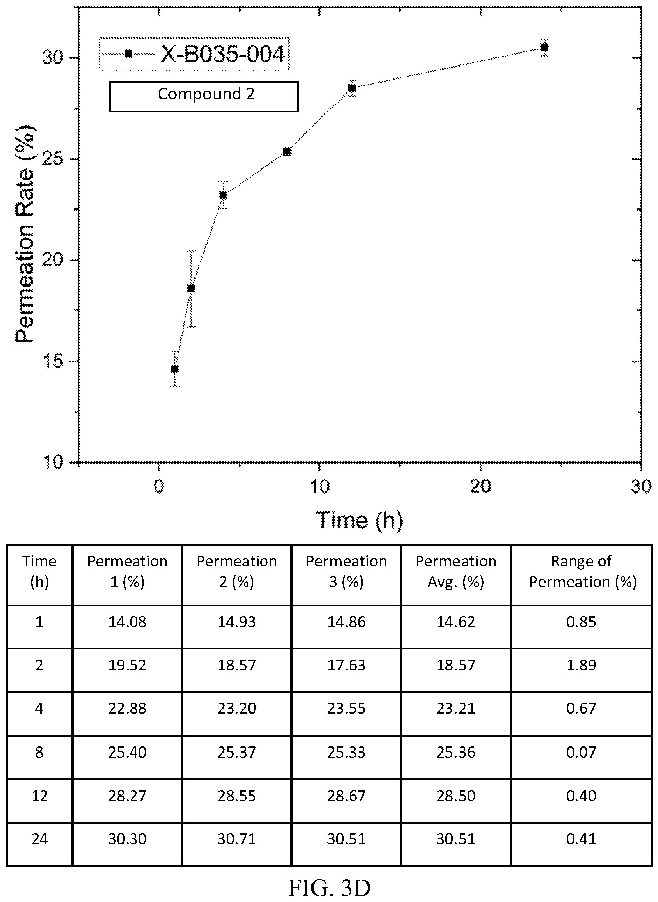

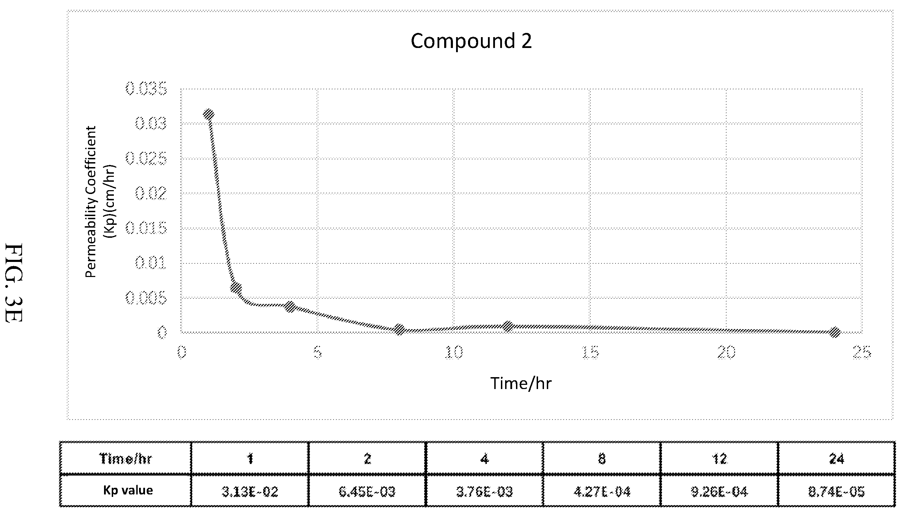

FIG. 3A provides the MS spectrum for Compound 2. FIG. 3B provides the HPLC chromatogram for Compound 2. FIG. 3C provides SPR assay curves showing the binding affinity of Compound 2 for IL-13, the x-axis represents time of reaction and y-axis represents altitude of response (Response Units). FIG. 3D illustrates skin permeation rate of Compound 2 for up to 24 hours. FIG. 3E illustrates average permeability coefficient (Kp) of Compound 2 for 1, 2, 4, 8, 12 and 24 hours.

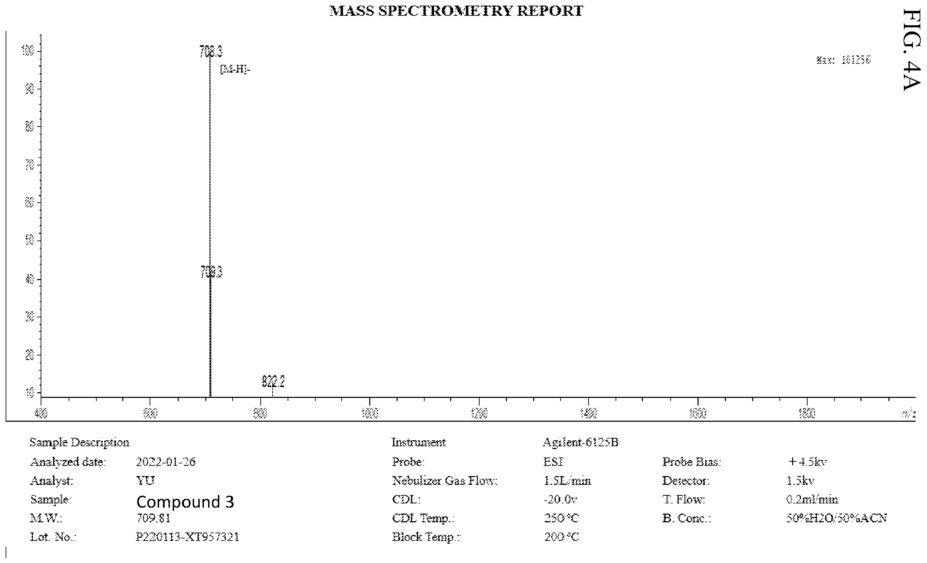

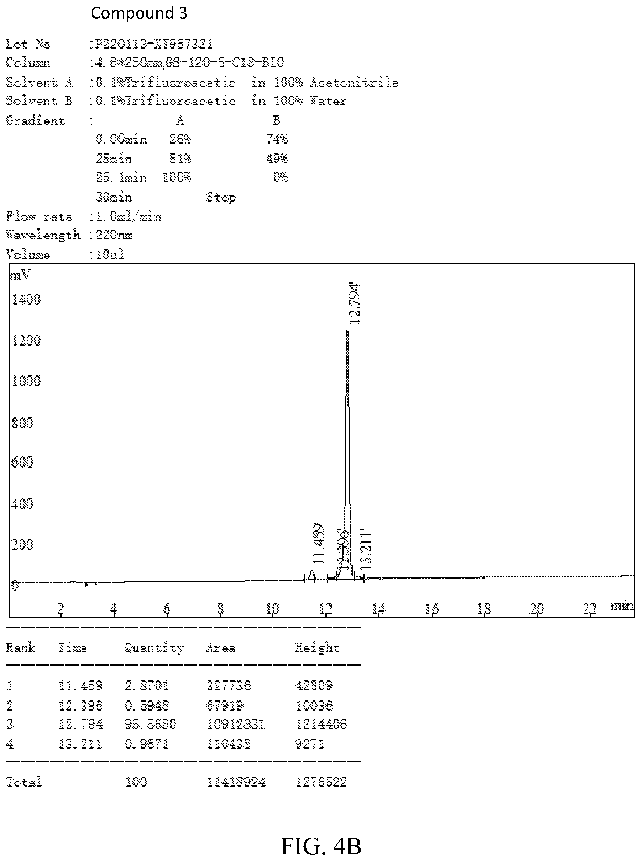

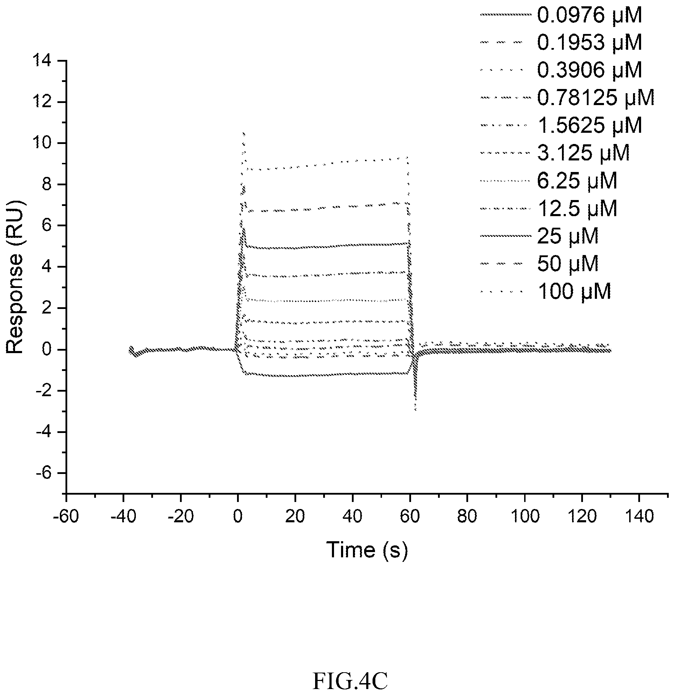

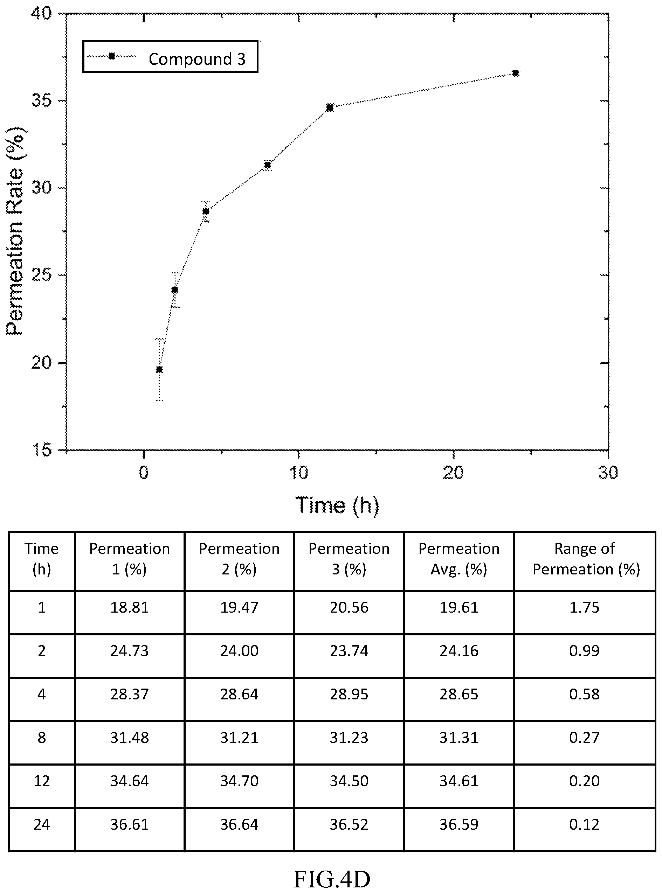

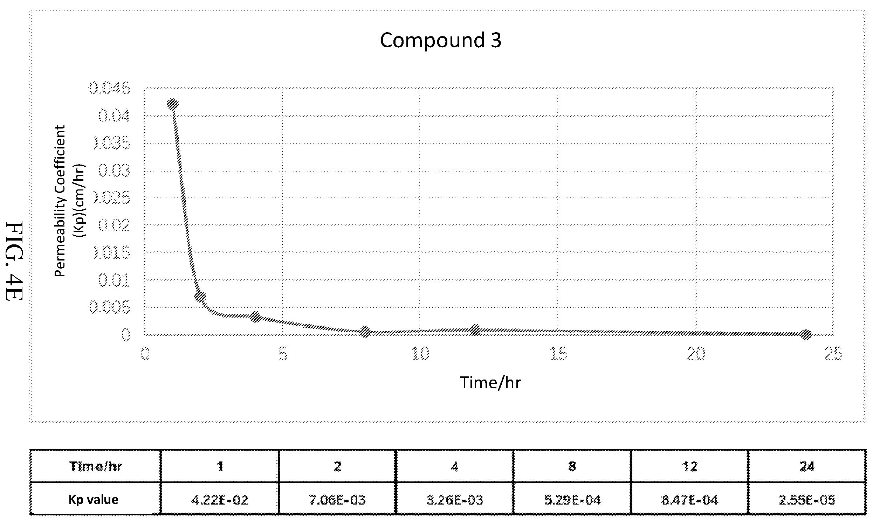

FIG. 4A provides the MS spectrum for Compound 3. FIG. 4B provides the HPLC chromatogram for Compound 3. FIG. 4C provides SPR assay curves showing the binding affinity of Compound 3 for IL-13, the x-axis represents time of reaction and y-axis represents altitude of response (Response Units). FIG. 4D illustrates skin permeation rate of Compound 3 for up to 24 hours. FIG. 4E illustrates average permeability coefficient (Kp) of Compound 3 for 1, 2, 4, 8, 12 and 24 hours.

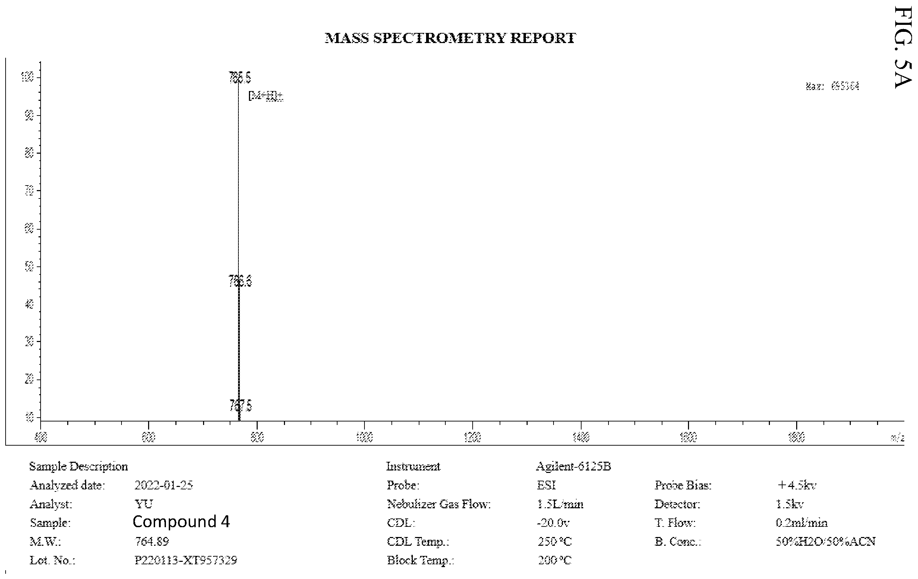

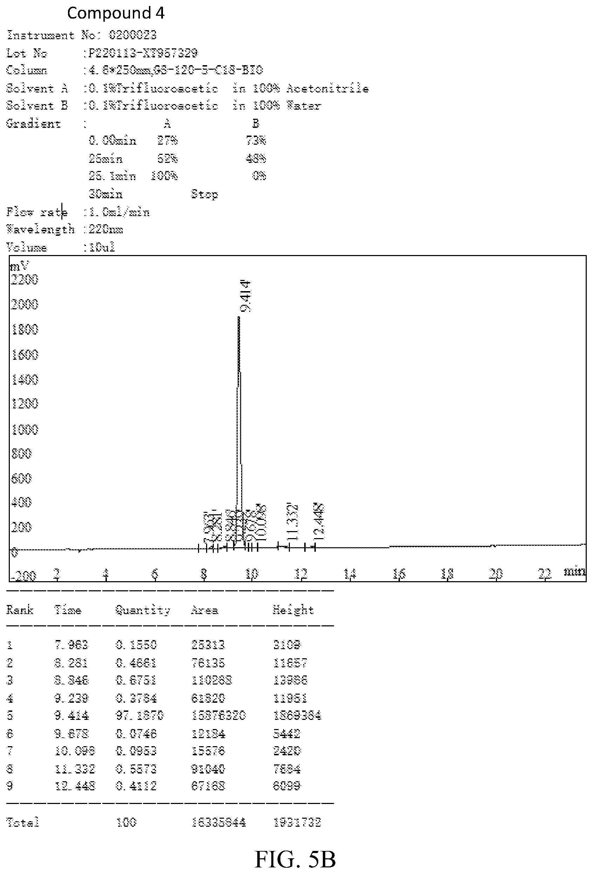

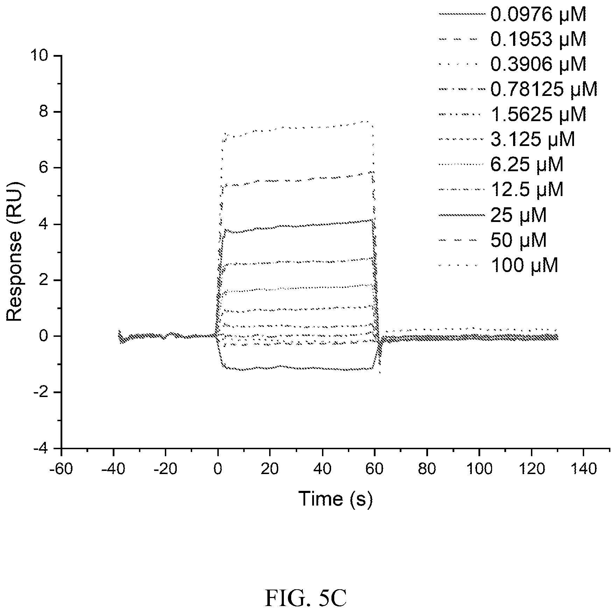

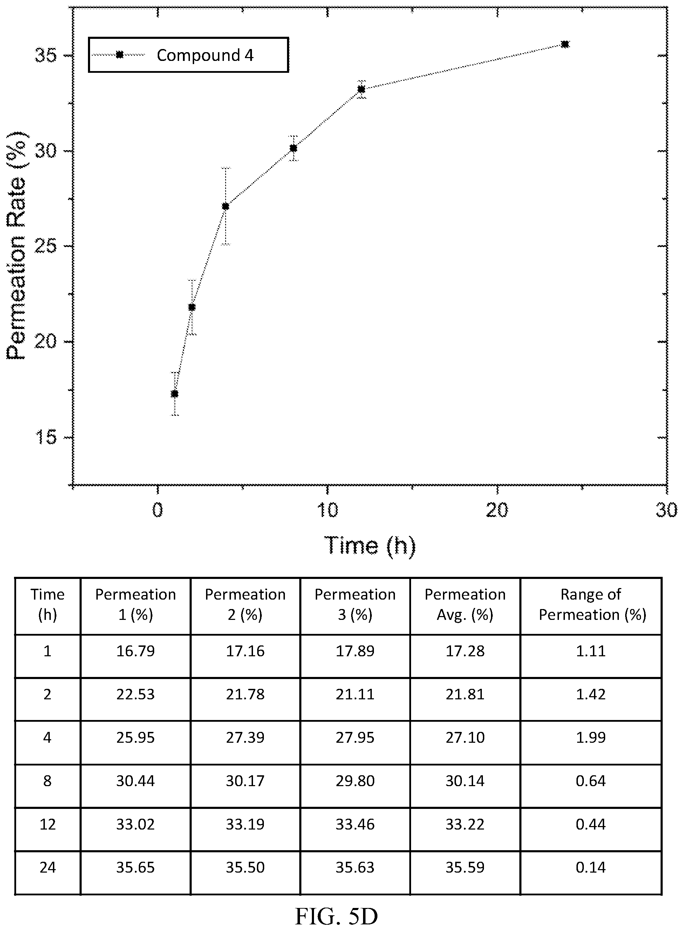

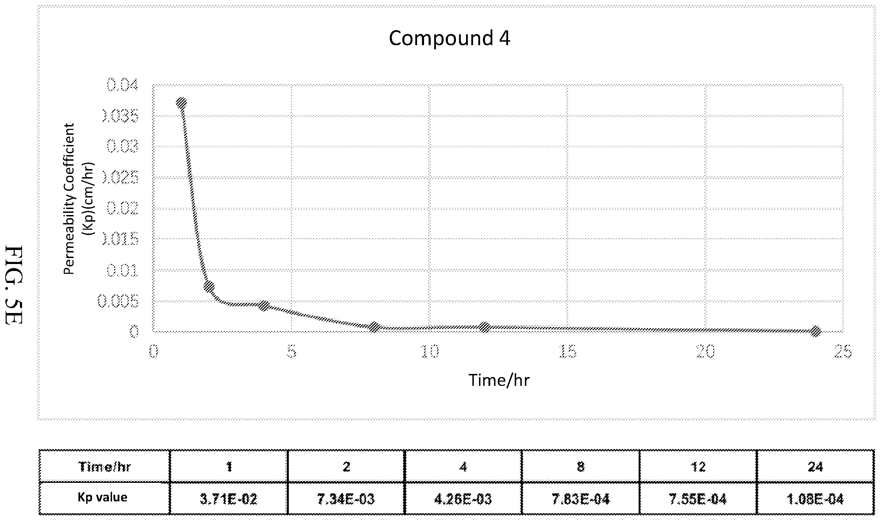

FIG. 5A provides the MS spectrum for Compound 4. FIG. 5B provides the HPLC chromatogram for Compound 4. FIG. 5C provides SPR assay curves showing the binding affinity of Compound 4 for IL-13, the x-axis represents time of reaction and y-axis represents altitude of response (Response Units). FIG. 5D illustrates skin permeation rate of Compound 4 for up to 24 hours. FIG. 5E illustrates average permeability coefficient (Kp) of Compound 4 for 1, 2, 4, 8, 12 and 24 hours.

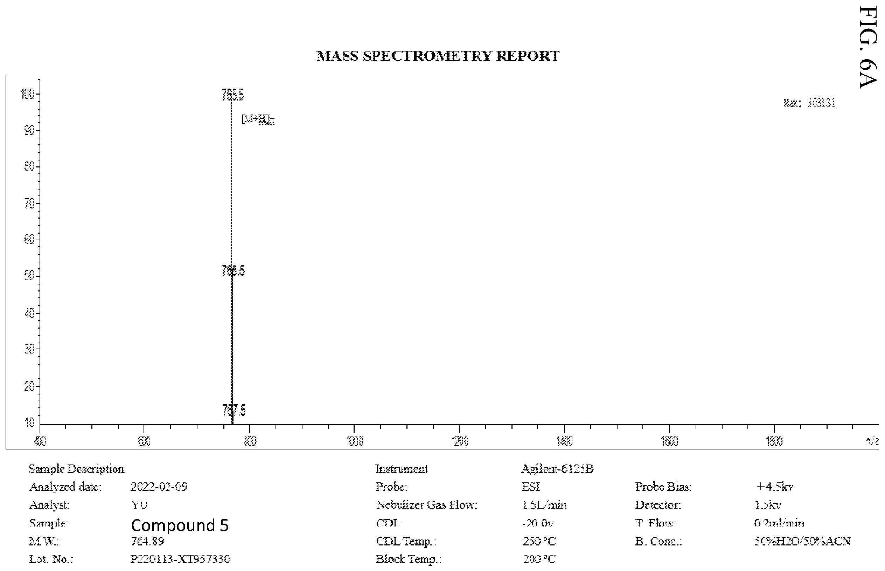

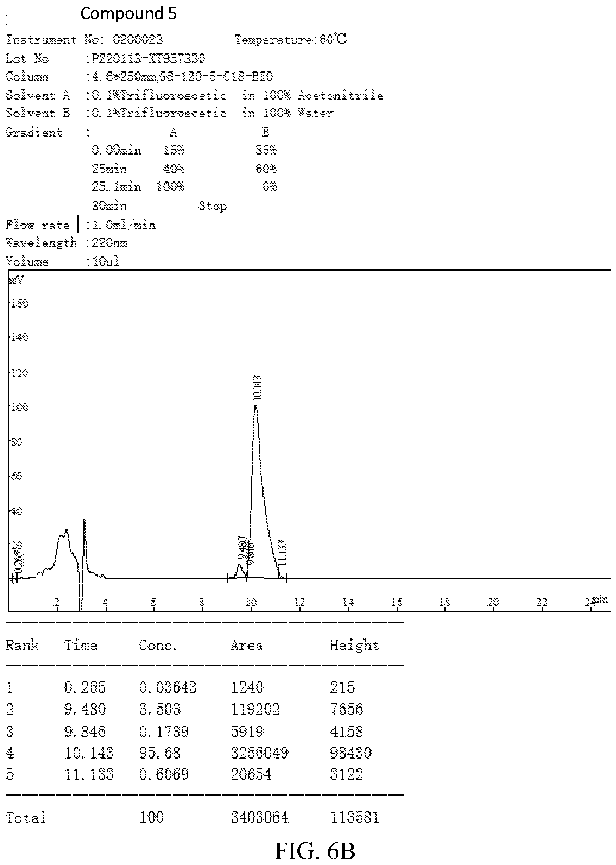

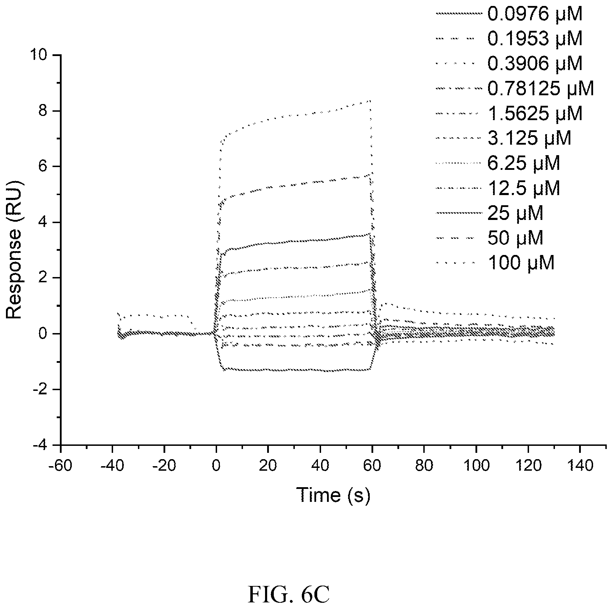

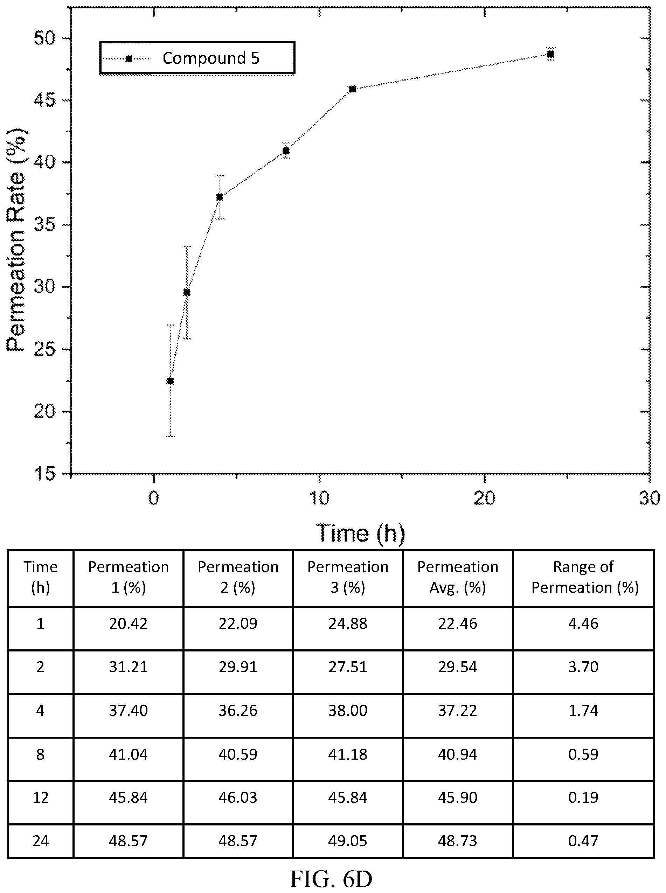

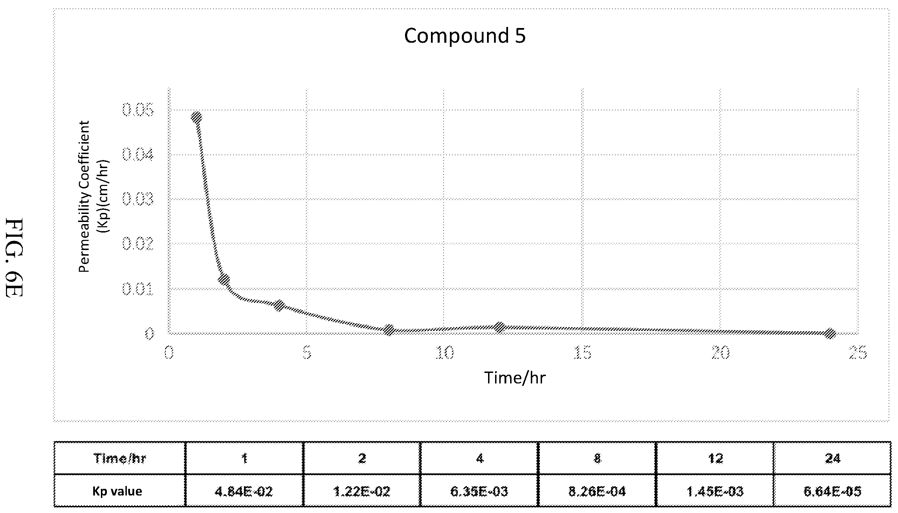

FIG. 6A provides the MS spectrum of Compound 5. FIG. 6B provides the HPLC chromatogram for Compound 5. FIG. 6C provides SPR assay curves showing the binding affinity of Compound 5 for IL-13, the x-axis represents time of reaction and y-axis represents altitude of response (Response Units). FIG. 6D illustrates skin permeation rate of Compound 5 for up to 24 hours. FIG. 6E illustrates average permeability coefficient (Kp) of Compound 5 for 1, 2, 4, 8, 12 and 24 hours.

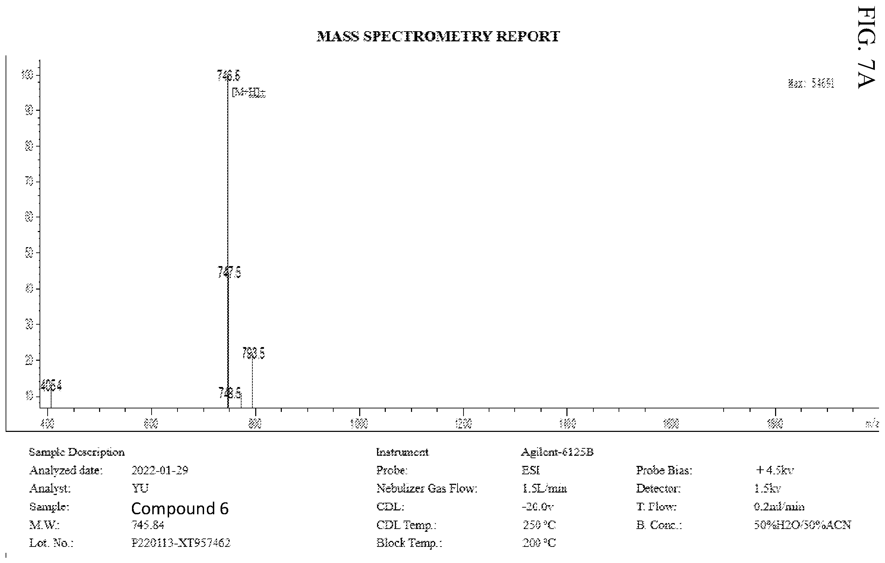

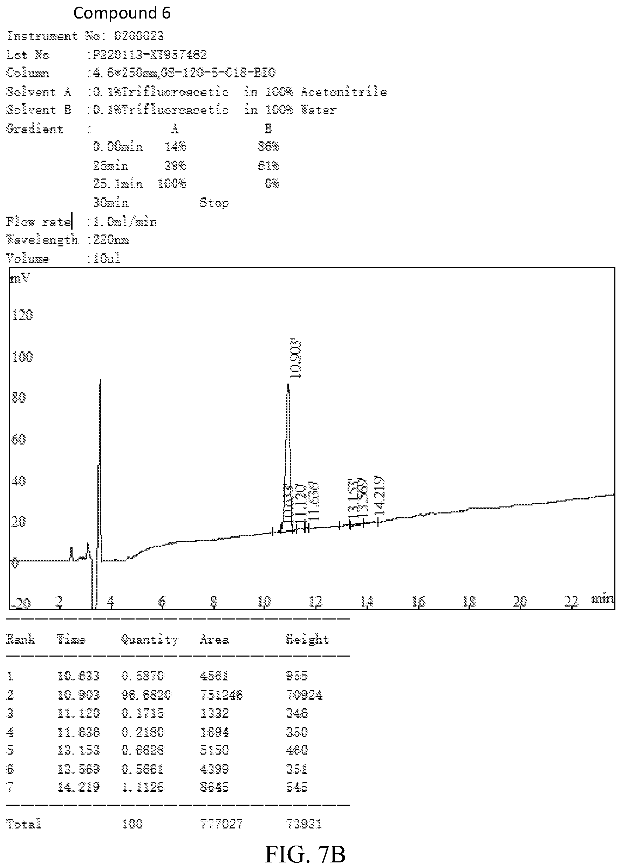

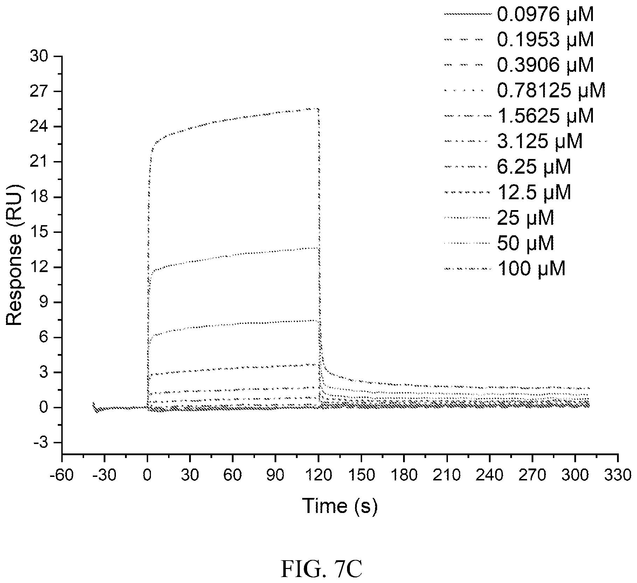

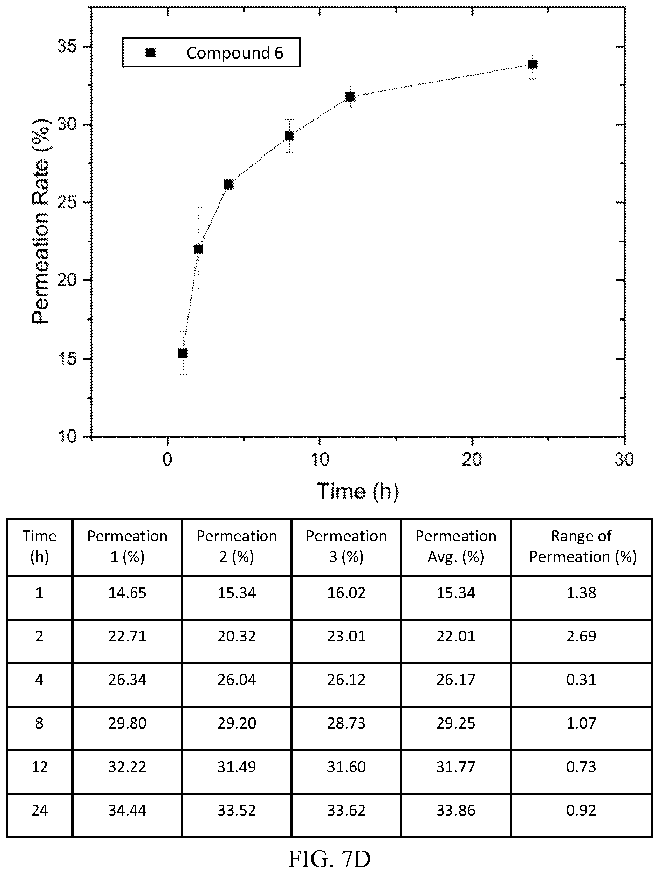

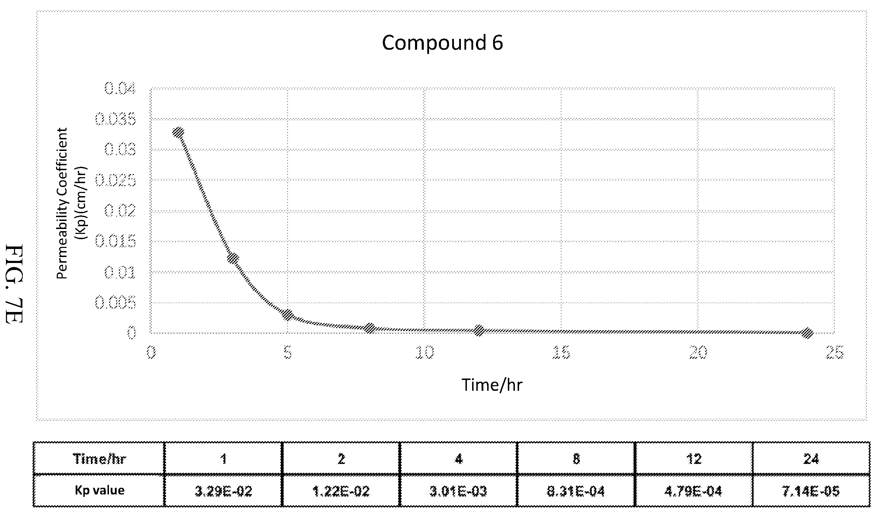

FIG. 7A provides the MS spectrum for Compound 6. FIG. 7B provides the HPLC chromatogram for Compound 6. FIG. 7C provides SPR assay curves showing the binding affinity of Compound 6 for IL-13, the x-axis represents time of reaction and y-axis represents altitude of response (Response Units). FIG. 7D illustrates skin permeation rate of Compound 6 for up to 24 hours. FIG. 7E illustrates average permeability coefficient (Kp) of Compound 6 for 1, 2, 4, 8, 12 and 24 hours.

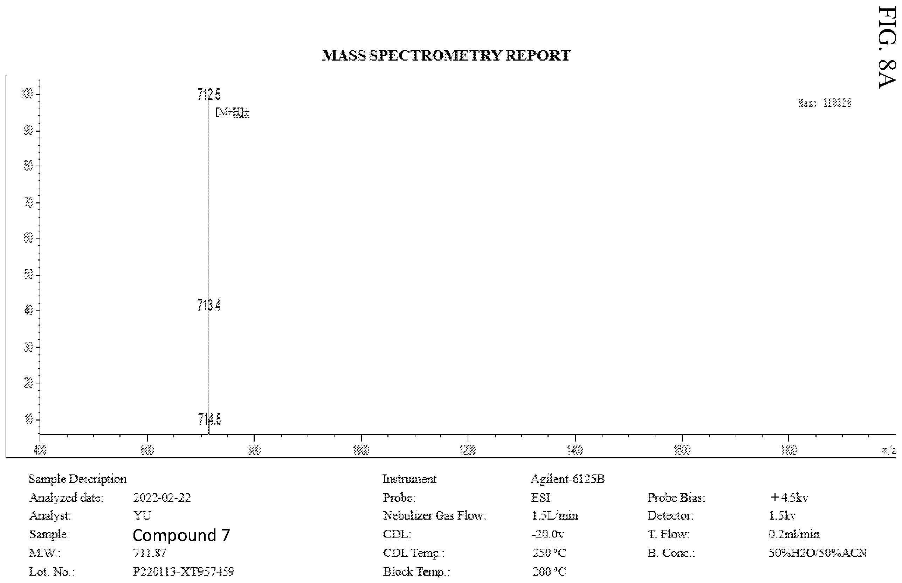

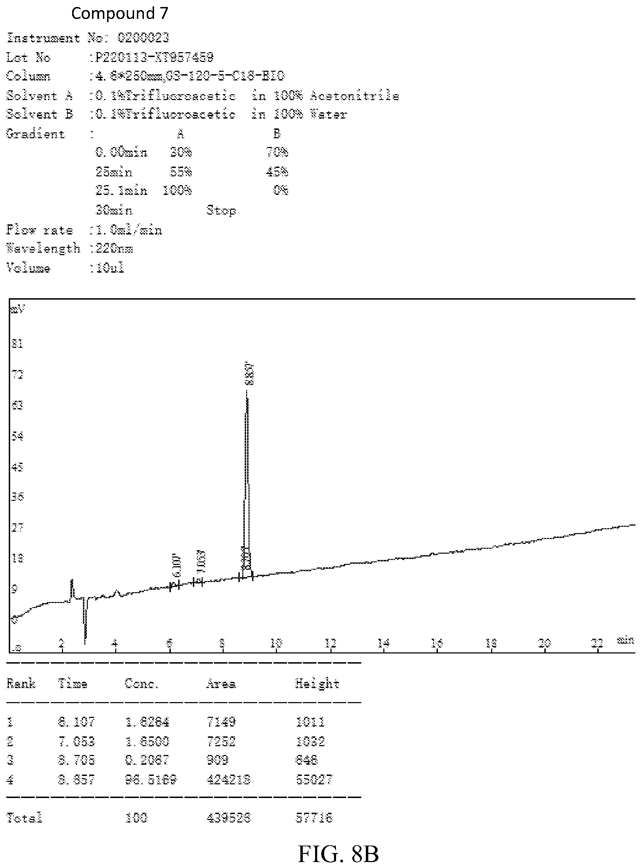

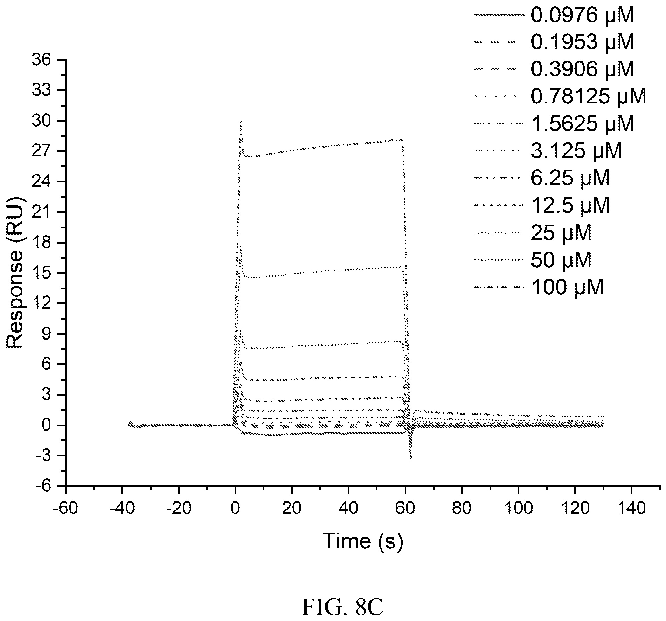

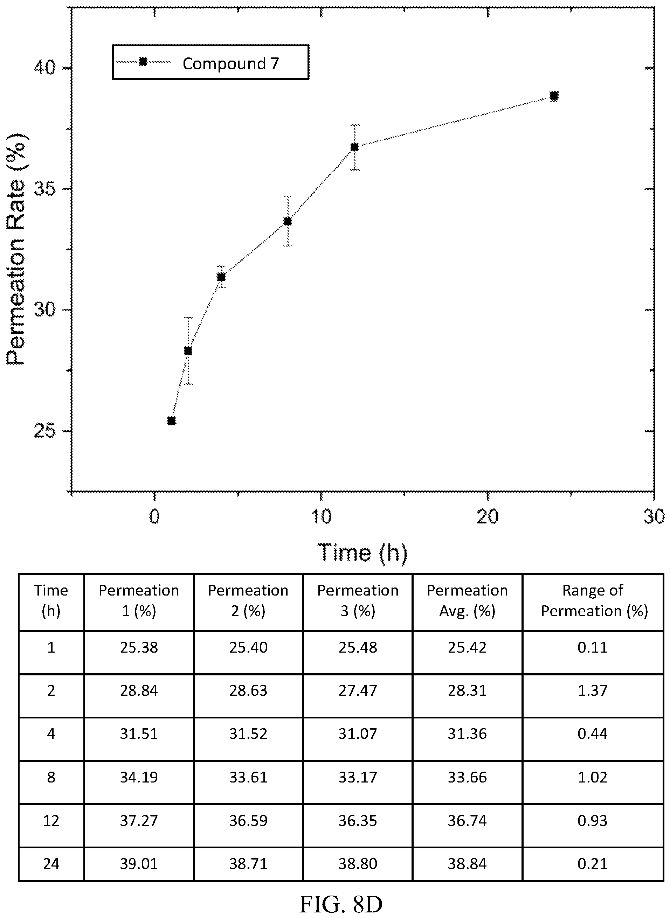

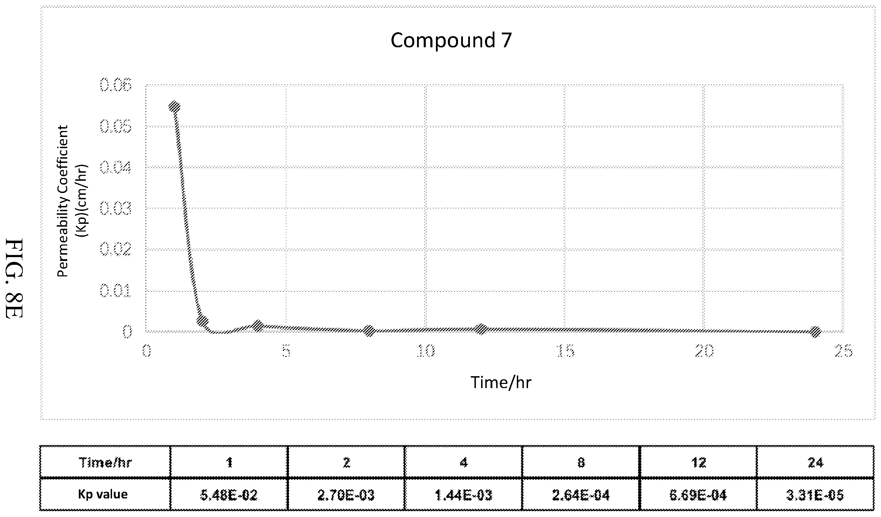

FIG. 8A provides the MS spectrum for Compound 7. FIG. 8B provides the HPLC chromatogram for Compound 7. FIG. 8C provides SPR assay curves showing the binding affinity of Compound 7 for IL-13, the x-axis represents time of reaction and y-axis represents altitude of response (Response Units). FIG. 8D illustrates skin permeation rate of Compound 7 for up to 24 hours. FIG. 8E illustrates average permeability coefficient (Kp) of Compound 7 for 1, 2, 4, 8, 12 and 24 hours.

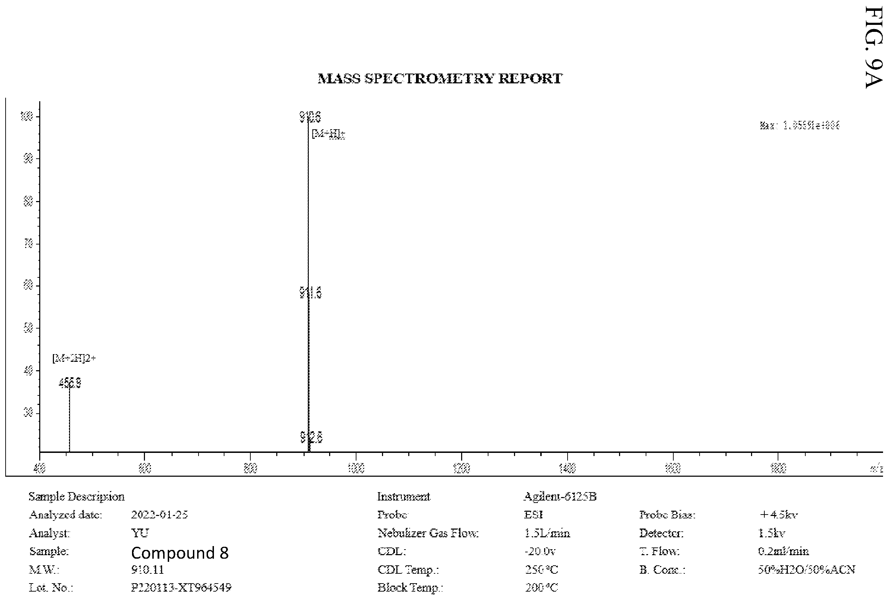

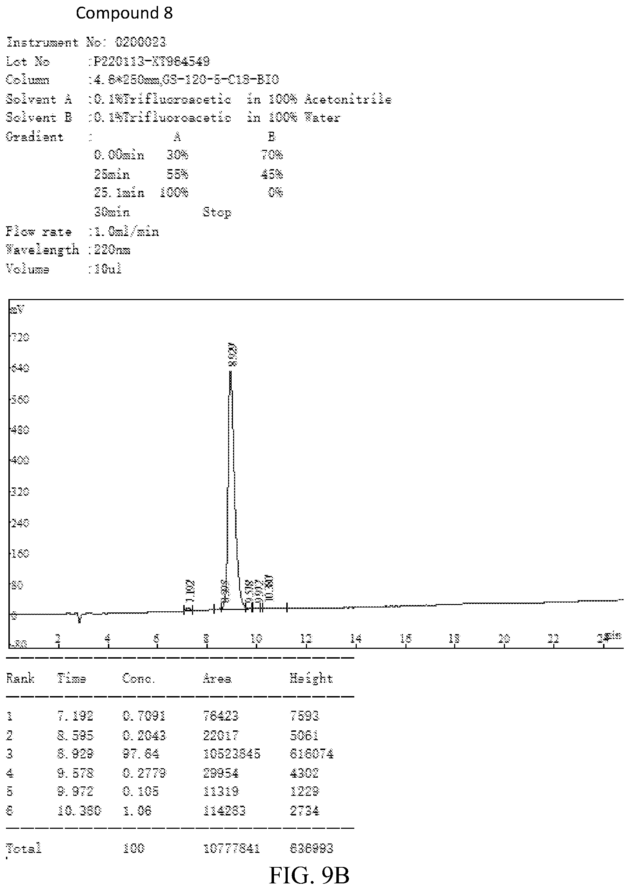

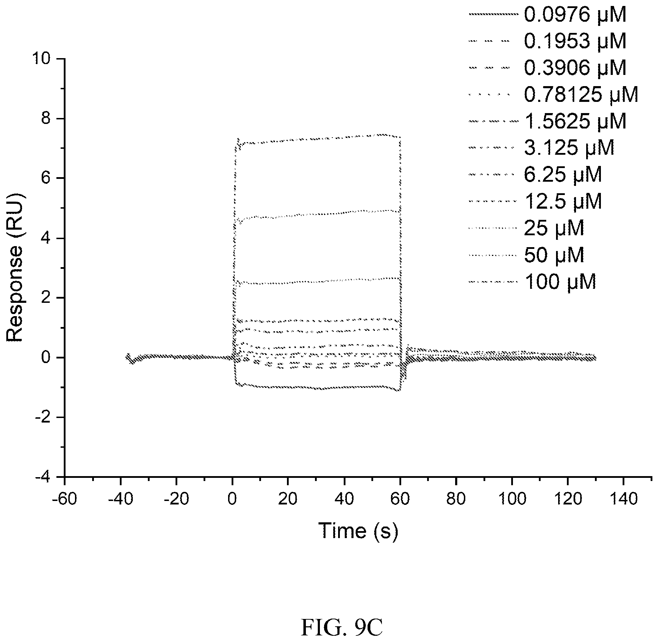

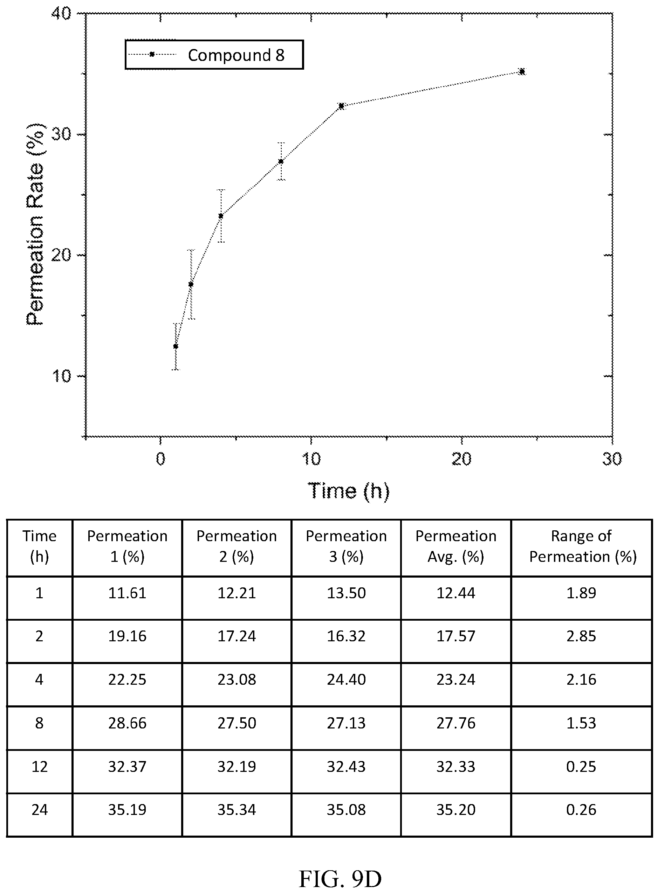

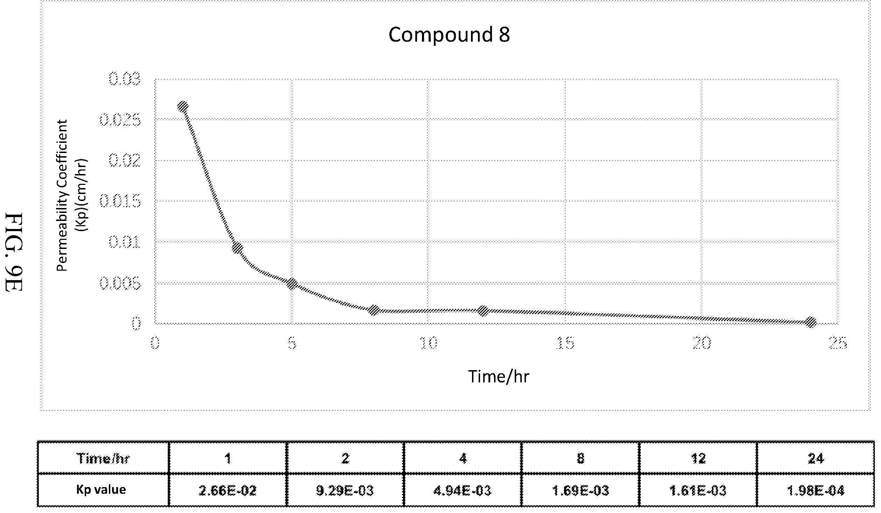

FIG. 9A provides the MS spectrum for Compound 8. FIG. 9B provides the HPLC chromatogram for Compound 8. FIG. 9C provides SPR assay curves showing the binding affinity of Compound 8 for IL-13, the x-axis represents time of reaction and y-axis represents altitude of response (Response Units). FIG. 9D illustrates skin permeation rate of Compound 8 for up to 24 hours. FIG. 9E illustrates average permeability coefficient (Kp) of Compound 8 for 1, 2, 4, 8, 12 and 24 hours.

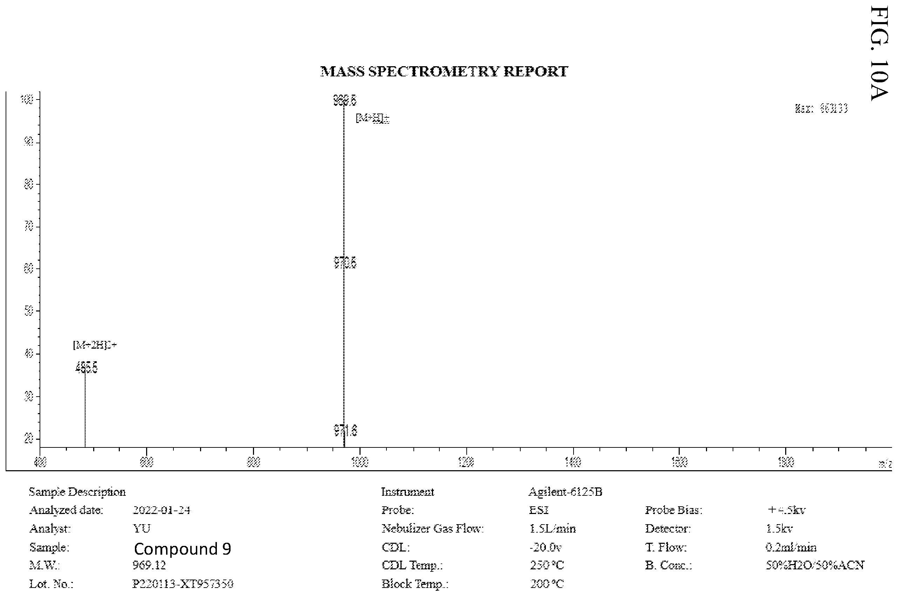

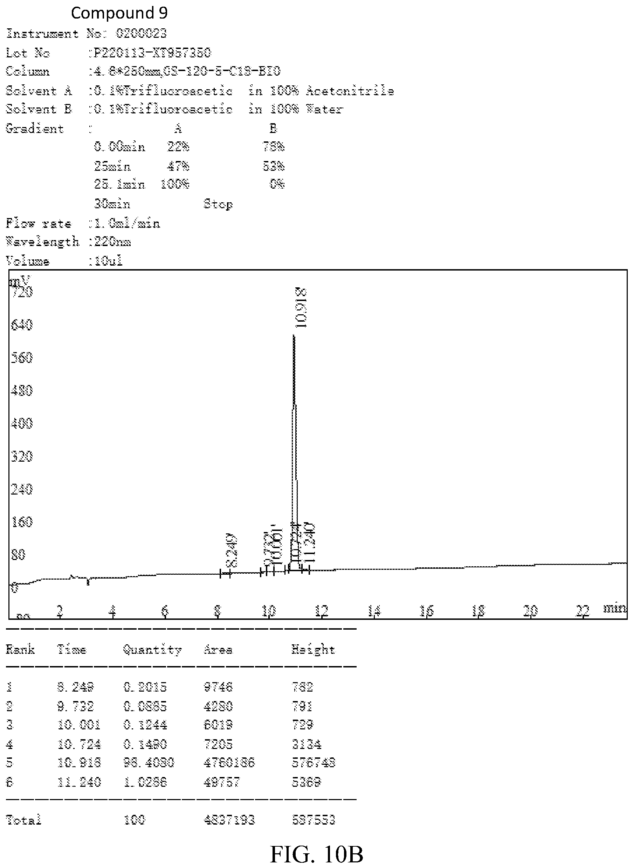

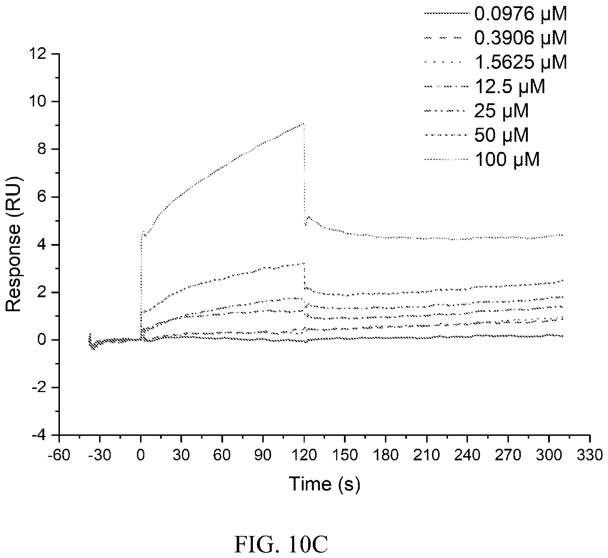

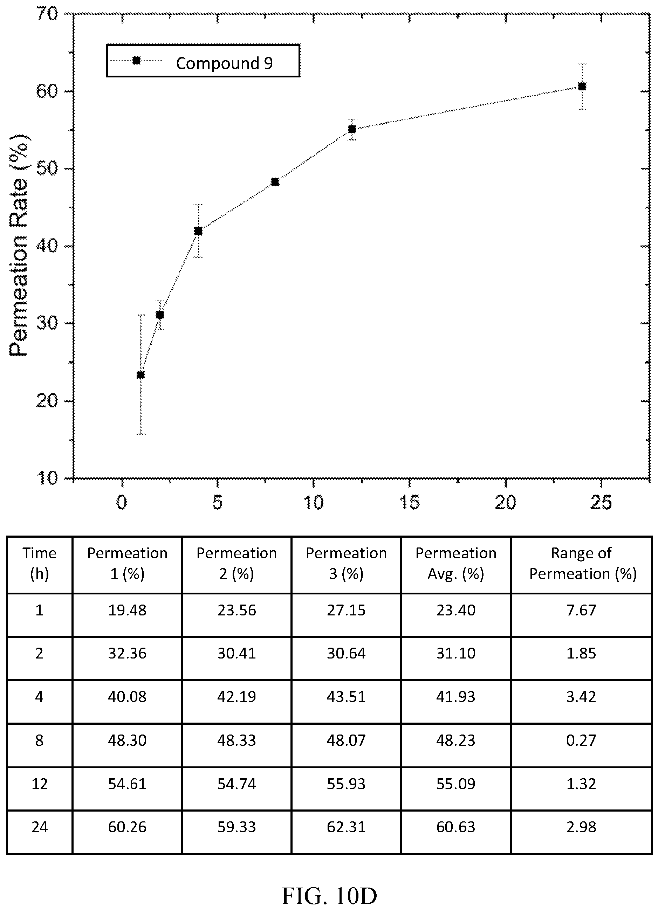

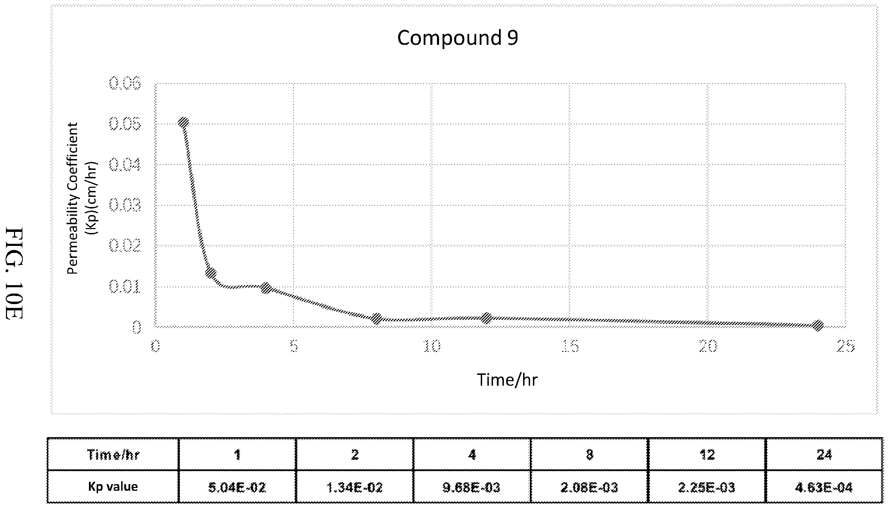

FIG. 10A provides the MS spectrum for Compound 9. FIG. 10B provides the HPLC chromatogram for Compound 9. FIG. 10C provides SPR assay curves showing the binding affinity of Compound 9 for IL-13, the x-axis represents time of reaction and y-axis represents altitude of response (Response Units). FIG. 10D illustrates skin permeation rate of Compound 9 for up to 24 hours. FIG. 10E illustrates average permeability coefficient (Kp) of Compound 9 for 1, 2, 4, 8, 12 and 24 hours.

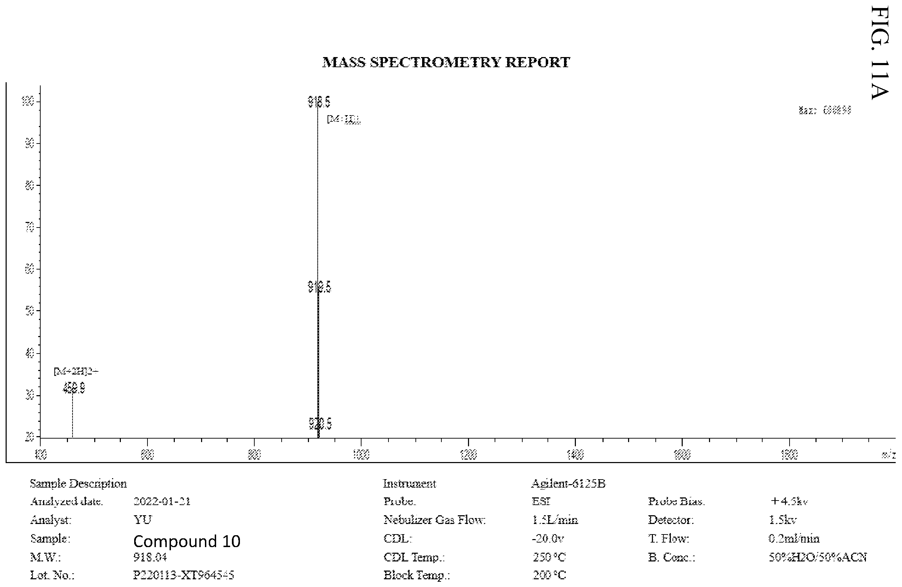

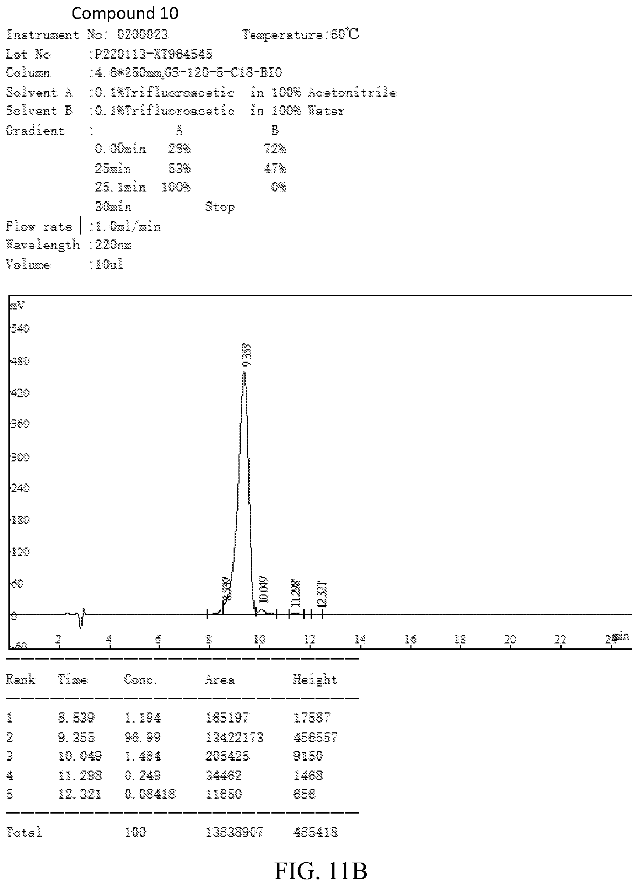

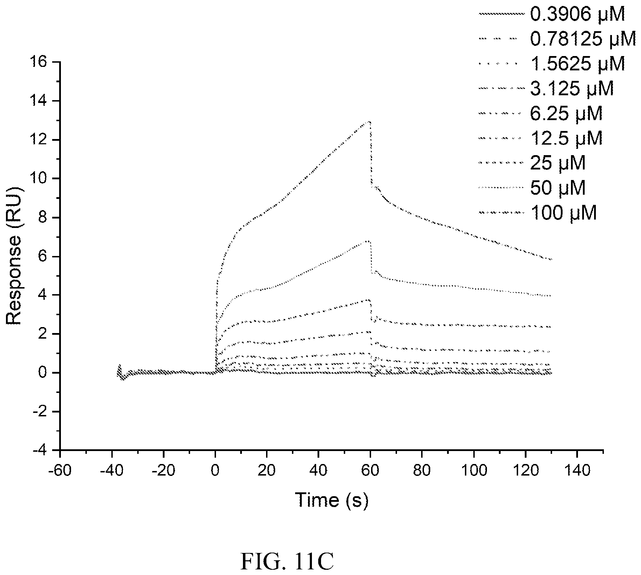

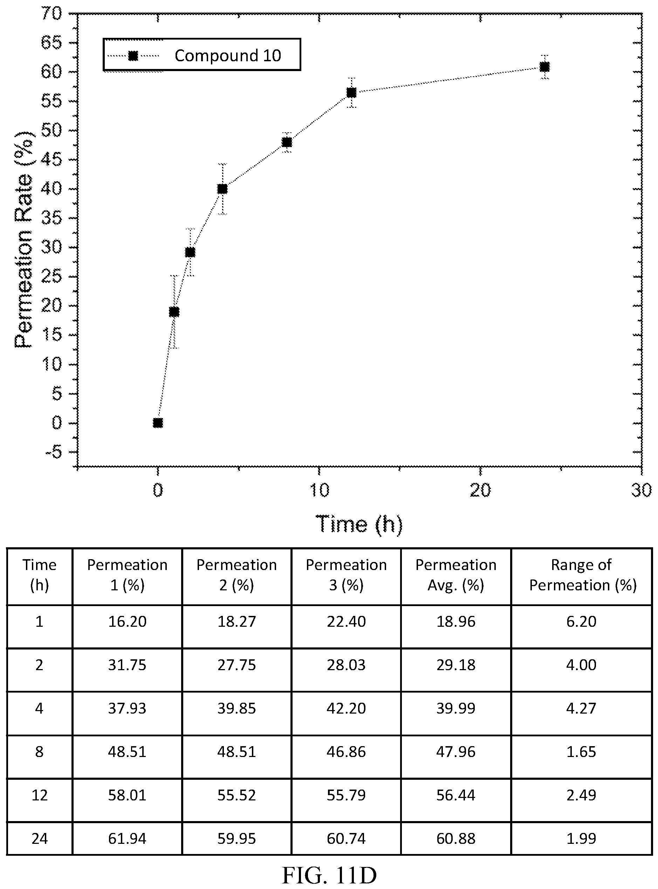

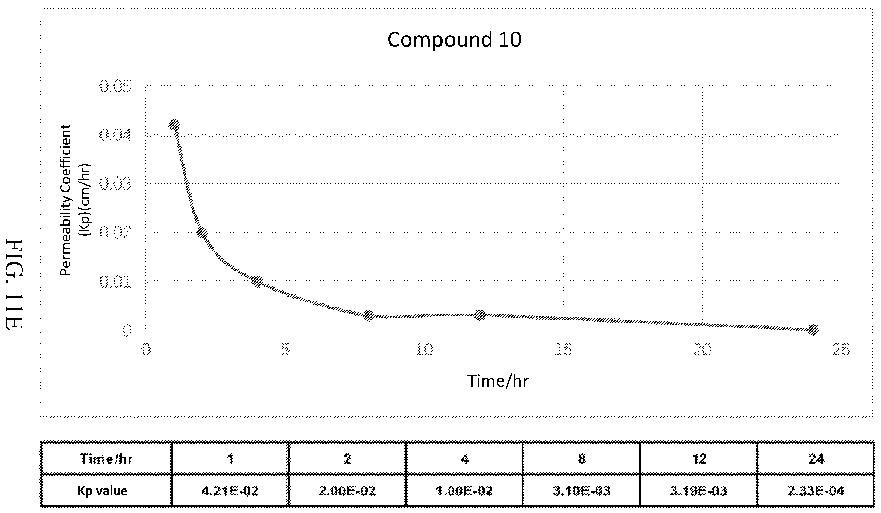

FIG. 11A provides the MS spectrum for Compound 10. FIG. 11B provides the HPLC chromatogram for Compound 10. FIG. 11C provides SPR assay curves showing the binding affinity of Compound 10 for IL-13, the x-axis represents time of reaction and y-axis represents altitude of response (Response Units). FIG. 11D illustrates skin permeation rate of Compound 10 for up to 24 hours. FIG. 11E illustrates average permeability coefficient (Kp) of Compound 10 for 1, 2, 4, 8, 12 and 24 hours.

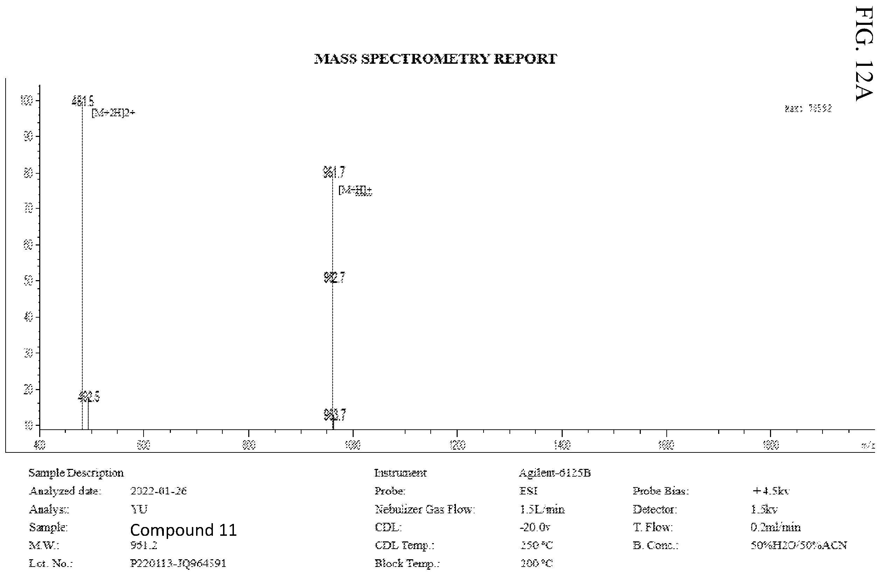

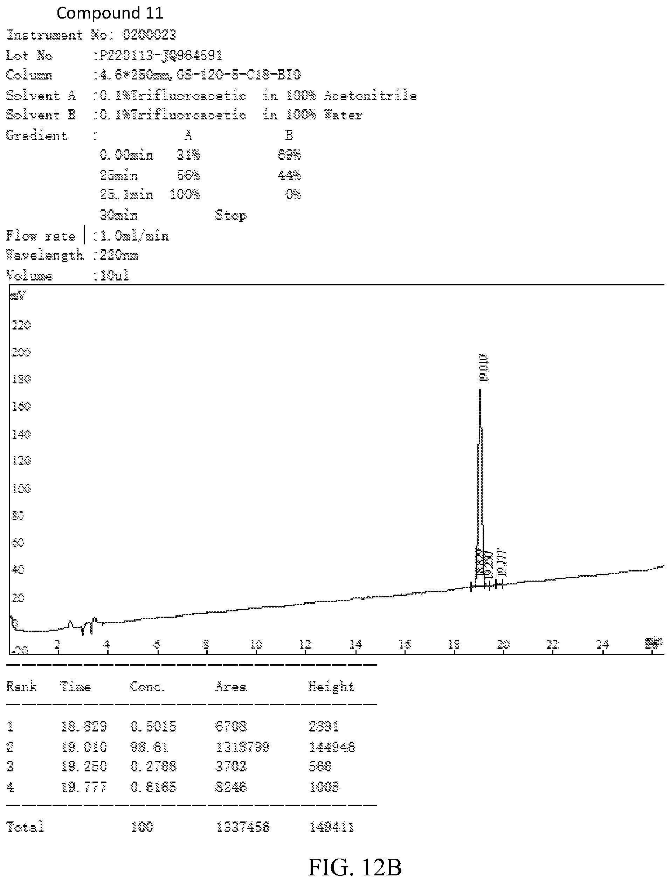

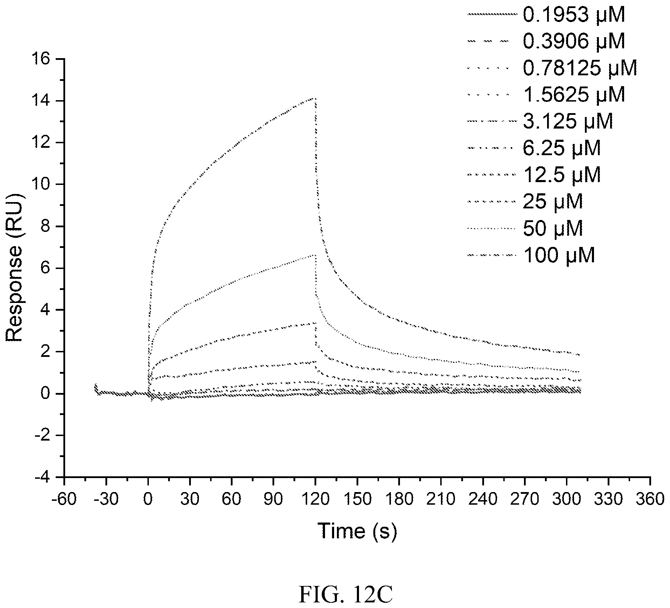

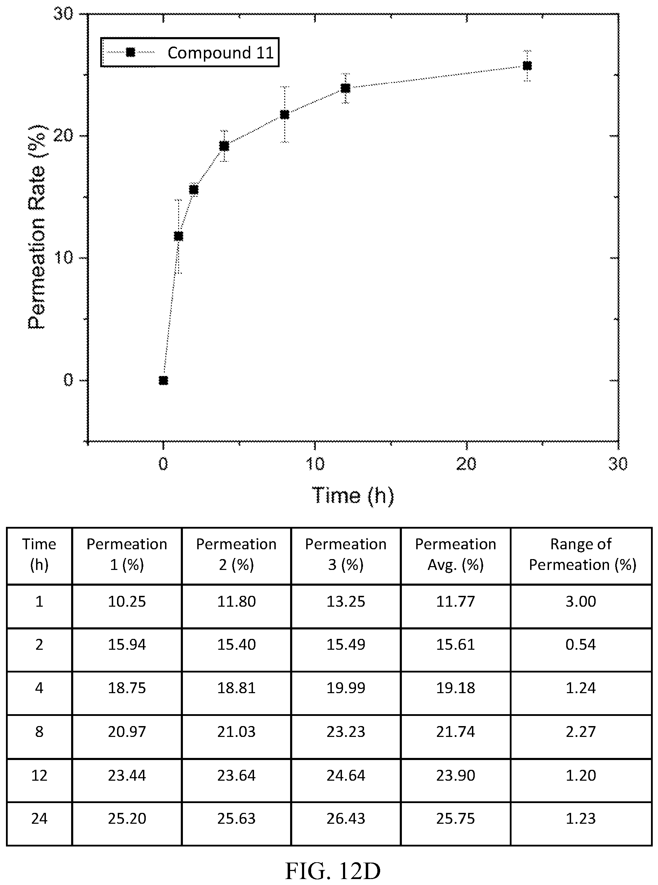

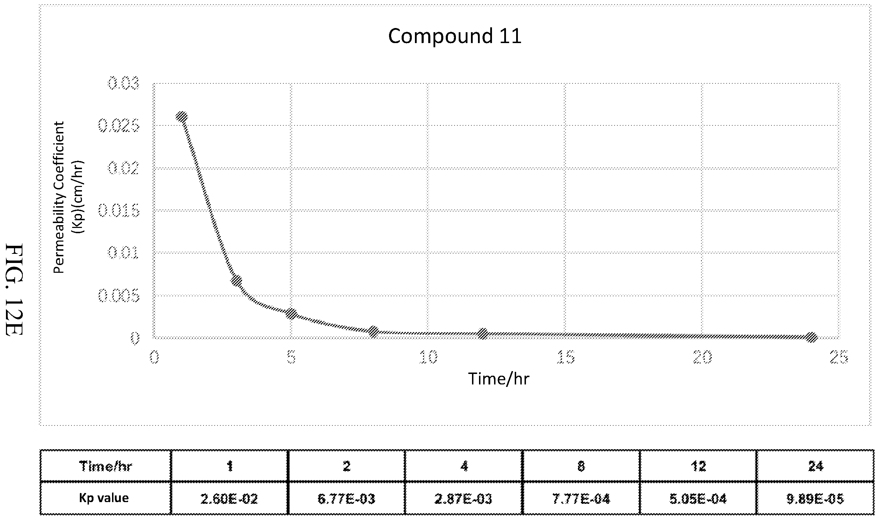

FIG. 12A provides the MS spectrum for Compound 11. FIG. 12B provides the HPLC chromatogram for Compound 11. FIG. 12C provides SPR assay curves showing the binding affinity of Compound 11 for IL-13, the x-axis represents time of reaction and y-axis represents altitude of response (Response Units). FIG. 12D illustrates skin permeation rate of Compound 11 for up to 24 hours. FIG. 12E illustrates average permeability coefficient (Kp) of Compound 11 for 1, 2, 4, 8, 12 and 24 hours.

### DETAILED DESCRIPTION OF THE DISCLOSURE

Unless otherwise defined herein, scientific and technical terms used in this application shall have the meanings that are commonly understood by those of ordinary skill in the art. In case of conflict, the present specification, including definitions, will control.

Where aspects or embodiments are described in terms of a Markush group or other grouping of alternatives, the present application encompasses not only the entire group listed as a whole, but each member of the group individually and all possible subgroups of the main group, and also the main group absent one or more of the group members.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

Throughout the specification, where compositions are described as having, including, or comprising (or variations thereof), specific components, it is contemplated that compositions also may consist essentially of, or consist of, the recited components. Similarly, where methods or processes are described as having, including, or comprising specific process steps, the processes also may consist essentially of, or consist of, the recited processing steps. Further, it should be understood that the order of steps or order for performing certain actions is immaterial so long as the compositions and methods described herein remains operable. Moreover, two or more steps or actions can be conducted simultaneously.

The term "including," as used herein, means "including but not limited to." "Including" and "including but not limited to" are used interchangeably. Thus, these terms will be understood to imply the inclusion of a stated integer (or components) or group of integers (or components), but not the exclusion of any other integer (or components) or group of integers (or components).

Any example(s) following the term "e.g." or "for example" is not meant to be exhaustive or limiting.

Unless otherwise required by context, singular terms shall include pluralities and plural terms shall include the singular.

The articles "a," "an" and "the" are used herein to refer to one or to more than one (i.e., to at least one) of the grammatical object of the article.

Notwithstanding that the disclosed numerical ranges and parameters are approximations, the numerical values set forth in the specific examples are reported as precisely as possible. Any numerical value, however, inherently contains certain errors necessarily resulting from the standard deviation found in their respective testing measurements. Accordingly, as used herein, the term "about" permits a variation of ±10%. As used herein, the term "about" refers to a value or parameter that includes (and describes) embodiments that are directed to that value or parameter per se. Moreover, all ranges disclosed herein are to be understood to encompass any and all subranges subsumed therein. For example, a stated range of "1 to 10" should be considered to include any and all subranges and individual numbers between (and inclusive of) the minimum value of 1 and the maximum value of 10; that is, all subranges beginning with a minimum value of 1 or more, e.g., 1 to 6.1, and ending with a maximum value of 10 or less, e.g., 5.5 to 10 or individual numbers within a stated range or subrange, e.g., 2, 5, 8 or 10.

As used herein, amino acids having L-configuration are represented using standard three letter codes, e.g. Ala for alanine, Gly for glycine., Val for valine, Leu for leucine, Ile for isoleucine, Pro for proline, Ser for serine, Thr for threonine, Asn for asparagine, Gln for glutamine, Cys for cysteine, Met for methionine, Phe for phenylalanine, Tyr for tyrosine, Trp for tryptophan, Asp for Aspartate, Glu for glutamate, His for histidine, Lys for lysine and Arg for arginine. As used herein, amino acids having D-configuration are represented by adding "D-" before the standard three letter codes, e.g. D-Ala for D-alanine, D-Gly for D-glycine., D-Val for D-valine, D-Leu for D-leucine, D-Ile for D-isoleucine, D-Pro for D-proline, D-Ser for D-serine, D-Thr for D-threonine, D-Asn for D-asparagine, D-Gln for D-glutamine, D-Cys for D-cysteine, D-Met for D-methionine, D-Phe for D-phenylalanine, D-Tyr for D-tyrosine, D-Trp for D-tryptophan, D-Asp for D-Aspartate, D-Glu for D-glutamate, D-His for D-histidine, D-Lys for D-lysine and D-Arg for D-arginine.

### DEFINITIONS

In order that the disclosure may be more readily understood, certain terms are first defined. These definitions should be read in light of the remainder of the disclosure, as understood by a person of ordinary skill in the art. Unless defined otherwise, all technical and scientific terms used herein have the same meaning as commonly understood by a person of ordinary skill in the art.

As used herein, the terms "administer" and "administration" include any suitable route for providing the subject with the cyclic oligopeptides or pharmaceutical compositions of the disclosure for treatment of a skin disorder or condition, such as an IL-13 associated skin disorder or condition. For example, the cyclic oligopeptide or pharmaceutical composition may be administered topically, transdermally, subcutaneously, intravenously or orally. Dosage forms useful in the methods of this disclosure may include films, dispersions, suspensions, solutions, ointments, lotions, creams, powders, drops (e.g. eye or ear drops), sprays, patches and the like. Administering can also be performed, for example, once, a plurality of times, and/or over one or more extended periods. In some embodiments, the administration includes both direct administration, including self-administration, and indirect administration, including the act of prescribing a drug. For example, as used herein, a physician who instructs a patient to self-administer a drug, or to have the drug administered by another and/or who provides a patient with a prescription for a drug is administering the drug to the patient. When a method is part of a therapeutic regimen involving more than one pharmaceutical composition or treatment modality, the disclosure contemplates that the pharmaceutical compositions may be administered at the same or differing times and via the same or differing routes of administration.

As used herein, the terms "bind" and "binding" refer to molecular binding of two or more molecules, e.g. IL-13 and cyclic oligopeptide, which is an attractive interaction between the two or more molecules that results in a stable association in which the molecules are in close proximity to each other. The strength of molecular binding or the affinity among the binding molecules in this disclosure can be quantified by, for example, a Surface Plasmon Resonance (SPR) assay.

As used herein, the term "cyclic oligopeptide" refers to oligopeptide chains which contain a circular sequence of bonds. Cyclic oligopeptides are typically first generated as linear oligopeptides and then cyclized, *e.g.,* by chemical or enzymatic means. The circular sequence of bonds can be formed by, for example, a connection between the amino and carboxyl ends of the linear oligopeptide, a connection between the amino end of the linear oligopeptide and a side chain of one of the amino acid residues in the oligopeptide, a connection between the carboxyl end of the linear oligopeptide and a side chain of one of the amino acid residues in the oligopeptide, and/or the side chains of two different amino acid residues in the oligopeptide. The length of a cyclic oligopeptide of this disclosure ranges from two amino acid residues to twenty amino acid residues. In some embodiments, the length of a cyclic oligopeptide of this disclosure ranges from four amino acid residues to ten amino acid residues. In some embodiments, the length of a cyclic oligopeptide of this disclosure ranges from six amino acid residues to ten amino acid residues.

The term "dosage unit form" as used herein refers to physically discrete units suited as unitary dosages for the mammalian subjects to be treated; each unit containing a predetermined quantity of active compound calculated to produce the desired therapeutic effect in association with the required pharmaceutical carrier.

As used herein, the terms "Interleukin-13" and "IL-13" refer to a protein that in humans is encoded by the *IL13* gene or a protein encoded by a homologous gene of human *IL13* gene in a non-human species.

As used herein, the term "IL-13-associated skin disorder or condition" refers to a skin disease, a skin disorder or a skin condition that is associated dysfunction of IL-13 involved immune regulations. An IL-13-associated skin disorder or condition may be an inflammatory, an allergic, or an autoimmune disorder or condition. Non-limiting examples of IL-13-associated skin disorders or conditions include atopic dermatitis, allergic contact dermatitis, urticaria, eczema, chronic hand eczema, bullous diseases (bullous pemphigoid), alopecia areata, prurigo and molluscum contagiosum.

The terms "oligopeptide" refers to a polymer of amino acid residues. The term also applies to amino acid polymers in which one or more amino acids are chemical analogues or modified derivatives of a corresponding naturally-occurring amino acids.

As used herein, "pharmaceutically acceptable carrier" and "pharmaceutically acceptable excipient" are used interchangeably refer to any and all solvents, dispersion media, coatings, antibacterial and antifungal agents, isotonic and absorption delaying agents, and the like that are physiologically compatible. Pharmaceutically acceptable carriers are well known in the art. See, *e.g.,* Remington's Pharmaceutical Sciences and U.S. Pharmacopeia: National Formulary, Mack Publishing Company, Easton, PA (1984). Some examples of pharmaceutically acceptable carriers are water, saline, phosphate buffered saline, dextrose, glycerol, ethanol and the like, as well as combinations thereof. In many cases, it will be preferable to include isotonic agents, for example, sugars, polyalcohols such as mannitol, sorbitol, or sodium chloride in the composition. Additional examples of pharmaceutically acceptable substances are wetting agents or minor amounts of auxiliary substances such as wetting or emulsifying agents, preservatives, or buffers, which enhance the shelf life or effectiveness of the antibody. Pharmaceutical compositions may be prepared by mixing an antibody disclosed herein with acceptable carriers, excipients, or stabilizers in the form of, *e.g.,* lyophilized powders, slurries, aqueous solutions or suspensions (see, *e.g.,* Brunton et al. (eds.), Goodman and Gilman's The Pharmacological Basis of Therapeutics, Thirteenth Edition, (2018) McGraw-Hill, New York, NY; Adejare, A. (ed.), Remington: The Science and Practice of Pharmacy, Twenty-Third Edition, (2020) Academic Press, New York, NY; Nema, et al. (eds.), Pharmaceutical Dosage Forms: Parenteral Medications, Third Edition, (2010) Informa Healthcare, NY, Augsburger et al. (eds.), Pharmaceutical Dosage Forms: Tablets, Third Edition (2008) CRC Press, NY; Lieberman, et al. (eds.) Pharmaceutical Dosage Forms: Disperse Systems, Second Edition, (2010) Informa Helathcare, NY; Weiner and Kotkoskie, Excipient Toxicity and Safety, (2007) Informa Healthcare, New York, NY).

As used herein, the term "pharmaceutically acceptable salt" refers to those salts which are suitable for pharmaceutical use, such as, for example, for use in humans and lower animals without undue irritation, allergic response and the like. Pharmaceutically acceptable salts of amines, carboxylic acids, and other types of compounds, are well known in the art. For example, S. M. Berge, et al., describe pharmaceutically acceptable salts in detail in J Pharmaceutical Sciences, 66: 1-19 (1977). The salts can be prepared in situ during the final isolation and purification of the cyclic oligopeptides disclosed herein, or separately by reacting a free base or free acid function with a suitable reagent, as described generally below. For example, a free base function can be reacted with a suitable acid. Suitable pharmaceutically acceptable salts can, include metal salts such as alkali metal salts, *e.g.,* sodium, potassium, and lithium salts; and alkaline earth metal salts, e.g., calcium or magnesium salts. Examples of pharmaceutically acceptable, nontoxic acid addition salts are salts of an amino group formed with inorganic acids such as hydrochloric acid, hydrobromic acid, hydroiodic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzoate, bisulfate, borate, butyrate, camphorate, camphorsulfonate, citrate, cyclopentanepropionate, digluconate, dodecylsulfate, formate, fumarate, glucoheptonate, glycerophosphate, gluconate, hernisulfate, heptanoate, hexanoate, hydroiodide, 2-hydroxyethanesulfonate, lactobionate, lactate, laurate, lauryl sulfate, malate, maleate, malonate, methanesulfonate, nicotinate, nitrate, oleate, oxalate, palmitate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like.

The term "residue," as used herein, refers to a position in an oligopeptide and its associated amino acid identity.

The terms "subject," "patient" and "individual" are used interchangeably and refer to mammals including, but not limited to, human patients and non-human primates, as well as experimental animals such as rabbits, dogs, cats, rats, mice, and other animals. Accordingly, the term "subject" or "patient" as used herein means any mammalian patient or subject to which the cyclic oligopeptides or related compositions of the disclosure can be administered. In some embodiments, the subject is a human patient. Subjects of the present invention include those with an IL-13-associated disorder or condition.

The terms "therapeutically effective amount," or "therapeutically effective dose" refer to an amount of a cyclic oligopeptide of the disclosure that will relieve, to some extent, one or more of the symptoms of an IL-13-associated skin disease or condition in a patient, e.g., effecting a beneficial and/or desirable alteration in the general health of a patient suffering from an IL-13-associated skin disorder or condition, as described herein. The full therapeutic effect does not necessarily occur by administration of one dose and may occur only after administration of a series of doses. Thus, a therapeutically effective amount may be administered in one or more administrations. The precise effective amount needed for a subject will depend upon, for example, the subject's size, health and age, the nature and extent of disease, the therapeutics or combination of therapeutics selected for administration, and the mode of administration. The skilled worker can readily determine the effective amount for a given situation by routine experimentation. The therapeutic methods or methods of treating described herein are not to be interpreted as or otherwise limited to "curing" the disease.

As used herein, the term "treat" and its cognates refer to reversing, reducing, or arresting the symptoms, clinical signs, and underlying pathology of an IL-13-associated skin disease or condition in manner to improve or stabilize a subject's condition. As used herein, and as well understood in the art, "treatment" is an approach for obtaining beneficial or desired results, including clinical results. Beneficial or desired results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, diminishment of the reoccurrence of disease, and remission (partially or completely). In some embodiments, the term "treat atopic dermatitis" refers to an approach for obtaining one or more beneficial or desired results including but not limited to, alleviation or amelioration of dry skin, itching, red to brownish-gray patches, small, raised bumps, thickened, cracked, scaly skin, and raw, sensitive, swollen skin from scratching. In some embodiments, the term "treat atopic dermatitis" refers to an approach to diminish extent of one or more symptoms of atopic dermatitis, wherein the one or more symptoms include but are not limited to dry skin, itching, red to brownish-gray patches, small, raised bumps, thickened, cracked, scaly skin, and raw, sensitive, swollen skin from scratching. In some embodiments, the term "treat atopic dermatitis" refers to an approach to stabilize (*i.e.* prevent the worsening) the state of one or more symptoms of atopic dermatitis, wherein the one or more symptoms include but are not limited to dry skin, itching, red to brownish-gray patches, small, raised bumps, thickened, cracked, scaly skin, and raw, sensitive, swollen skin from scratching. In some embodiments, the term "treat atopic dermatitis" refers to an approach to prevent spread of one or more symptoms of atopic dermatitis, wherein the one or more symptoms include but are not limited to dry skin, itching, red to brownish-gray patches, small, raised bumps, thickened, cracked, scaly skin, and raw, sensitive, swollen skin from scratching. In some embodiments, the term "treat atopic dermatitis" refers to an approach to delay or slow down progression of one or more symptoms of atopic dermatitis, wherein the one or more symptoms include but are not limited to dry skin, itching, red to brownish-gray patches, small, raised bumps, thickened, cracked, scaly skin, and raw, sensitive, swollen skin from scratching. In some embodiments, the term "treat atopic dermatitis" refers to an approach to partially or completely diminish the reoccurrence or remission of one or more symptoms of atopic dermatitis, wherein the one or more symptoms include but are not limited to dry skin, itching, red to brownish-gray patches, small, raised bumps, thickened, cracked, scaly skin, and raw, sensitive, swollen skin from scratching.

As used herein, the term "skin permeation rate" of a cyclic oligopeptide is measured using artificial skin (Start M, Merck), and refers to the percentage of the cyclic oligopeptide that penetrate the artificial skin after certain amount of time. For example, if 60% of the total amount of a cyclic oligopeptide of this disclosure has penetrated the artificial skin after 24 hours, then the skin permeation rate of the cyclic oligopeptide is 60% after 24 hours.

As used herein, the term "skin permeability coefficient (Kp)" refers to the rate at which the cyclic oligopeptide penetrates the skin, as measured using artificial skin (Start M, Merck). In some embodiments, permeability coefficient (Kp)(cm/hr) is calculated with the following equation: $permeability coefficient \left(Kp\right) = \frac{Q}{ATΔC} ;$ wherein Q(mg) refers to the total amount of cyclic oligopeptide that pass through the artificial skin within a certain amount of time; A(cm²) refers to the internal cross-section area of the artificial skin in a diffusion cell; T(hr) refers to the total amount of time of cyclic oligopeptide permeation; and ΔC(mg/mL) refers to the concentration difference of the cyclic oligopeptide between the two sides of the artificial skin. In some embodiments, the diffusion cell is a Franz Cell.

As used herein, the term "fast-acting" or "rapid-acting" refers to that an agent is able to penetrate skin at a relatively fast rate after application to the skin. In some embodiments, a fast-acting or a rapid-acting agent has a skin permeation rate of at least 10% after 1 hour. In some embodiments, a fast-acting or a rapid-acting agent has an average permeability coefficient of at least 1.0 x 10⁻² cm/hr after 1 hour. In some embodiments, a fast-acting or a rapid-acting agent has an average permeability coefficient of at least 2.0x 10⁻² cm/hr after 1 hour.

As used herein, the term "long-lasting delivery" refers to that an agent is able to penetrate skin through a relatively long period time after application to the skin. In some embodiments, a long-lasting delivery agent has a skin permeation rate of at least 25% after 24 hours. In some embodiments, a long-lasting delivery agent has an average permeability coefficient of at least 1.0 x 10⁻⁵ cm/hr after 24 hours. In some embodiments, a long-lasting delivery agent has an average permeability coefficient of at least 2.0 x 10⁻⁵ cm/hr after 24 hours.

### CYCLIC OLIGOPEPTIDES OF THE DISCLOSURE

In a first aspect, the present disclosure provides a cyclic oligopeptide that binds to IL-13. Without being bound by theory, the cyclic oligopeptides disclosed herein are advantageous for use as active ingredients in disease treatment and other contexts, because they have both the high specificity of biological molecules and the bioavailability of small molecules. In addition, these cyclic oligopeptides can be locally applied, with superior skin permeability, concentrating on skin areas that affected by an IL-13 associated disorder or conditions. Linear oligopeptides have been proven to be effective when used to target extracellular receptors, but their applications are limited by their instability due to degradation by proteases. The cyclic oligopeptides disclosed herein, which are conformationally rigid, are advantageous compares to their linear counterparts due, in part, to their improved target affinity and specificity, as well as improved stability due to their resistance to proteolysis.

In some embodiments, the cyclic oligopeptide that binds to IL-13 comprises between two and twenty-two amino acid residues. In some embodiments, the cyclic oligopeptide that binds to IL-13 comprises between four and ten amino acid residues. In some embodiments, the cyclic oligopeptide that binds to IL-13 comprises between six and eight amino acid residues. In some embodiments, the cyclic oligopeptide that binds to IL-13 comprises six amino acid residues. In some embodiments, the cyclic oligopeptide that binds to IL-13 comprises seven amino acid residues. In some embodiments, the cyclic oligopeptide that binds to IL-13 comprises eight amino acid residues.

In some embodiments, the cyclic oligopeptide that binds to IL-13 comprises a L-proline residue or a D-proline residue. According to the invention, the cyclic oligopeptide that binds to IL-13 comprises an amino acid sequence selected from the group consisting of SEQ ID NO:1-11. In some embodiments, the cyclic oligopeptide that binds to IL-13 comprises the amino acid sequence of SEQ ID NO:1. In some embodiments, the cyclic oligopeptide that binds to IL-13 comprises the amino acid sequence of SEQ ID NO:2. In some embodiments, the cyclic oligopeptide that binds to IL-13 comprises the amino acid sequence SEQ ID NO:3. In some embodiments, the cyclic oligopeptide that binds to IL-13 comprises the amino acid sequence of SEQ ID NO:4. In some embodiments, the cyclic oligopeptide that binds to IL-13 comprises the amino acid sequence of SEQ ID NO:5. In some embodiments, the cyclic oligopeptide that binds to IL-13 comprises the amino acid sequence of SEQ ID NO:6. In some embodiments, the cyclic oligopeptide that binds to IL-13 comprises the amino acid sequence of SEQ ID NO:7. In some embodiments, the cyclic oligopeptide that binds to IL-13 comprises the amino acid sequence of SEQ ID NO:8. In some embodiments, the cyclic oligopeptide that binds to IL-13 comprises the amino acid sequence of SEQ ID NO:9. In some embodiments, the cyclic oligopeptide that binds to IL-13 comprises the amino acid sequence of SEQ ID NO:10. In some embodiments, the cyclic oligopeptide that binds to IL-13 comprises the amino acid sequence of SEQ ID NO:11.

In some embodiments, the cyclic oligopeptide that binds to IL-13 has the structure of any one of Compounds 1-11, as defined herein (*see, e.g.,* Table 1). In some embodiments, the cyclic oligopeptide that binds to IL-13 has the structure of Compound 1, as defined herein. In some embodiments, the cyclic oligopeptide that binds to IL-13 has the structure of Compound 2, as defined herein. In some embodiments, the cyclic oligopeptide that binds to IL-13 has the structure of Compound 3, as defined herein. In some embodiments, the cyclic oligopeptide that binds to IL-13 has the structure of Compound 4, as defined herein. In some embodiments, the cyclic oligopeptide that binds to IL-13 has the structure of Compound 5, as defined herein. In some embodiments, the cyclic oligopeptide that binds to IL-13 has the structure of Compound 6, as defined herein. In some embodiments, the cyclic oligopeptide that binds to IL-13 has the structure of Compound 7, as defined herein. In some embodiments, the cyclic oligopeptide that binds to IL-13 has the structure of Compound 8, as defined herein. In some embodiments, the cyclic oligopeptide that binds to IL-13 has the structure of Compound 9, as defined herein. In some embodiments, the cyclic oligopeptide that binds to IL-13 has the structure of Compound 10, as defined herein. In some embodiments, the cyclic oligopeptide that binds to IL-13 has the structure of Compound 11, as defined herein.

In some embodiments, the cyclic oligopeptide that binds to IL-13 has the structure of any one of Compounds 1-11, as defined herein (*see, e.g.,* Table 1), or pharmaceutically acceptable salts thereof. In some embodiments, the cyclic oligopeptide that binds to IL-13 has the structure of Compound 1, as defined herein, or a pharmaceutically acceptable salt thereof. In some embodiments, the cyclic oligopeptide that binds to IL-13 has the structure of Compound 2, as defined herein, or a pharmaceutically acceptable salt thereof. In some embodiments, the cyclic oligopeptide that binds to IL-13 has the structure of Compound 3, as defined herein, or a pharmaceutically acceptable salt thereof. In some embodiments, the cyclic oligopeptide that binds to IL-13 has the structure of Compound 4, as defined herein, or a pharmaceutically acceptable salt thereof. In some embodiments, the cyclic oligopeptide that binds to IL-13 has the structure of Compound 5, as defined herein, or a pharmaceutically acceptable salt thereof. In some embodiments, the cyclic oligopeptide that binds to IL-13 has the structure of Compound 6, as defined herein, or a pharmaceutically acceptable salt thereof. In some embodiments, the cyclic oligopeptide that binds to IL-13 has the structure of Compound 7, as defined herein, or a pharmaceutically acceptable salt thereof. In some embodiments, the cyclic oligopeptide that binds to IL-13 has the structure of Compound 8, as defined herein, or a pharmaceutically acceptable salt thereof. In some embodiments, the cyclic oligopeptide that binds to IL-13 has the structure of Compound 9, as defined herein, or a pharmaceutically acceptable salt thereof. In some embodiments, the cyclic oligopeptide that binds to IL-13 has the structure of Compound 10, as defined herein, or a pharmaceutically acceptable salt thereof. In some embodiments, the cyclic oligopeptide that binds to IL-13 has the structure of Compound 11, as defined herein, or a pharmaceutically acceptable salt thereof.

In some embodiments, the cyclic oligopeptide that binds to IL-13 is lipidated. In some embodiments, the cyclic oligopeptide that binds to IL-13 is PEGylated.

In some embodiments, the cyclic oligopeptide that binds to IL-13 has a skin permeation rate of at least 25% after 24 hours. In some embodiments, the cyclic oligopeptide that binds to IL-13 has a skin permeation rate of at least 30% after 24 hours. In some embodiments, the cyclic oligopeptide that binds to IL-13 has a skin permeation rate of at least 35% after 24 hours. In some embodiments, the cyclic oligopeptide that binds to IL-13 has a skin permeation rate of at least 40% after 24 hours. In some embodiments, the cyclic oligopeptide that binds to IL-13 has a skin permeation rate of at least 45% after 24 hours. In some embodiments, the cyclic oligopeptide that binds to IL-13 has a skin permeation rate of at least 50% after 24 hours. In some embodiments, the cyclic oligopeptide that binds to IL-13 has a skin permeation rate of at least 55% after 24 hours. In some embodiments, the cyclic oligopeptide that binds to IL-13 has a skin permeation rate of at least 60% after 24 hours. In some embodiments, the cyclic oligopeptide that binds to IL-13 has a skin permeation rate of not more than 65% after 24 hours. In some embodiments, the cyclic oligopeptide that binds to IL-13 has a skin permeation rate of about 25% after 24 hours. In some embodiments, the cyclic oligopeptide that binds to IL-13 has a skin permeation rate of about 30% after 24 hours. In some embodiments, the cyclic oligopeptide that binds to IL-13 has a skin permeation rate of about 35% after 24 hours. In some embodiments, the cyclic oligopeptide that binds to IL-13 has a skin permeation rate of about 40% after 24 hours. In some embodiments, the cyclic oligopeptide that binds to IL-13 has a skin permeation rate of about 45% after 24 hours. In some embodiments, the cyclic oligopeptide that binds to IL-13 has a skin permeation rate of about 50% after 24 hours. In some embodiments, the cyclic oligopeptide that binds to IL-13 has a skin permeation rate of about 55% after 24 hours. In some embodiments, the cyclic oligopeptide that binds to IL-13 has a skin permeation rate of about 60% after 24 hours.

In some embodiments, the cyclic oligopeptide that binds to IL-13 has a skin permeation rate of between 25% and 60% after 24 hours. In some embodiments, the cyclic oligopeptide that binds to IL-13 has a skin permeation rate of between 25% and 55% after 24 hours. In some embodiments, the cyclic oligopeptide that binds to IL-13 has a skin permeation rate of between 25% and 50% after 24 hours. In some embodiments, the cyclic oligopeptide that binds to IL-13 has a skin permeation rate of between 25% and 45% after 24 hours. In some embodiments, the cyclic oligopeptide that binds to IL-13 has a skin permeation rate of between 25% and 40% after 24 hours. In some embodiments, the cyclic oligopeptide that binds to IL-13 has a skin permeation rate of between 25% and 35% after 24 hours. In some embodiments, the cyclic oligopeptide that binds to IL-13 has a skin permeation rate of between 25% and 30% after 24 hours. In some embodiments, the cyclic oligopeptide that binds to IL-13 has a skin permeation rate of between 30% and 60% after 24 hours. In some embodiments, the cyclic oligopeptide that binds to IL-13 has a skin permeation rate of between 35% and 60% after 24 hours. In some embodiments, the cyclic oligopeptide that binds to IL-13 has a skin permeation rate of between 40% and 60% after 24 hours. In some embodiments, the cyclic oligopeptide that binds to IL-13 has a skin permeation rate of between 45% and 60% after 24 hours. In some embodiments, the cyclic oligopeptide that binds to IL-13 has a skin permeation rate of between 50% and 60% after 24 hours. In some embodiments, the cyclic oligopeptide that binds to IL-13 has a skin permeation rate of between 55% and 60% after 24 hours. In some embodiments, the cyclic oligopeptide that binds to IL-13 has a skin permeation rate of between 30% and 50% after 24 hours. In some embodiments, the cyclic oligopeptide that binds to IL-13 has a skin permeation rate of between 35% and 45% after 24 hours. In some embodiments, the cyclic oligopeptide that binds to IL-13 has a skin permeation rate of between 30% and 40% after 24 hours. In some embodiments, the cyclic oligopeptide that binds to IL-13 has a skin permeation rate of between 35% and 40% after 24 hours. In some embodiments, the cyclic oligopeptide that binds to IL-13 has a skin permeation rate of between 40% and 45% after 24 hours.

In some embodiments, the cyclic oligopeptide that binds to IL-13 has a skin permeation rate of at least 10% after 1 hour. In some embodiments, the cyclic oligopeptide that binds to IL-13 has a skin permeation rate of at least 15% after 1 hour. In some embodiments, the cyclic oligopeptide that binds to IL-13 has a skin permeation rate of at least 20% after 1 hour. In some embodiments, the cyclic oligopeptide that binds to IL-13 has a skin permeation rate of at least 25% after 1 hour. In some embodiments, the cyclic oligopeptide that binds to IL-13 has a skin permeation rate of not more than 30% after 1 hour. In some embodiments, the cyclic oligopeptide that binds to IL-13 has a skin permeation rate of about 10% after 1 hour. In some embodiments, the cyclic oligopeptide that binds to IL-13 has a skin permeation rate of about 15% after 1 hour. In some embodiments, the cyclic oligopeptide that binds to IL-13 has a skin permeation rate of about 20% after 1 hour. In some embodiments, the cyclic oligopeptide that binds to IL-13 has a skin permeation rate of about 25% after 1 hour.

In some embodiments, the cyclic oligopeptide that binds to IL-13 has a skin permeation rate of between 10% and 25% after 1 hour. In some embodiments, the cyclic oligopeptide that binds to IL-13 has a skin permeation rate of between 10% and 20% after 1 hour. In some embodiments, the cyclic oligopeptide that binds to IL-13 has a skin permeation rate of between 10% and 15% after 1 hour. In some embodiments, the cyclic oligopeptide that binds to IL-13 has a skin permeation rate of between 15% and 25% after 1 hour. In some embodiments, the cyclic oligopeptide that binds to IL-13 has a skin permeation rate of between 20% and 25% after 1 hour. In some embodiments, the cyclic oligopeptide that binds to IL-13 has a skin permeation rate of between 15% and 20% after 1 hour.

In some embodiments, the cyclic oligopeptide that binds to IL-13 has an average skin permeability coefficient of at least 1.0 x 10⁻⁵ cm/hr after 24 hours. In some embodiments, the cyclic oligopeptide that binds to IL-13 has an average skin permeability coefficient of at least 2.0 x 10⁻⁵ cm/hr after 24 hours. In some embodiments, the cyclic oligopeptide that binds to IL-13 has an average skin permeability coefficient of at least 5.0 x 10⁻⁵ cm/hr after 24 hours. In some embodiments, the cyclic oligopeptide that binds to IL-13 has an average skin permeability coefficient of at least 1.0 x 10⁻⁴ cm/hr after 24 hours. In some embodiments, the cyclic oligopeptide that binds to IL-13 has an average skin permeability coefficient of at least 2.0 x 10⁻⁴ cm/hr after 24 hours. In some embodiments, the cyclic oligopeptide that binds to IL-13 has an average skin permeability coefficient of at least 3.0 x 10⁻⁴ cm/hr after 24 hours. In some embodiments, the cyclic oligopeptide that binds to IL-13 has an average skin permeability coefficient of at least 4.0 x 10⁻⁴ cm/hr after 24 hours. In some embodiments, the cyclic oligopeptide that binds to IL-13 has an average skin permeability coefficient of not more than 5.0 x 10⁻⁴ cm/hr after 24 hours. In some embodiments, the cyclic oligopeptide that binds to IL-13 has an average skin permeability coefficient of about 1.0 x 10⁻⁵ cm/hr after 24 hours. In some embodiments, the cyclic oligopeptide that binds to IL-13 has an average skin permeability coefficient of about 2.0 x 10⁻⁵ cm/hr after 24 hours. In some embodiments, the cyclic oligopeptide that binds to IL-13 has an average skin permeability coefficient of about 5.0 x 10⁻⁵ cm/hr after 24 hours. In some embodiments, the cyclic oligopeptide that binds to IL-13 has an average skin permeability coefficient of about 1.0 x 10⁻⁴ cm/hr after 24 hours. In some embodiments, the cyclic oligopeptide that binds to IL-13 has an average skin permeability coefficient of about 2.0 x 10⁻⁴ cm/hr after 24 hours. In some embodiments, the cyclic oligopeptide that binds to IL-13 has an average skin permeability coefficient of about 3.0 x 10⁻⁴ cm/hr after 24 hours. In some embodiments, the cyclic oligopeptide that binds to IL-13 has an average skin permeability coefficient of about 4.0 x 10⁻⁴ cm/hr after 24 hours. In some embodiments, the cyclic oligopeptide that binds to IL-13 has an average skin permeability coefficient of about 4.5 x 10⁻⁴ cm/hr after 24 hours.

In some embodiments, the cyclic oligopeptide that binds to IL-13 has an average skin permeability coefficient of between 1.0 x 10⁻⁵ cm/hr and 5.0 x 10⁻⁴ cm/hr after 24 hours. In some embodiments, the cyclic oligopeptide that binds to IL-13 has an average skin permeability coefficient of between 5.0 x 10⁻⁵ cm/hr and 5.0 x 10⁻⁴ cm/hr after 24 hours. In some embodiments, the cyclic oligopeptide that binds to IL-13 has an average skin permeability coefficient of between 1.0 x 10⁻⁴ cm/hr and 5.0 x 10⁻⁴ cm/hr after 24 hours. In some embodiments, the cyclic oligopeptide that binds to IL-13 has an average skin permeability coefficient of between 1.0 x 10⁻⁵ cm/hr and 1.0 x 10⁻⁴ cm/hr after 24 hours. In some embodiments, the cyclic oligopeptide that binds to IL-13 has an average skin permeability coefficient of between 1.0 x 10⁻⁵ cm/hr and 5.0 x 10⁻⁵ cm/hr after 24 hours. In some embodiments, the cyclic oligopeptide that binds to IL-13 has an average skin permeability coefficient of between 5.0 x 10⁻⁵ cm/hr and 1.0 x 10⁻⁴ cm/hr after 24 hours.

In some embodiments, the cyclic oligopeptide that binds to IL-13 has an average skin permeability coefficient of at least 1.0 x 10⁻² cm/hr after 1 hour. In some embodiments, the cyclic oligopeptide that binds to IL-13 has an average skin permeability coefficient of at least 2.0 x 10⁻² cm/hr after 1 hour. In some embodiments, the cyclic oligopeptide that binds to IL-13 has an average skin permeability coefficient of at least 3.0 x 10⁻² cm/hr after 1 hour. In some embodiments, the cyclic oligopeptide that binds to IL-13 has an average skin permeability coefficient of at least 4.0 x 10⁻² cm/hr after 1 hour. In some embodiments, the cyclic oligopeptide that binds to IL-13 has an average skin permeability coefficient of at least 5.0 x 10⁻² cm/hr after 1 hour. In some embodiments, the cyclic oligopeptide that binds to IL-13 has an average skin permeability coefficient of not more than 6.0 x 10⁻² cm/hr after 1 hour. In some embodiments, the cyclic oligopeptide that binds to IL-13 has an average skin permeability coefficient of about 1.0 x 10⁻² cm/hr after 1 hour. In some embodiments, the cyclic oligopeptide that binds to IL-13 has an average skin permeability coefficient of about 2.0 x 10⁻² cm/hr after 1 hour. In some embodiments, the cyclic oligopeptide that binds to IL-13 has an average skin permeability coefficient of about 3.0 x 10⁻² cm/hr after 1 hour. In some embodiments, the cyclic oligopeptide that binds to IL-13 has an average skin permeability coefficient of about 4.0 x 10⁻² cm/hr after 1 hour. In some embodiments, the cyclic oligopeptide that binds to IL-13 has an average skin permeability coefficient of about 5.0 x 10⁻² cm/hr after 1 hour. In some embodiments, the cyclic oligopeptide that binds to IL-13 has an average skin permeability coefficient of about 5.5 x 10⁻² cm/hr after 1 hour.

In some embodiments, the cyclic oligopeptide that binds to IL-13 has an average skin permeability coefficient of between 1.0 x 10⁻² cm/hr and 5.0 x 10⁻² cm/hr after 1 hour. In some embodiments, the cyclic oligopeptide that binds to IL-13 has an average skin permeability coefficient of between 2.0 x 10⁻² cm/hr and 5.0 x 10⁻² cm/hr after 1 hour. In some embodiments, the cyclic oligopeptide that binds to IL-13 has an average skin permeability coefficient of between 3.0 x 10⁻² cm/hr and 5.0 x 10⁻² cm/hr after 1 hour. In some embodiments, the cyclic oligopeptide that binds to IL-13 has an average skin permeability coefficient of between 4.0 x 10⁻² cm/hr and 5.0 x 10⁻² cm/hr after 1 hour. In some embodiments, the cyclic oligopeptide that binds to IL-13 has an average skin permeability coefficient of between 1.0 x 10⁻² cm/hr and 4.0 x 10⁻² cm/hr after 1 hour. In some embodiments, the cyclic oligopeptide that binds to IL-13 has an average skin permeability coefficient of between 1.0 x 10⁻² cm/hr and 3.0 x 10⁻² cm/hr after 1 hour. In some embodiments, the cyclic oligopeptide that binds to IL-13 has an average skin permeability coefficient of between 1.0 x 10⁻² cm/hr and 2.0 x 10⁻² cm/hr after 1 hour. In some embodiments, the cyclic oligopeptide that binds to IL-13 has an average skin permeability coefficient of between 2.0 x 10⁻² cm/hr and 4.0 x 10⁻² cm/hr after 1 hour. In some embodiments, the cyclic oligopeptide that binds to IL-13 has an average skin permeability coefficient of between 3.0 x 10⁻² cm/hr and 4.0 x 10⁻² cm/hr after 1 hour. In some embodiments, the cyclic oligopeptide that binds to IL-13 has an average skin permeability coefficient of between 2.0 x 10⁻² cm/hr and 3.0 x 10⁻² cm/hr after 1 hour.

### PHARMACEUTICAL COMPOSITIONS AND ADMINISTRATION

In a second aspect, the present disclosure provides a pharmaceutical formulation comprising a cyclic oligopeptide disclosed herein and a pharmaceutically acceptable carrier. In some embodiments, the pharmaceutical composition comprises a therapeutically effective amount of the cyclic oligopeptide described herein.

The pharmaceutical compositions may be in a variety of forms, for example, liquid, semi-solid and solid dosage forms, such as liquid solutions (e.g., injectable and infusible solutions), dispersions or suspensions, powders, capsules, pills, tablets and liposomes. The preferred form depends on the intended mode of administration and therapeutic application. In some embodiments, the pharmaceutical compositions are in the form of injectable or infusible solutions. Optionally, mode of administration is topical. The pharmaceutical composition may be administered by intravenous infusion or injection. In some embodiments, the pharmaceutical composition is formulated for subcutaneous injection. Optionally, the pharmaceutical composition is formulated for transdermal administration. In some embodiments, the cyclic oligopeptide is formulated in a tape or a patch. Formulations for injection may be presented in unit dosage form, e.g., in ampoules, pre-filled syringes, or in multi-dose containers, with or without an added preservative. The pharmaceutical compositions may take such forms as suspensions, solutions, or emulsions in oily or aqueous vehicles, and may contain formulatory agents such as suspending, stabilizing and/or dispersing agents. Alternatively, the active ingredient may be prepared in powder form for reconstitution with a suitable vehicle, e.g., sterile pyrogen-free water, before use.

Therapeutic compositions typically must be sterile and stable under the conditions of manufacture and storage. The composition may be formulated as a solution, microemulsion, dispersion, liposome, or other ordered structure suitable to high drug concentration. Sterile injectable solutions may be prepared by incorporating the cyclic oligopeptide in the required amount in an appropriate solvent with one or a combination of ingredients enumerated above, as required, followed by filtered sterilization. Dispersions may be prepared by incorporating the cyclic oligopeptide into a sterile vehicle that contains a basic dispersion medium and the required other ingredients from those enumerated above. In the case of sterile powders for the preparation of sterile injectable or topical solutions, the preferred methods of preparation include vacuum drying and freeze drying that yield a powder of the cyclic oligopeptide and any additional desired ingredient from a previously sterile filtered solution thereof. The proper fluidity of a solution can be maintained, for example, by the use of a coating such as lecithin, by the maintenance of the required particle size in the case of dispersion and/or by the use of surfactants. Prolonged absorption of injectable compositions can be brought about by including in the composition an agent that delays absorption, for example, monostearate salts and gelatin.

The pharmaceutical compositions may be administered by a variety of methods known in the art. In some embodiments, the route/mode of administration is topical, transdermal, subcutaneous injection, intravenous infusion or oral. In some embodiments, the route/mode of administration is topical, transdermal, or subcutaneous injection. In some embodiments, the route/mode of administration is topical. In some embodiments, the route/mode of administration is transdermal. In some embodiments, the route/mode of administration is subcutaneous injection. In some embodiments, the route/mode of administration is intravenous infusion. In some embodiments, the route/mode of administration is oral. As will be appreciated by the skilled artisan, the route and/or mode of administration will vary depending upon the desired results.

Dosage regimens may be adjusted to provide the optimum desired response (e.g., a therapeutic or prophylactic response). For example, a single bolus can be administered, several divided doses can be administered over time or the dose can be proportionally reduced or increased as indicated by the exigencies of the therapeutic situation. It may be advantageous to formulate pharmaceutical compositions in dosage unit form for ease of administration and uniformity of dosage. The specification for the dosage unit forms may be dictated by and directly dependent on (a) the unique characteristics of the cyclic oligopeptide and the particular therapeutic or prophylactic effect to be achieved, and (b) the limitations inherent in the art of compounding such a cyclic oligopeptide for the treatment of sensitivity in individuals. Dosage values may vary with the type and severity of the condition to be alleviated. For any particular subject, specific dosage regimens may be adjusted over time according to the individual need and the professional judgment of the person administering or supervising the administration of the compositions, and that dosage ranges set forth herein are exemplary only and are not intended to limit the scope or practice of the paragraphed composition.

### METHODS OF TREATING IL-13-ASSOCIATED SKIN DISEASES AND CONDITIONS

In a third aspect, the present disclosure provides a method of treating or preventing an IL-13-associated skin disorder or condition in a subject in need thereof. In some embodiments, the method comprises administering to the subject a therapeutically effective amount of any one of the cyclic oligopeptides disclosed herein or a combination thereof. In some embodiments, the subject is a human. The cyclic oligopeptide may be administered as a pharmaceutical composition disclosed herein. The cyclic oligopeptide may be administered according to any of the routes of administration disclosed herein.

According to the invention, the cyclic oligopeptide used for treatment comprises an amino acid sequence selected from the group consisting of SEQ ID NO:1-11 . In some embodiments, the cyclic oligopeptide used for treatment comprises the amino acid sequence of D-ARG D-PHE D-VAL TYR GLU PRO (SEQ ID NO:1). Optionally, the cyclic oligopeptide used for treatment comprises the amino acid sequence of ARG THR D-VAL GLU D-PHE D-PRO (SEQ ID NO:2). The cyclic oligopeptide used for treatment may comprise the amino acid sequence of GLU D-THR D-VAL TRP D-PRO D-PRO (SEQ ID NO:3). In some embodiments, the cyclic oligopeptide used for treatment comprises the amino acid sequence of ARG GLU D-VAL TRP D-PRO D-PRO (SEQ ID NO:4). Optionally, the cyclic oligopeptide used for treatment comprises the amino acid sequence of TRP VAL ARG GLU D-PRO D-PRO (SEQ ID NO:5). The cyclic oligopeptide used for treatment may comprise the amino acid sequence of TYR ARG GLU D-THR D-VAL D-PRO (SEQ ID NO:6). In some embodiments, the cyclic oligopeptide used for treatment comprises the amino acid sequence of THR ARG D-PHE PRO D-LEU D-PRO (SEQ ID NO:7). Optionally, the cyclic oligopeptide used for treatment comprises the amino acid sequence of D-LEU ARG GLU PRO TRP MET PRO (SEQ ID NO:8). The cyclic oligopeptide used for treatment may comprise the amino acid sequence of D-ARG LEU D-TRP TRP D-THR GLU PRO (SEQ ID NO:9). In some embodiments, the cyclic oligopeptide used for treatment comprises the amino acid sequence of ARG ASP TYR CYS D-PRO D-TRP D-PRO (SEQ ID NO:10). Optionally, the cyclic oligopeptide used for treatment comprises the amino acid sequence of VAL PRO D-LEU D-TRP D-VAL LEU ARG PRO (SEQ ID NO:11).

In some embodiments, the cyclic oligopeptide used for treatment has the structure of any one of Compounds 1-11, as defined herein (*see, e.g.,* Table 1). In some embodiments, the cyclic oligopeptide used for treatment has the structure of Compound 1, as defined herein. Optionally, the cyclic oligopeptide used for treatment has the structure of Compound 2, as defined herein. The cyclic oligopeptide used for treatment may have the structure of Compound 3, as defined herein. In some embodiments, the cyclic oligopeptide used for treatment has the structure of Compound 4, as defined herein. Optionally, the cyclic oligopeptide used for treatment has the structure of Compound 5, as defined herein. The cyclic oligopeptide used for treatment may have the structure of Compound 6, as defined herein. In some embodiments, the cyclic oligopeptide used for treatment has the structure of Compound 7, as defined herein. Optionally, the cyclic oligopeptide used for treatment has the structure of Compound 8, as defined herein. The cyclic oligopeptide used for treatment may have the structure of Compound 9, as defined herein. In some embodiments, the cyclic oligopeptide used for treatment has the structure of Compound 10, as defined herein. Optionally, the cyclic oligopeptide used for treatment has the structure of Compound 11, as defined herein.

In some embodiments, the cyclic oligopeptide used for treatment has the structure of any one of Compounds 1-11, as defined herein (*see, e.g.,* Table 1), or pharmaceutically acceptable salts thereof. In some embodiments, the cyclic oligopeptide used for treatment has the structure of Compound 1, as defined herein, or a pharmaceutically acceptable salt thereof. Optionally, the cyclic oligopeptide used for treatment has the structure of Compound 2, as defined herein, or a pharmaceutically acceptable salt thereof. The cyclic oligopeptide used for treatment may have the structure of Compound 3, as defined herein, or a pharmaceutically acceptable salt thereof. In some embodiments, the cyclic oligopeptide used for treatment has the structure of Compound 4, as defined herein, or a pharmaceutically acceptable salt thereof. Optionally, the cyclic oligopeptide used for treatment has the structure of Compound 5, as defined herein, or a pharmaceutically acceptable salt thereof. The cyclic oligopeptide used for treatment may have the structure of Compound 6, as defined herein, or a pharmaceutically acceptable salt thereof. In some embodiments, the cyclic oligopeptide used for treatment has the structure of Compound 7, as defined herein, or a pharmaceutically acceptable salt thereof. Optionally, the cyclic oligopeptide used for treatment has the structure of Compound 8, as defined herein, or a pharmaceutically acceptable salt thereof. The cyclic oligopeptide used for treatment may have the structure of Compound 9, as defined herein, or a pharmaceutically acceptable salt thereof. In some embodiments, the cyclic oligopeptide used for treatment has the structure of Compound 10, as defined herein, or a pharmaceutically acceptable salt thereof. Optionally, the cyclic oligopeptide used for treatment has the structure of Compound 11, as defined herein, or a pharmaceutically acceptable salt thereof.

In some embodiments, the IL-13-associated skin disorder or condition is an inflammatory, an allergic, or an autoimmune disorder or condition. In some embodiments, the IL-13-associated skin disorder or condition is an inflammatory disorder or condition. In some embodiments, the IL-13-associated skin disorder or condition is an allergic disorder or condition. In some embodiments, the IL-13-associated skin disorder or condition is an autoimmune disorder or condition. In some embodiments, the IL-13-associated skin disorder or condition is selected from the group consisting of: atopic dermatitis, allergic contact dermatitis, urticaria, eczema, chronic hand eczema, bullous diseases (bullous pemphigoid), alopecia areata, and prurigo and molluscum contagiosum. In some embodiments, the IL-13-associated skin disorder or condition is atopic dermatitis. In some embodiments, the IL-13-associated skin disorder or condition is allergic contact dermatitis. In some embodiments, the IL-13-associated skin disorder or condition is urticaria. In some embodiments, the IL-13-associated skin disorder or condition is eczema. In some embodiments, the IL-13-associated skin disorder or condition is chronic hand eczema. In some embodiments, the IL-13-associated skin disorder or condition is bullous diseases (bullous pemphigoid). In some embodiments, the IL-13-associated skin disorder or condition is alopecia areata. In some embodiments, the IL-13-associated skin disorder or condition is prurigo. In some embodiments, the IL-13-associated skin disorder or condition is molluscum contagiosum.

In some embodiments, the one or more cyclic oligopeptides bind to IL-13 and reduces biological activities of IL-13 and/or an IL-13 receptor (IL-13R). In some embodiments, the one or more cyclic oligopeptides bind to IL-13 and reduces biological activities of IL-13. In some embodiments, the one or more cyclic oligopeptides bind to IL-13 and reduces biological activities of an IL-13 receptor (IL-13R). In some embodiments, the one or more cyclic oligopeptides bind to IL-13 and reduces biological activities of IL-13 or an IL-13 receptor (IL-13R). In some embodiments, the one or more cyclic oligopeptides bind to IL-13 and reduces biological activities of IL-13 and an IL-13 receptor (IL-13R).

In some embodiments, the present disclosure provides a method of treating or preventing an IL-13-associated skin disorder or condition in a subject in need thereof, comprising administering to the subject a therapeutically effective amount of any one of the cyclic oligopeptides of this disclosure or a combination thereof. In some embodiments, the cyclic oligopeptide or the combination thereof is administered topically. In some embodiments, the cyclic oligopeptide or the combination thereof is administered transdermally. In some embodiments, the cyclic oligopeptide or the combination thereof is administered subcutaneously. In some embodiments, the cyclic oligopeptide or the combination thereof is administered intravenously. In some embodiments, the cyclic oligopeptide or the combination thereof is administered orally. Methods for oral administration of biologically active proteins and peptides are known in the art. A number of strategies for preventing degradation of orally administered peptides have been suggested. Examples of methods for oral administration of the cyclic oligopeptide include, but are not limited to, the use of core-shell particles (US 7,090,868) and nanotubes (US 7,195,780); liposomes and aqueous emulsions and suspensions (US 7,316,818; WO 06/062544; US 6,071,535; and US 5,874,105); gas-filled liposomes (US 6,551,576; US 6,808,720; and US 7,083,572); nanodroplets dispersed in an aqueous medium (US 2007/0184076); matrix-carriers containing peptide-effectors that provide penetration across biological barriers for administration of hydrophobic proteins (WO 06/097793, WO 05/094785, and WO 03/066859); use of non-covalent protein-polysaccharide complexes (EP0491114B1); use of pharmaceutical compositions described in US 8,936,786; use of Peptelligence^{®} system (US 8,377,863; WO 2014/138241; and WO 2016/115082).

### METHODS OF PRODUCING CYCLIC OLIGOPEPTIDES

In a fourth aspect, the present disclosure provides a method of producing a cyclic oligopeptide that binds to IL-13. In some embodiments, the methods comprises the steps of: (1) contacting FMOC (9-fluorenylmethyloxycarbonyl)-D-Pro-2cl-resin or FMOC-Pro-2cl-resin with a first amino acid residue under conditions suitable to form a peptide bond between the first amino acid residue and the resin-bound D-Pro or Pro to form a resin-bound oligopeptide; (2) washing the resin-bound oligopeptide to remove any unbound amino acid residue; (3) contacting the resin-bound oligopeptide with an additional amino acid residue under conditions suitable to form a peptide bond between the additional amino acid residue and the resin-bound oligopeptide; (4) washing the resin-bound oligopeptide to remove any unbound amino acid residue; (5) removing the resin from the oligopeptide; and (6) cyclizing the oligopeptide.

In some embodiments, the conditions suitable to form a peptide bond in steps (1) and (3) include the addition of O-(Benzotriazol-1-yl)-N, N, N', N'-tetramethyluronium tetrafluoroborate (TBTU). In some embodiments, the conditions suitable to form a peptide bond in steps (1) and (3) further include the addition of N,N-Dimethylformamide (DMF) and N,N-Diisopropylethylamine (DIEA). In some embodiments, steps (3) and (4) are repeated between 1 and 20 times before the resin is removed from the oligopeptide.

In some embodiments, the cyclizing step comprises the carboxyl group of the D-Pro or Pro residue forming a covalent bond with one or more amino groups of the linear oligopeptide. In some embodiments, the cyclizing step comprises the addition of DMF, PyBOP (Benzotriazol-1-yl-oxytripyrrolidinophosphonium hexafluorophosphate), HOBT (1-Hydroxybenzotriazole), and DIEA to the linear oligopeptide and incubation of the mixture thereof at room temperature.

### EXAMPLES

Exemplary methods and materials are described herein, although methods and materials similar or equivalent to those described herein can also be used in the practice or testing of the various aspects and embodiments of the disclosure. The materials, methods, and examples are illustrative only and not intended to be limiting.

### Example 1. Production of cyclic oligopeptides that bind to IL-13

Cyclic oligopeptides of the disclosure were produced using the following steps:
1) FMOC (9-fluorenylmethyloxycarbonyl)-D-Pro-2cl-resin or FMOC-Pro-2cl-resin (selected according to the sequence and structure of oligopeptides to be produced) was weighed and placed into a glass reaction column, and Dichloromethane (methylene chloride or DCM) was added and incubated for 30 minutes to allow the resin to swell.
2) DCM was removed by vacuum.
3) The resin 3 was washed times with N,N-Dimethylformamide (DMF).
4) 20% piperidine/DMF solution was added and incubated for 20 minutes to remove the FMOC protective group.
5) The liquid solution was removed by vacuum, and the resin was washed 6 times with DMF.
6) The second amino acid, which would be added to the N-terminus of D-Pro or Pro, and TBTU (O-(Benzotriazol-1-yl)-N, N, N', N'-tetramethyluronium tetrafluoroborate) were weighed and added to the resin.
7) DIEA (N,N-Diisopropylethylamine) was dissolved in DMF and added to the resin and react for 30 minutes. The progress of the reaction was followed using a color detection reaction. The reaction was determined to be complete when the solution was bright yellow, and the resin was yellow.
8) The solvent was removed by vacuum.
9) Steps 6) to 8) were repeated to add each additional amino acid residue to the N-terminus of the growing oligopeptide until the last amino acid residue was added to the oligopeptide. The last amino acid residue is the residue that was subsequently cyclized with the C-terminus of the initial D-Pro or Pro residue, as shown in the structures Table 1, below.
10) The resin was washed three times with DMF, DCM, and methanol and dried by vacuum.
11) Full protection lysis solution was added, and the resin was removed by filtering the solution with sand core.
12) 0.5ml DIEA (N,N-Diisopropylethylamine) was added to the passthrough.
13) The DCM was removed from the passthrough by spin-drying with a vacuum rotary evaporator until the solution became viscous. Analytically pure DMF, PyBOP (Benzotriazol-1-yl-oxytripyrrolidinophosphonium hexafluorophosphate), HOBT (1-Hydroxybenzotriazole), and DIEA were added and incubated for 4 hours at room temperature.
14) After the reaction is complete, an acidic solution was added to separate out the solid, which was dried under vacuum.
15) A lysis solution was added to remove the protective groups from the amino acid side chains.
16) Ether was added to the solution to separate out solid products, which were centrifuged and washed three times and then dried under vacuum.

The synthesized cyclic oligopeptides were analyzed for purity and structural characteristics by MS (Mass Spectrometry) (*see,* FIG. 2A, 3A, 4A, 5A, 6A, 7A, 8A, 9A, 10A, 11A, and 12A) and HPLC (*see,* FIG. 2B, 3B, 4B, 5B, 6B, 7B, 8B, 9B, 10B, 11B, and 12B).Exemplary structure and amino acid sequences of cyclic oligopeptides that bind to IL-13 are illustrated in Table 1.

**Table 1. Structure and amino acid sequence of cyclic oligopeptides**

| SEQ ID NO: | Structure | Amino Acid Sequence |
|---|---|---|
| 1 | 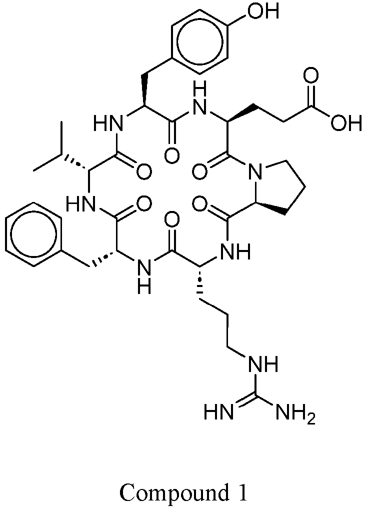 | D-Arg D-Phe D- Val Tyr Glu Pro (SEQ ID NO: 1) |
| 2 | 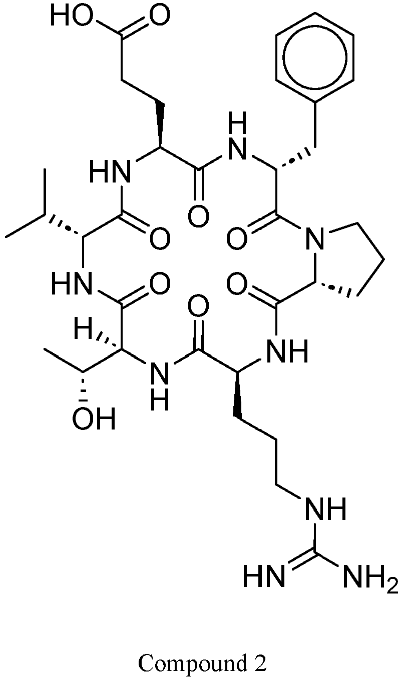 | Arg Thr D-Val Glu D-Phe D-Pro (SEQ ID NO: 2) |
| 3 | 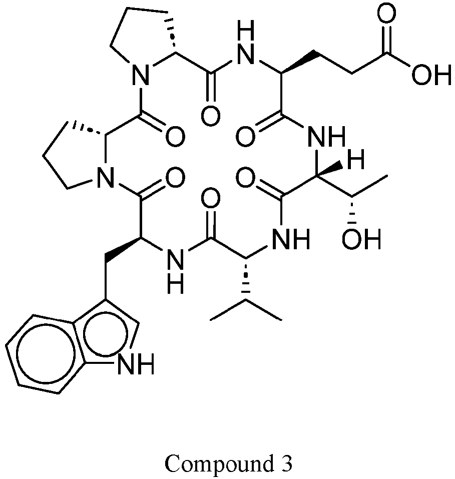 | Glu D-Thr D-Val Trp D-Pro D-Pro (SEQ ID NO: 3) |
| 4 | 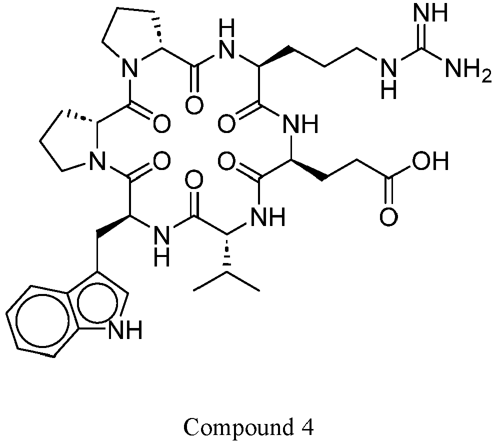 | Arg Glu D-Val Trp D-Pro D-Pro (SEQ ID NO: 4) |
| 5 | 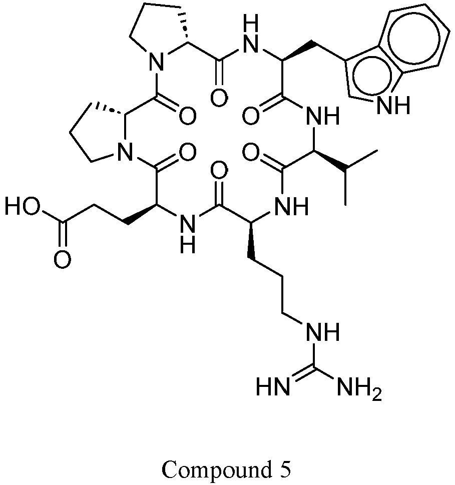 | Trp Val Arg Glu D-Pro D-Pro (SEQ ID NO: 5) |
| 6 | 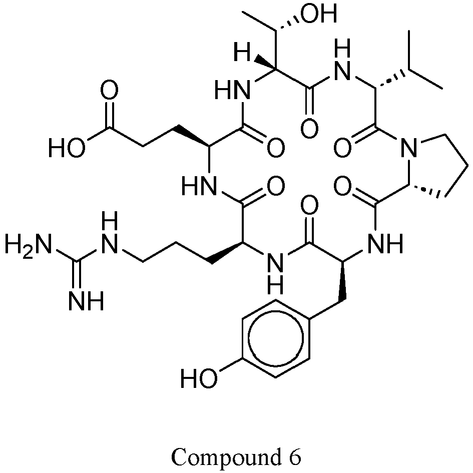 | Tyr Arg Glu D- Thr D-Val D-Pro (SEQ ID NO: 6) |
| 7 | 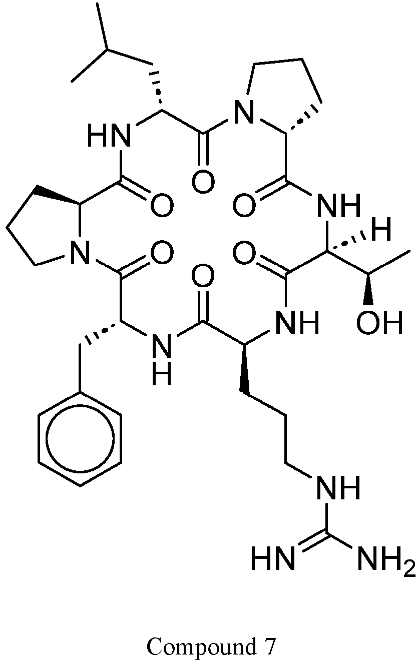 | Thr Arg D-Phe Pro D-Leu D-Pro (SEQ ID NO: 7) |
| 8 | 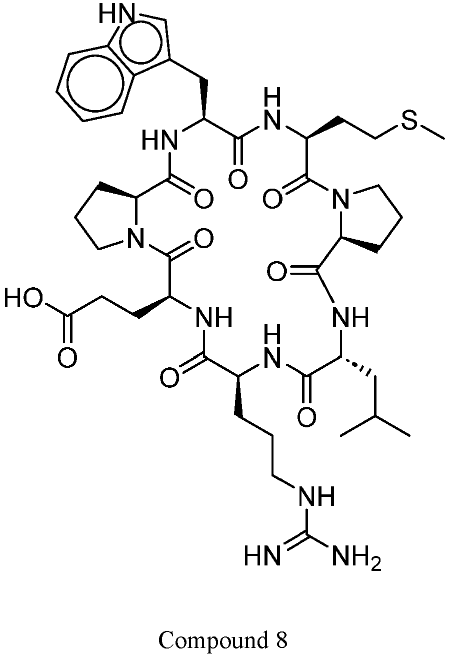 | D-Leu Arg Glu Pro Trp Met Pro (SEQ ID NO: 8) |
| 9 | 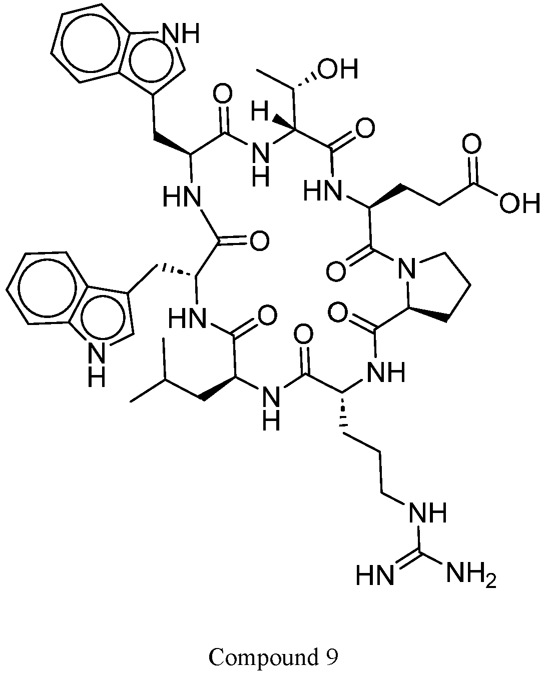 | D-Arg Leu D-Trp Trp D-Thr Glu Pro (SEQ ID NO: 9) |
| 10 | 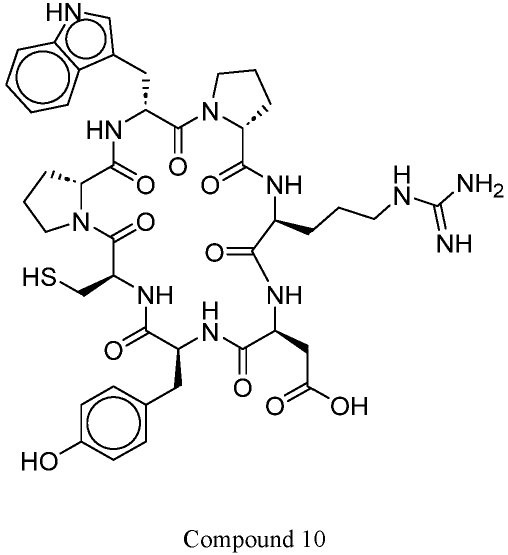 | Arg Asp Tyr Cys D-Pro D-Trp D- Pro (SEQ ID NO: 10) |
| 11 | 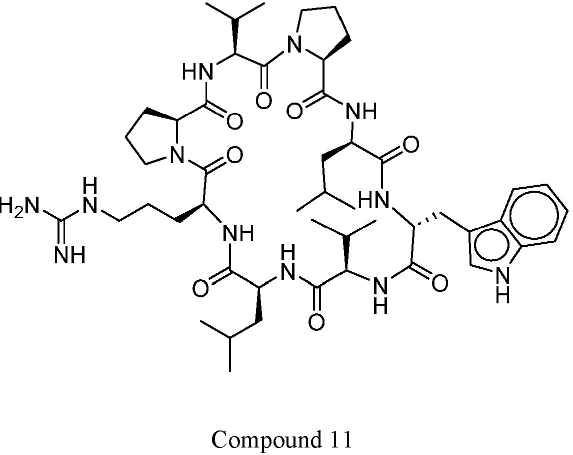 | Val Pro D-Leu D- Trp D-Val Leu Arg Pro (SEQ ID NO: 11) |

### Example 2. Evaluation of Cyclic Oligopeptides by Surface Plasmon Resonance (SPR)

A SPR assay was conducted to determine the binding affinity of the cyclic oligopeptides generated in Example 1 for IL-13 using a BIAcore 8K plus instrument (GE Healthcare). Human IL-13 with an Fc-tag was immobilized onto the surfaces of Series S Sensor CM5 (GE Healthcare) chips in the range of 5000 resonance units (RU). The binding kinetics were performed at 25°C with a flow rate of 30 µL/min. The cyclic oligopeptides were analyzed in running buffer comprising 20 mM PBS, 2.7 mM KCl, 37 mM NaCl, 0.05% Surfactant P20, pH 7.4, 1% DMSO, starting at 100 µM with gradient of ten two-fold dilutions using solvent correction mode. The affinity between IL-13 and the cyclic oligopeptides, as demonstrated by K_{D} values, was determined using the BIAcore 8K plus evaluation software.

The efficiency and effectiveness of SPR assay were confirmed using tralokinumab, a monoclonal antibody against IL-13, as a positive control. Figures 2C, 3C, 4C, 5C, 6C, 7C, 8C, 9C, 10C, 11C, and 12C provide SPR response curves for the cyclic oligopeptides generated in Example 1, wherein the x-axis represents time of reaction and y-axis represents altitude of response (Response Units). As summarized in Table 2, *infra,* the SPR results demonstrate that the exemplary cyclic oligopeptides of this disclosure bind to IL-13 with K_{D} values ranging from 16 to 622 µM.

**Table 2. Binding Affinity (K_{D}) of exemplary cyclic oligopeptides for IL-13.**

| Cyclic Oligopeptide | KD |
|---|---|
| Compound 1 | 16 µM |
| Compound 2 | 17.4 µM |
| Compound 3 | 22.8 µM |
| Compound 4 | 26.2 µM |
| Compound 5 | 47.1 µM |
| Compound 6 | 55.4 µM |
| Compound 7 | 85.1 µM |
| Compound 8 | 99.5µM |
| Compound 9 | 337 µM |
| Compound 10 | 368 µM |
| Compound 11 | 622 µM |
| Tralokinumab | 309 µM |

### Example 3. Skin Permeability of cyclic oligopeptides

Skin permeability of the cyclic oligopeptides generated in Example 1 was measured using Transdermal Diffusion Tester (TK-12D, Shanghai Kai Kai Industrial Co Ltd) and artificial skin (Start M, Merck). Three parallel experimental groups were set up for each sample. The skin permeability of cyclic oligopeptides was evaluated by the following steps:
1) 2.0 mg cyclic oligopeptide was weighed and dissolved in 100 µL DMSO. Three parallel experimental groups were set up for each cyclic oligopeptide. The cyclic oligopeptide-containing solution was then slowly added dropwise to 1900 µL 1×PBS solution and mixed evenly to prepare a PBS solution with a final concentration of 1.0 mg/mL cyclic polypeptide.
2) A magnetron was inserted into a Franz Cell diffusion cell, and an artificial skin was sandwiched in the middle of the diffusion cell with the smooth side of the artificial skin facing up. The diffusion cell was then fixed with clamps after assembly.
3) 7.75 ml 1xPBS solution were then added to the diffusion cell without generating bubbles in the diffusion cell. The sampling port of the diffusion cell was then sealed with parafilm to reduce evaporation.
4) The temperature of a Transdermal Diffusion Tester was then set to 32°C, and the magneton speed was set to 180 r/min.
5) The assembled diffusion cell was then put into the Transdermal Diffusion Tester and preheated for 5-10 mins.
6) 400 µL of 1.0 mg/mL cyclic oligopeptide solution prepared in step 1) was then added to the upper chamber of the diffusion cell, making it evenly dispersed on the surface of the artificial skin without air bubbles. The top port of the diffusion cell was then covered with parafilm to reduce evaporation.
7) Starting from the time of adding the cyclic oligopeptide solution, samples of about 0.5 mL of the receiving solution were taken at six time points, 1h, 2h, 4h, 8h, 12h and 24h, using a plastic dropper to take out the samples from the sampling port of the diffusion cell. 0.5 mL of 1×PBS solution was added into the diffusion cell to restore the initial volume of the receiving solution. When sampling, the diffusion cell was tilted appropriately to avoid the generation of bubbles. The samples were then numbered and store at -20°C.
8) A BCA Assay (Micro BCA Protein Assay Kit, Thermo Scientific) was conducted to measure the concentration of cyclic oligopeptide in samples obtained in step 7) (Actual Concentration of the cyclic oligopeptide at the particular time point). Three parallel groups were used. The weight of cyclic oligopeptide in each sample obtained in step 7) equals to Actual Concentration x 0.5 mL.
9) Permeation rate of each cyclic oligopeptide at each time point was calculated according to the following equation: $\begin{matrix}Permeation rate \left(%\right) = \frac{\begin{matrix}7.75 \times Actual Concentration + Σweight of cyclic oligopeptide in previous \\ samples\end{matrix}}{0.4} \\ \times 100 %\end{matrix}$
10) The relationship between the permeation rate of each cyclic oligopeptide and time was calculated based on the average results of the triplicate experimental groups (illustrated by FIG. 2D, FIG. 3D, FIG. 4D, FIG. 5D, FIG. 6D, FIG. 7D, FIG. 8D, FIG. 9D, FIG. 10D, FIG. 11D and FIG. 12D).

All the cyclic oligopeptides had more than 25% average permeation rates after 24 hours. As shown in Table 3, Compound 9 and Compound 10 had more than 60% permeation rates after 24 hours. Compound 1 and Compound 5 had permeation rates between 40% and 50% after 24 hours. Compound 2, Compound 3, Compound 4, Compound 6, Compound 7, and Compound 8 had permeation rates between 30% and 40% after 24 hours. Compound 11 had permeation rate of about 26% after 24 hours. As shown in FIG. 2D, FIG. 3D, FIG. 4D, FIG. 5D, FIG. 6D, FIG. 7D, FIG. 8D, FIG. 9D, FIG. 10D, FIG. 11D and FIG. 12D, from 1 hour to 24 hours, the permeation rates of the oligopeptides increased steadily. These data demonstrated the relative high skin permeation efficiency of the cyclic oligopeptides of this disclosure throughout a relative long period of time.

In addition, as shown in Table 3 and FIG. 2D, FIG. 3D, FIG. 4D, FIG. 5D, FIG. 6D, FIG. 7D, FIG. 8D, FIG. 9D, FIG. 10D, FIG. 11D and FIG. 12D, all the cyclic oligopeptides had more than 10% average permeation rates after 1 hours. Compound 5, Compound 7 and Compound 9 had more than 20% permeation rates after 1 hour. Compound 1, Compound 3, Compound 4, Compound 6 and Compound 10 had permeation rates between 15% and 20% after 1 hour. Compound 2, Compound 8, and Compound 11 had permeation rates between 10% and 15% after 24 hours. These data demonstrated the fast-acting quality of the oligopeptides of this disclosure when applied topically, indicated by the relative high percentage of skin permeation rate within the first hour.

**Table 3. Permeation rate of exemplary cyclic oligopeptides that bind to IL-13.**

| **Cyclic Oligopeptide** | **Average Permeation Rate After 1 Hour (%)** | **Average Permeation Rate After 24 Hours (%)** |
|---|---|---|
| Compound 1 | 18.54 | 42.06 |
| Compound 2 | 14.62 | 30.51 |
| Compound 3 | 19.61 | 36.59 |
| Compound 4 | 17.28 | 35.59 |
| Compound 5 | 22.46 | 48.73 |
| Compound 6 | 15.34 | 33.86 |
| Compound 7 | 25.42 | 38.84 |
| Compound 8 | 12.44 | 35.20 |
| Compound 9 | 23.40 | 60.63 |
| Compound 10 | 18.96 | 60.88 |
| Compound 11 | 11.77 | 25.75 |

The skin permeability coefficient (Kp)(cm/hr) values for the cyclic oligopeptides were also calculated with the following equation: $permeability coefficient \left(Kp\right) = \frac{Q}{ATΔC} ;$ wherein Q(mg) refers to the total amount of cyclic oligopeptide that pass through the artificial skin within a certain amount of time; A(cm²) refers to the internal cross-section area of the artificial skin in a diffusion cell; T(hr) refers to the total amount of time of cyclic oligopeptide permeation; and ΔC(mg/mL) refers to the concentration difference of the cyclic oligopeptide between the two sides of the artificial skin.

As shown in Table 4 and illustrated by FIG. 2E, FIG. 3E, FIG. 4E, FIG. 5E, FIG. 6E, FIG. 7E, FIG. 8E, FIG. 9E, FIG. 10E, FIG. 11E and FIG. 12E, all the cyclic oligopeptides had more than 2 x 10⁻⁵ average Kp values after 24 hours. Compound 1, Compound 4, Compound 8, Compound 9 and Compound 10 had average Kp values higher than 1 x 10⁻⁴ cm/hr after 24 hours. Compound 2, Compound 3, Compound 5, Compound 6, Compound 7 and Compound 11 had average Kp values between 1 x 10⁻⁵ cm/hr and 1 x 10⁻⁴ cm/hr after 24 hours. These data also demonstrated that the cyclic oligopeptides of this disclosure were able to penetrate skin throughout a relative long period of time.

In addition, as also shown in Table 4 and illustrated by FIG. 2E, FIG. 3E, FIG. 4E, FIG. 5E, FIG. 6E, FIG. 7E, FIG. 8E, FIG. 9E, FIG. 10E, FIG. 11E and FIG. 12E, all the cyclic oligopeptides had more than 2 x 10⁻² cm/hr average Kp values within the first hour, indicating that the cyclic oligopeptides of this disclosure were fast-acting after being topically applied to skin.

**Table 4. Average permeability coefficient (Kp) of exemplary cyclic oligopeptides that bind to IL-13.**

| **Cyclic Oligopeptide** | **Average Permeability Coefficient (Kp) After 1 hour (cm/hr)** | **Average Permeability Coefficient (Kp) After 24 hours (cm/hr)** |
|---|---|---|
| Compound 1 | 3.98 x 10⁻² | 1.19 x 10⁻⁴ |
| Compound 2 | 3.13 x 10⁻² | 8.74 x 10⁻⁵ |
| Compound 3 | 4.22 x 10⁻² | 2.55 x 10⁻⁵ |
| Compound 4 | 3.71 x 10⁻² | 1.08 x 10⁻⁴ |
| Compound 5 | 4.84 x 10⁻² | 6.64 x 10⁻⁵ |
| Compound 6 | 3.29 x 10⁻² | 7.14 x 10⁻⁵ |
| Compound 7 | 5.48 x 10⁻² | 3.31 x 10⁻⁵ |
| Compound 8 | 2.66 x 10⁻² | 1.98 x 10⁻⁴ |
| Compound 9 | 5.04 x 10⁻² | 4.63 x 10⁻⁴ |
| Compound 10 | 4.21 x 10⁻² | 2.33 x 10⁻⁴ |
| Compound 11 | 2.60 x 10⁻² | 9.89 x 10⁻⁵ |

## Claims

1. A cyclic oligopeptide that binds to IL-13, wherein the cyclic oligopeptide comprises an amino acid sequence selected from the group consisting of:
(a) D-ARG D-PHE D-VAL TYR GLU PRO (SEQ ID NO:1);
(b) ARG THR D-VAL GLU D-PHE D-PRO (SEQ ID NO:2);
(c) GLU D-THR D-VAL TRP D-PRO D-PRO (SEQ ID NO:3);
(d) ARG GLU D-VAL TRP D-PRO D-PRO (SEQ ID NO:4);
(e) TRP VAL ARG GLU D-PRO D-PRO (SEQ ID NO:5);
(f) TYR ARG GLU D-THR D-VAL D-PRO (SEQ ID NO:6);
(g) THR ARG D-PHE PRO D-LEU D-PRO (SEQ ID NO:7);
(h) D-LEU ARG GLU PRO TRP MET PRO (SEQ ID NO:8);
(i) D-ARG LEU D-TRP TRP D-THR GLU PRO (SEQ ID NO:9);
(j) ARG ASP TYR CYS D-PRO D-TRP D-PRO (SEQ ID NO:10); and
(k) VAL PRO D-LEU D-TRP D-VAL LEU ARG PRO (SEQ ID NO:11).

2. The cyclic oligopeptide of claim 1, wherein the cyclic oligopeptide has the structure of any one of Compounds 1-11 or pharmaceutically acceptable salts thereof, wherein
Compound 1 has the structure of: 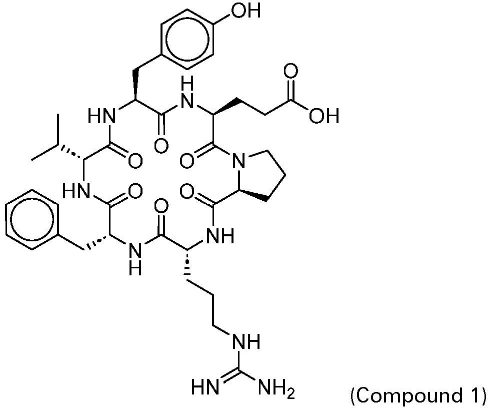 or a pharmaceutically acceptable salt thereof;
Compound 2 has the structure of: 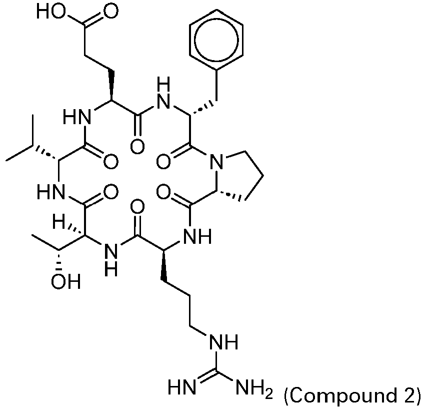 or a pharmaceutically acceptable salt thereof;
Compound 3 has the structure of: 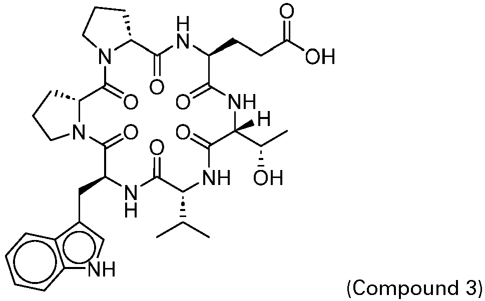 or a pharmaceutically acceptable salt thereof;
Compound 4 has the structure of: 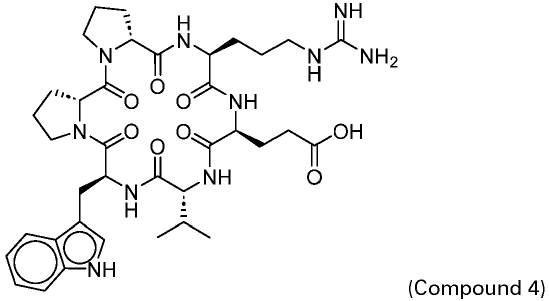 or a pharmaceutically acceptable salt thereof;
Compound 5 has the structure of: 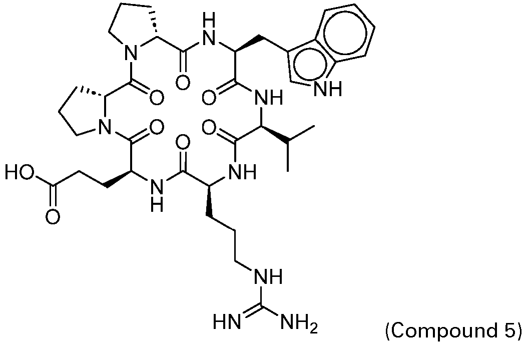 or a pharmaceutically acceptable salt thereof;
Compound 6 has the structure of: 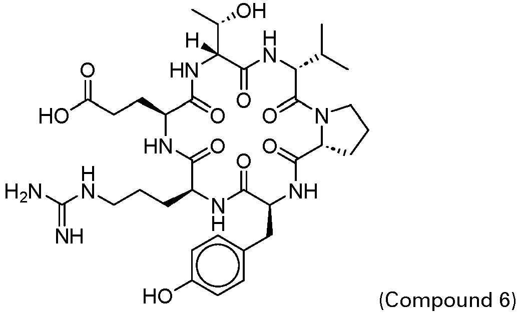 or a pharmaceutically acceptable salt thereof;
Compound 7 has the structure of: 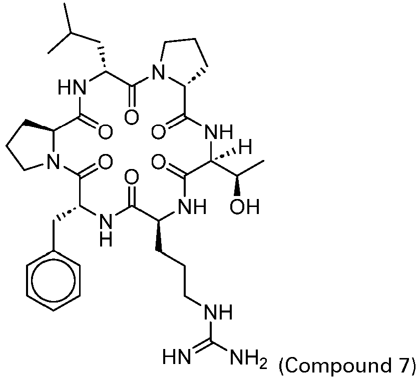 or a pharmaceutically acceptable salt thereof;
Compound 8 has the structure of: 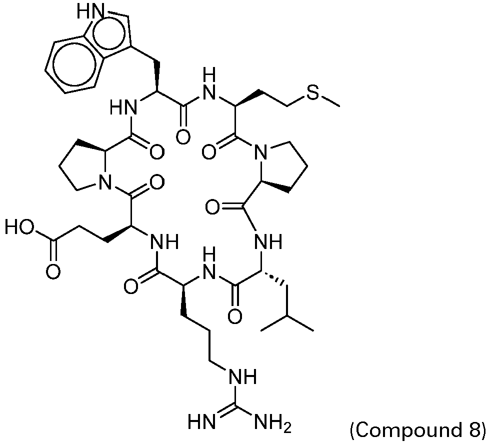 or a pharmaceutically acceptable salt thereof;
Compound 9 has the structure of: 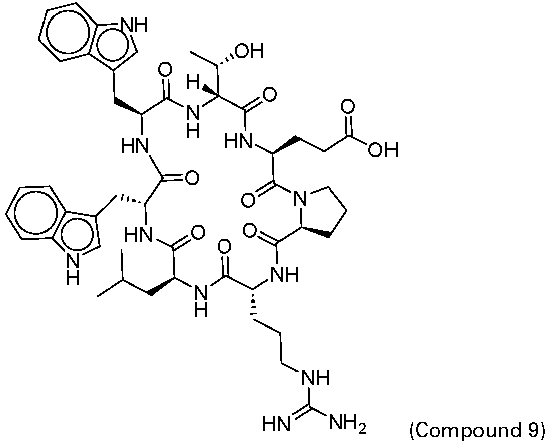 or a pharmaceutically acceptable salt thereof;
Compound 10 has the structure of: 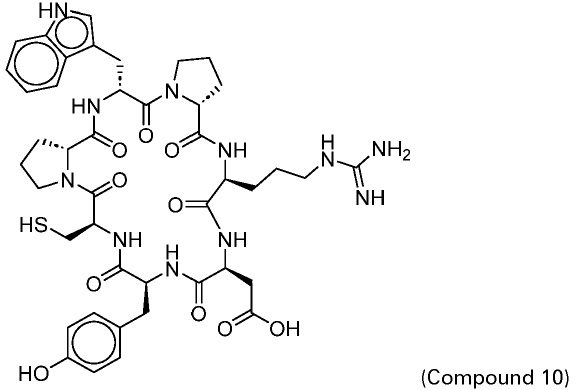 or a pharmaceutically acceptable salt thereof; and
Compound 11_has the structure of: 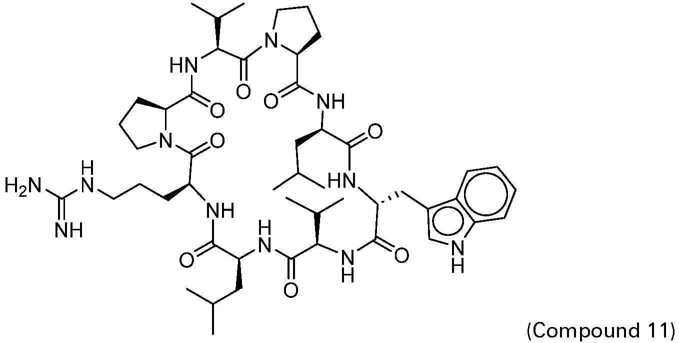 or a pharmaceutically acceptable salt thereof.

3. The cyclic oligopeptide of claim 1 or 2, wherein the cyclic oligopeptide has the structure of any one of Compounds 1-11, wherein
Compound 1 has the structure of: 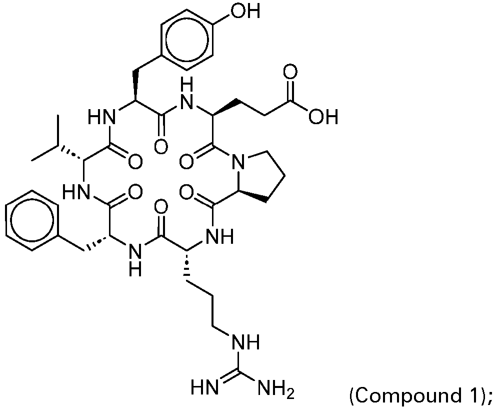
Compound 2 has the structure of: 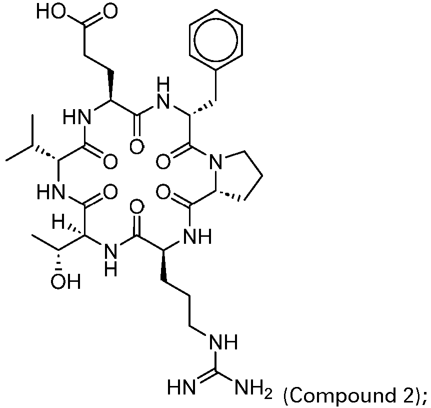
Compound 3 has the structure of: 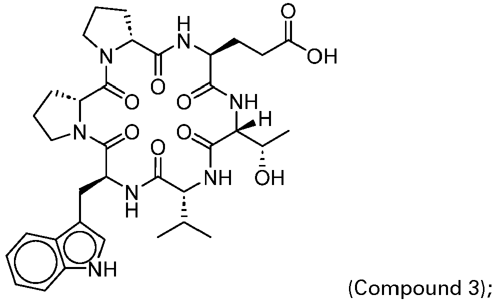
Compound 4 has the structure of: 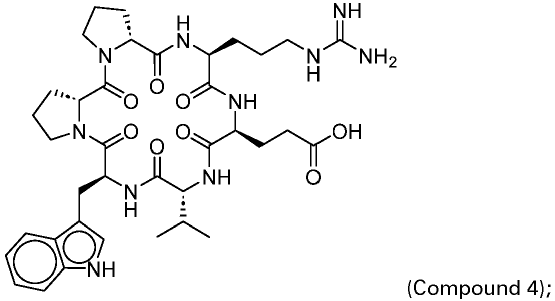
Compound 5 has the structure of: 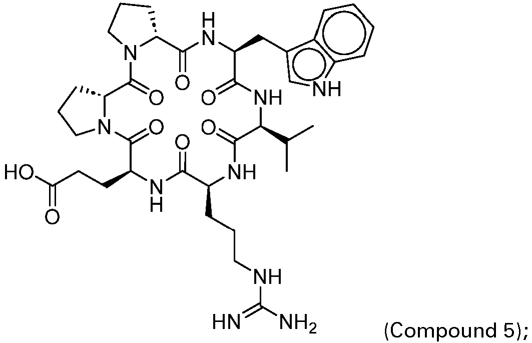
Compound 6 has the structure of: 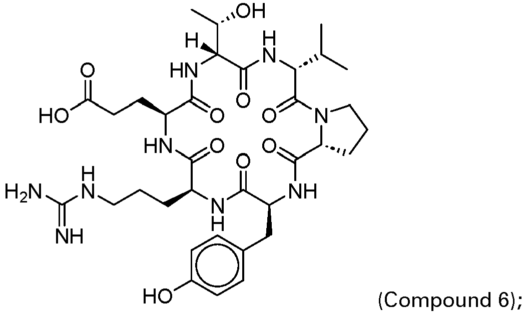
Compound 7 has the structure of: 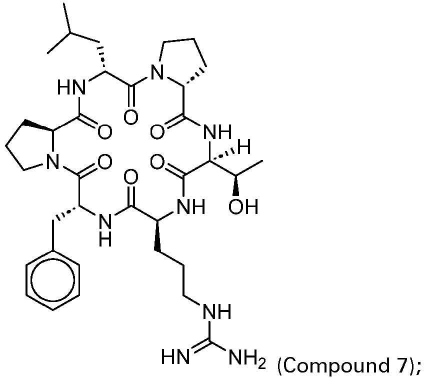
Compound 8 has the structure of: 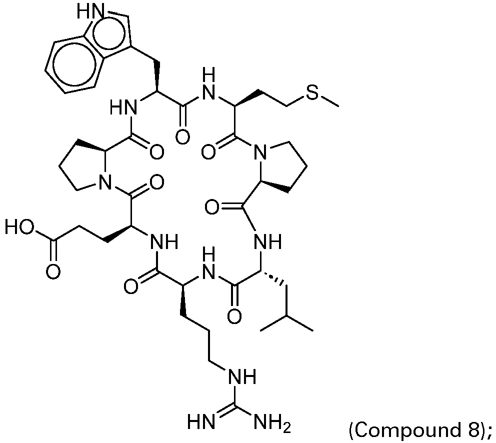
Compound 9 has the structure of: 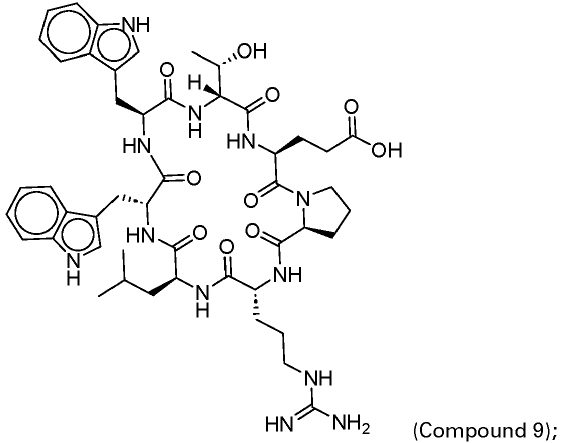
Compound 10 has the structure of: 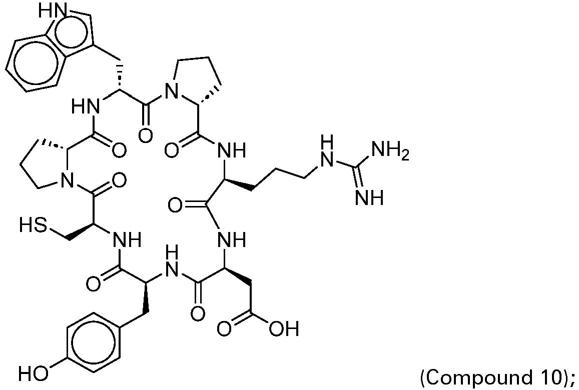 and
Compound 11 has the structure of:

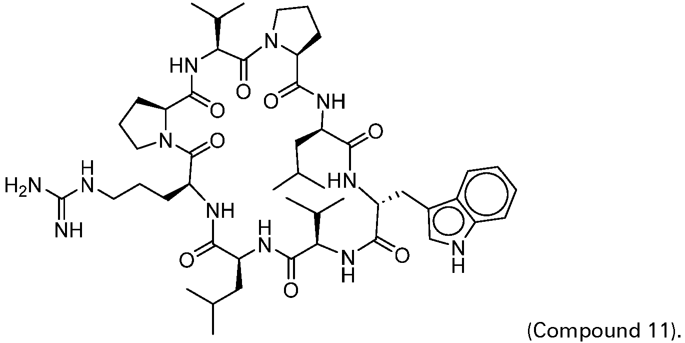

4. A pharmaceutical or cosmetic formulation comprising a cyclic oligopeptide of any one of claims 1-3 and a pharmaceutically or cosmetically acceptable carrier.

5. The cyclic oligopeptide of any one of claims 1-3, or the pharmaceutical or cosmetic formulation of claim 4, for use in a method_of treating or preventing an IL-13-associated skin disorder or condition, wherein the IL-13-associated skin disorder or condition is an inflammatory, an allergic, or an autoimmune disorder or condition, wherein, optionally, the IL-13-associated skin disorder or condition is selected from the group consisting of: atopic dermatitis, allergic contact dermatitis, urticaria, eczema, chronic hand eczema, bullous diseases (bullous pemphigoid), alopecia areata, prurigo and molluscum contagiosum, in a subject in need thereof.

6. The cyclic oligopeptide for use of claim 5, wherein the cyclic oligopeptide or the combination thereof is formulated to be administered topically, transdermally, subcutaneously, or intravenously.

7. A method of producing the cyclic oligopeptide according to any one of claims 1-3, the method comprising the steps of:
(1) contacting FMOC (9-fluorenylmethyloxycarbonyl)-D-Pro-2cl-resin or FMOC-Pro-2cl-resin with a first amino acid residue under conditions suitable to form a peptide bond between the first amino acid residue and the resin-bound D-Pro or Pro to form a resin-bound oligopeptide;
(2) washing the resin-bound oligopeptide to remove any unbound amino acid residue;
(3) contacting the resin-bound oligopeptide with an additional amino acid residue under conditions suitable to form a peptide bond between the additional amino acid residue and the resin-bound oligopeptide;
(4) washing the resin-bound oligopeptide to remove any unbound amino acid residue;
(5) removing the resin from the oligopeptide; and
(6) cyclizing the oligopeptide.

8. The method of claim 7, wherein:
a) the conditions suitable to form a peptide bond in steps (1) and (3) include the addition of O-(Benzotriazol-1-yl)-N, N, N', N'-tetramethyluronium tetrafluoroborate (TBTU);
b) the conditions suitable to form a peptide bond in steps (1) and (3) further include the addition of N,N-Dimethylformamide (DMF) and N,N-Diisopropylethylamine (DIEA);
c) steps (3) and (4) are repeated between 1 and 20 times before the resin is removed from the oligopeptide;
d) the cyclizing step comprises the carboxyl group of the D-Pro or Pro residue forming a covalent bond with one or more amino groups of the linear oligopeptide; and
e) the cyclizing step comprises the addition of DMF, PyBOP (Benzotriazol-1-yl-oxytripyrrolidinophosphonium hexafluorophosphate), HOBT (1-Hydroxybenzotriazole), and DIEA to the linear oligopeptide and incubation of the mixture thereof at room temperature.

## Patentansprüche

1. Ein zyklisches Oligopeptid, das IL-13 bindet, wobei das zyklische Oligopeptid eine Aminosäuresequenz umfasst, die aus der Gruppe ausgewählt ist, bestehend aus:
(a) D-ARG D-PHE D-VAL TYR GLU PRO (SEQ ID NO:1);
(b) ARG THR D-VAL GLU D-PHE D-PRO (SEQ ID NO:2);
(c) GLU D-THR D-VAL TRP D-PRO D-PRO (SEQ ID NO:3);
(d) ARG GLU D-VAL TRP D-PRO D-PRO (SEQ ID NO:4);
(e) TRP VAL ARG GLU D-PRO D-PRO (SEQ ID NO:5);
(f) TYR ARG GLU D-THR D-VAL D-PRO (SEQ ID NO:6);
(g) THR ARG D-PHE PRO D-LEU D-PRO (SEQ ID NO:7);
(h) D-LEU ARG GLU PRO TRP MET PRO (SEQ ID NO:8);
(i) D-ARG LEU D-TRP TRP D-THR GLU PRO (SEQ ID NO:9);
(j) ARG ASP TYR CYS D-PRO D-TRP D-PRO (SEQ ID NO:10); und
(k) VAL PRO D-LEU D-TRP D-VAL LEU ARG PRO (SEQ ID NO:11).

2. Das zyklische Oligopeptid nach Anspruch 1, wobei das zyklische Oligopeptid die Struktur einer der Verbindungen 1-11 oder eines pharmazeutisch akzeptablen Salzes davon aufweist, wobei
Verbindung 1 die Struktur hat: 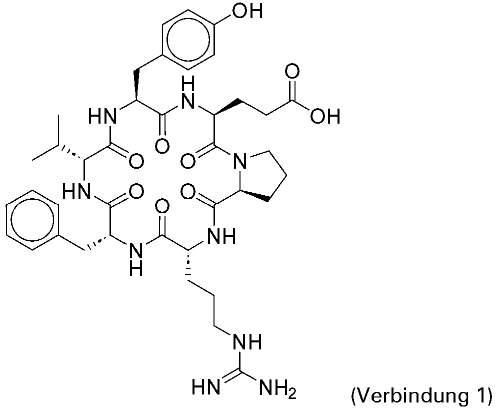 oder ein pharmazeutisch akzeptables Salz davon;
Verbindung 2 die Struktur hat: 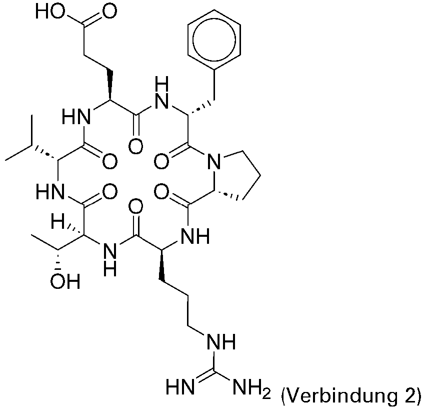 oder ein pharmazeutisch akzeptables Salz davon;
Verbindung 3 die Struktur hat: 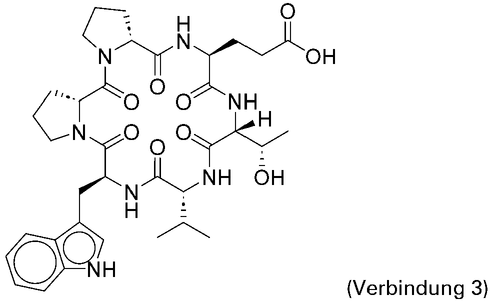 oder ein pharmazeutisch akzeptables Salz davon;
Verbindung 4 die Struktur hat: 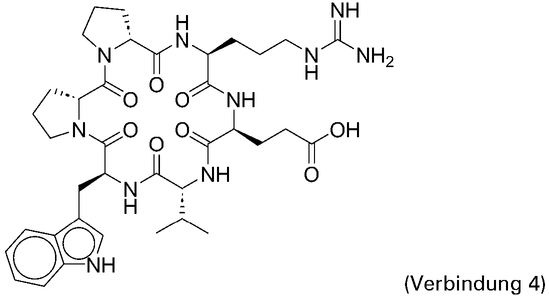 oder ein pharmazeutisch akzeptables Salz davon;
Verbindung 5 die Struktur hat: 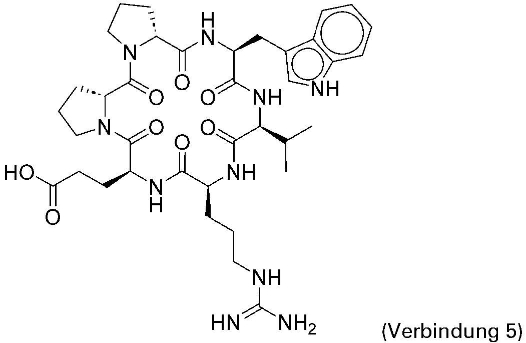 oder ein pharmazeutisch akzeptables Salz davon;
Verbindung 6 die Struktur hat: 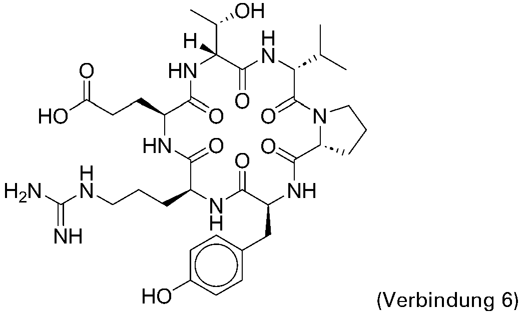 oder ein pharmazeutisch akzeptables Salz davon;
Verbindung 7 die Struktur hat: 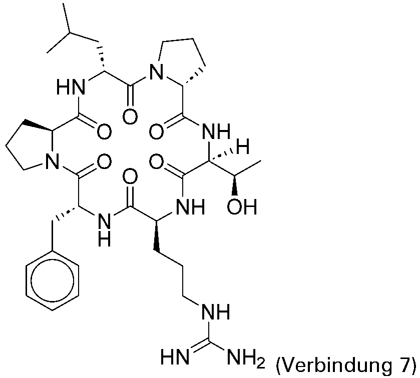 oder ein pharmazeutisch akzeptables Salz davon;
Verbindung 8 die Struktur hat: 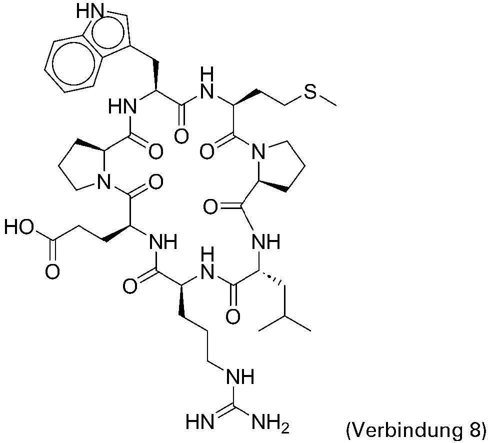 oder ein pharmazeutisch akzeptables Salz davon;
Verbindung 9 die Struktur hat: 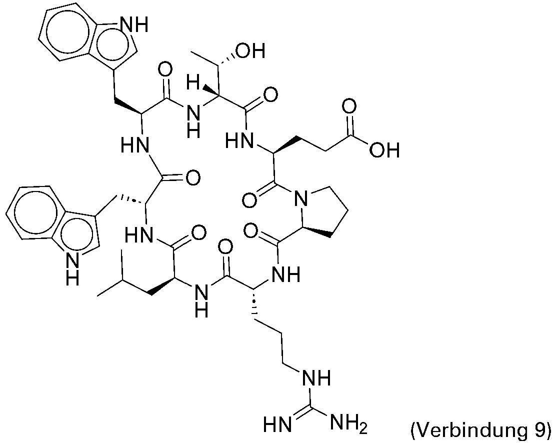 oder ein pharmazeutisch akzeptables Salz davon;
Verbindung 10 die Struktur hat: 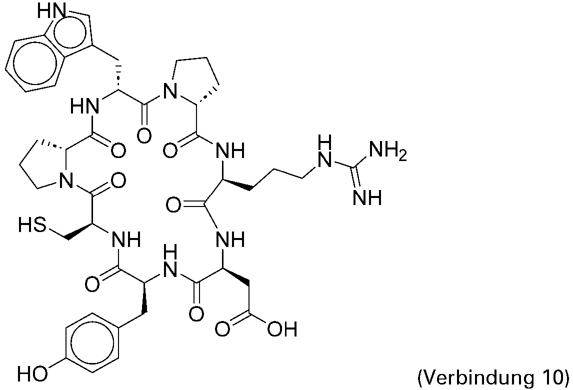 oder ein pharmazeutisch akzeptables Salz davon; und
Verbindung 11 die Struktur hat: 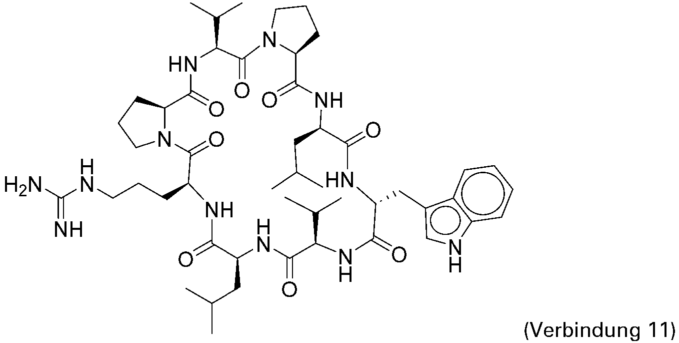 oder ein pharmazeutisch akzeptables Salz davon.

3. Das zyklische Oligopeptid nach Anspruch 1 oder 2, wobei das zyklische Oligopeptid die Struktur einer der Verbindungen 1-11 aufweist, wobei
Verbindung 1 die Struktur hat: 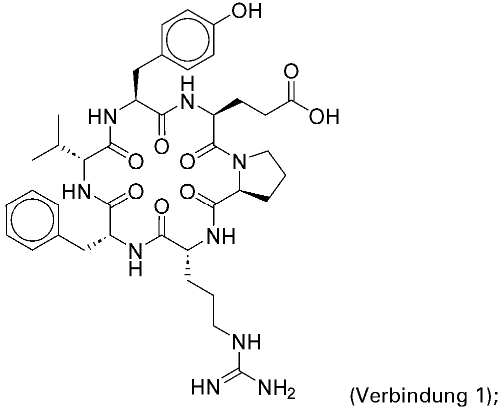
Verbindung 2 die Struktur hat: 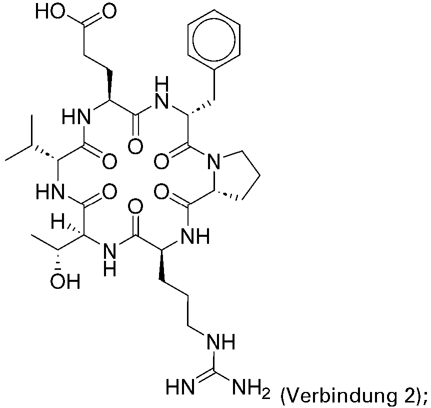
Verbindung 3 die Struktur hat: 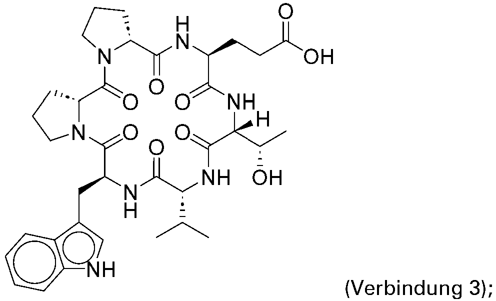
Verbindung 4 die Struktur hat: 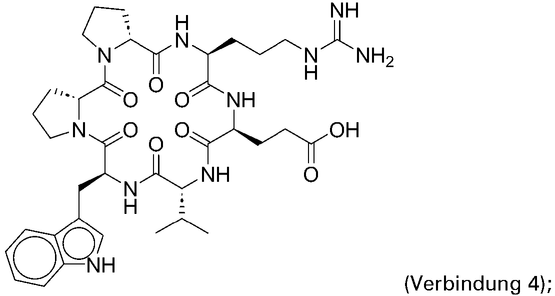
Verbindung 5 die Struktur hat: 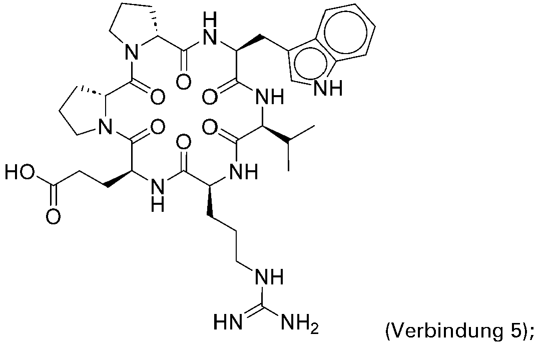
Verbindung 6 die Struktur hat: 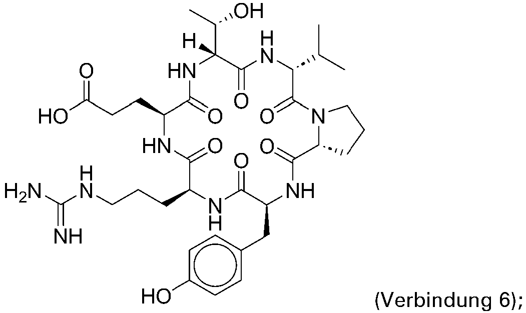
Verbindung 7 die Struktur hat: 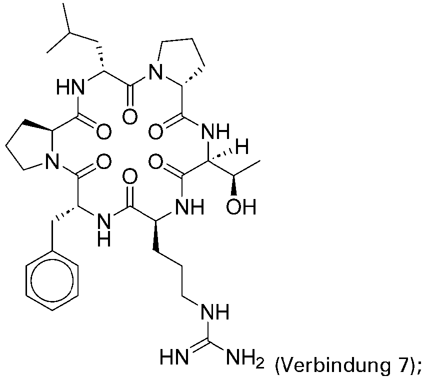
Verbindung 8 die Struktur hat: 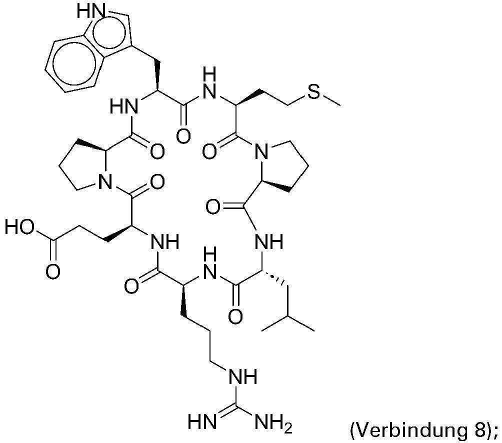
Verbindung 9 die Struktur hat: 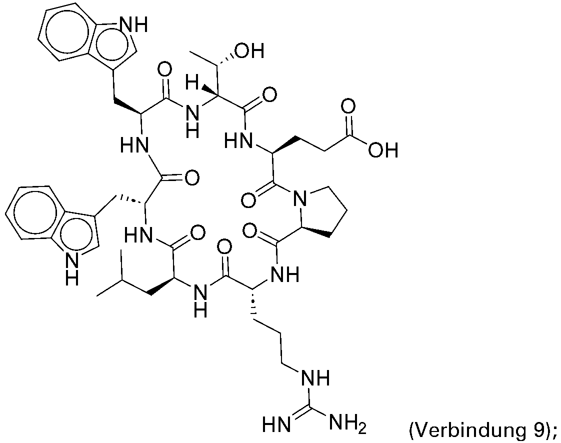
Verbindung 10 die Struktur hat: 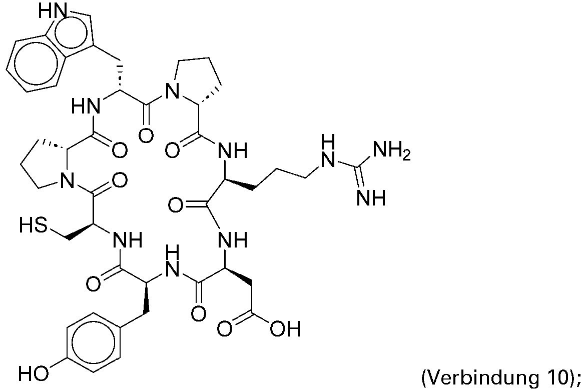 und
Verbindung 11 die Struktur hat:

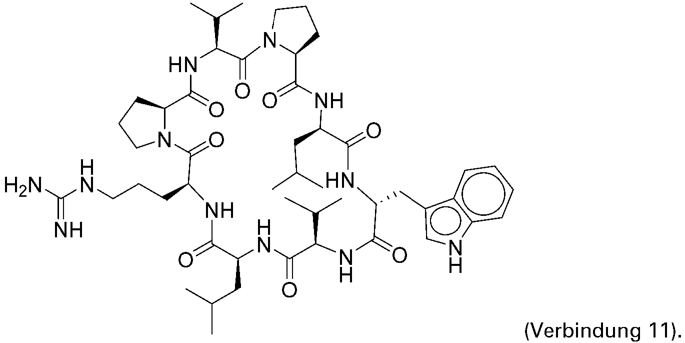

4. Pharmazeutische oder kosmetische Formulierung, umfassend ein zyklisches Oligopeptid nach einem der Ansprüche 1 bis 3 und einen pharmazeutisch oder kosmetisch akzeptablen Träger.

5. Das zyklische Oligopeptid nach einem der Ansprüche 1 bis 3 oder die pharmazeutische oder kosmetische Formulierung nach Anspruch 4 zur Verwendung in einem Verfahren zur Behandlung oder Vorbeugung einer IL-13-assoziierten Hauterkrankung oder -beschwerde, wobei die IL-13-assoziierte Hauterkrankung oder -beschwerde eine entzündliche, eine allergische oder eine autoimmune Erkrankung oder Beschwerde ist, wobei optional die IL-13-assoziierte Hauterkrankung oder -beschwerden aus der Gruppe ausgewählt ist, bestehend aus: atopische Dermatitis, allergische Kontaktdermatitis, Urtikaria, Ekzeme, chronisches Handekzem, bullösen Erkrankungen (bullöses Pemphigoid), Alopecia areata, Prurigo und Molluscum contagiosum, bei einer Person, der dies benötigt.

6. Das zyklische Oligopeptid zur Verwendung nach Anspruch 5, wobei das zyklische Oligopeptid oder dessen Kombination so formuliert ist, dass es topisch, transdermal, subkutan oder intravenös verabreicht werden kann.

7. Verfahren zur Herstellung des zyklischen Oligopeptids nach einem der Ansprüche 1 bis 3, wobei das Verfahren die folgenden Schritte umfasst:
(1) In Kontakt bringen von FMOC (9-Fluorenylmethyloxycarbonyl)-D-Pro-2cl-Harz oder FMOC-Pro-2cl-Harz mit einem ersten Aminosäurerest unter Bedingungen, die geeignet sind, eine Peptidbindung zwischen dem ersten Aminosäurerest und dem harzgebundenen D-Pro oder Pro zu bilden, um ein harzgebundenes Oligopeptid zu bilden;
(2) Waschen des harzgebundenen Oligopeptids, um etwaige ungebundene Aminosäurereste zu entfernen;
(3) In Kontakt bringen des harzgebundenen Oligopeptids mit einem weiteren Aminosäurerest unter Bedingungen, die geeignet sind, eine Peptidbindung zwischen dem weiteren Aminosäurerest und dem harzgebundenen Oligopeptid zu bilden;
(4) Waschen des harzgebundenen Oligopeptids, um etwaige ungebundene Aminosäurereste zu entfernen;
(5) Entfernen des Harzes von dem Oligopeptid; und
(6) Zyklisieren des Oligopeptids.

8. Verfahren nach Anspruch 7, wobei:
a) die zur Bildung einer Peptidbindung geeigneten Bedingungen in den Schritten (1) und (3) die Zugabe von O-(Benzotriazol-1-yl)-N,N,N',N'-tetramethyluroniumtetrafluorborat (TBTU) umfassen;
b) die zur Bildung einer Peptidbindung in den Schritten (1) und (3) geeigneten Bedingungen ferner die Zugabe von N,N-Dimethylformamid (DMF) und N,N-Diisopropylethylamin (DIEA) umfassen;
c) die Schritte (3) und (4) zwischen 1- und 20-mal wiederholt werden, bevor das Harz von dem Oligopeptid entfernt wird;
d) der Zyklisierungsschritt umfasst, dass die Carboxylgruppe des D-Pro- oder Pro-Rests eine kovalente Bindung mit einer oder mehreren Aminogruppen des linearen Oligopeptids eingeht; und
e) der Zyklisierungsschritt die Zugabe von DMF, PyBOP (Benzotriazol-1-yl-oxytripyrrolidinophosphoniumhexafluorophosphat), HOBT (1-Hydroxybenzotriazol) und DIEA zu dem linearen Oligopeptid und die Inkubation der Mischung davon bei Raumtemperatur umfasst.

## Revendications

1. Oligopeptide cyclique qui se lie à l'IL-13, dans lequel l'oligopeptide cyclique comprend une séquence d'acides aminés choisie dans le groupe constitué de :
(a) D-ARG D-PHE D-VAL TYR GLU PRO (SEQ ID NO: 1) ;
(b) ARG THR D-VAL GLU D-PHE D-PRO (SEQ ID NO : 2) ;
(c) GLU D-THR D-VAL TRP D-PRO D-PRO (SEQ ID NO : 3) ;
(d) ARG GLU D-VAL TRP D-PRO D-PRO (SEQ ID NO : 4) ;
(e) TRP VAL ARG GLU D-PRO D-PRO (SEQ ID NO : 5) ;
(f) TYR ARG GLU D-THR D-VAL D-PRO (SEQ ID NO: 6) ;
(g) THR ARG D-PHE PRO D-LEU D-PRO (SEQ ID NO : 7) ;
(h) D-LEU ARG GLU PRO TRP MET PRO (SEQ ID NO : 8) ;
(i) D-ARG LEU D-TRP TRP D-THR GLU PRO (SEQ ID NO : 9) ;
(j) ARG ASP TYR CYS D-PRO D-TRP D-PRO (SEQ ID NO : 10) ; et
(k) VAL PRO D-LEU D-TRP D-VAL LEU ARG PRO (SEQ ID NO : 11).

2. Oligopeptide cyclique selon la revendication 1, dans lequel l'oligopeptide cyclique a la structure de l'un quelconque des composés 1 à 11 ou de sels pharmaceutiquement acceptables de ceux-ci, dans lequel
le composé 1 a la structure suivante : 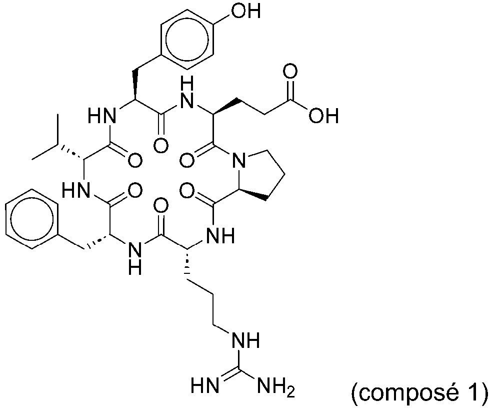 ou un sel pharmaceutiquement acceptable de celui-ci ;
le composé 2 a la structure suivante : 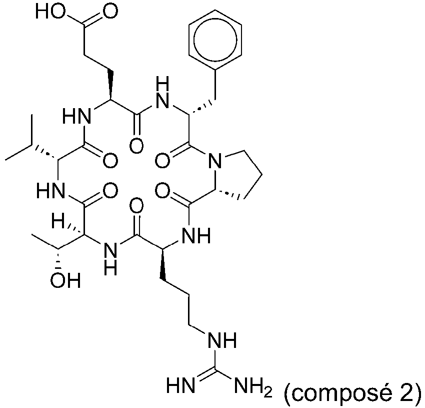 ou un sel pharmaceutiquement acceptable de celui-ci ;
le composé 3 a la structure suivante : 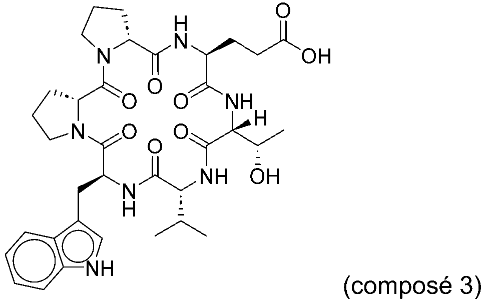 ou un sel pharmaceutiquement acceptable de celui-ci ;
le composé 4 a la structure suivante : 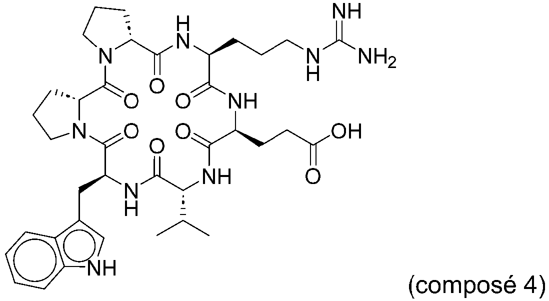 ou un sel pharmaceutiquement acceptable de celui-ci ;
le composé 5 a la structure suivante : 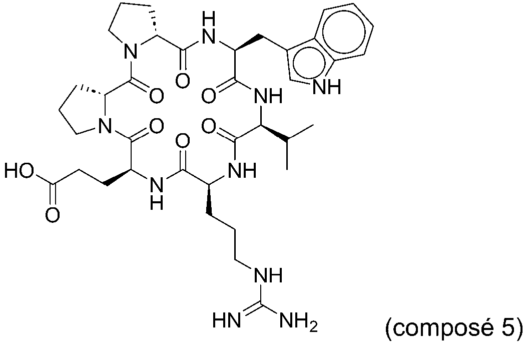 ou un sel pharmaceutiquement acceptable de celui-ci ;
le composé 6 a la structure suivante : 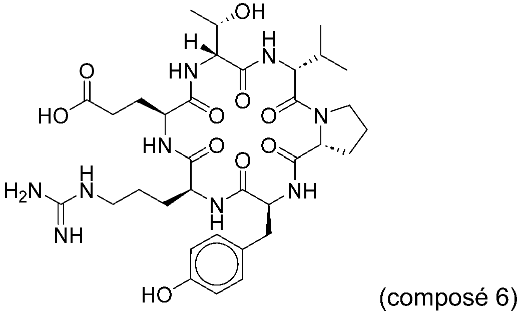 ou un sel pharmaceutiquement acceptable de celui-ci ;
le composé 7 a la structure suivante : 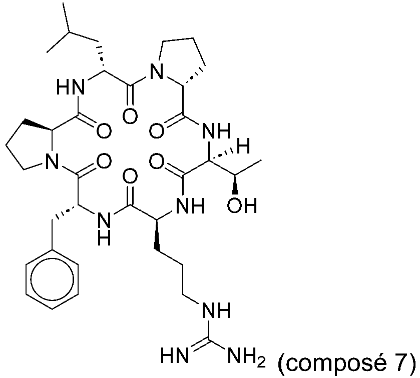 ou un sel pharmaceutiquement acceptable de celui-ci ;
le composé 8 a la structure suivante : 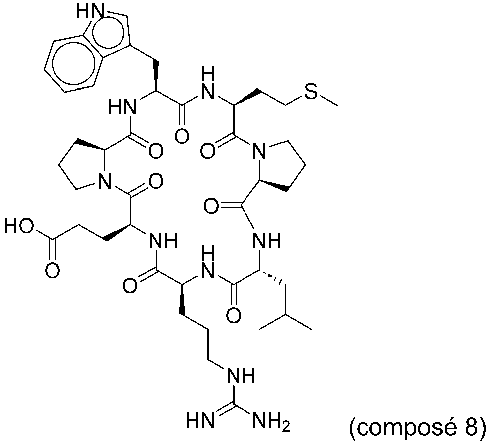 ou un sel pharmaceutiquement acceptable de celui-ci ;
le composé 9 a la structure suivante : 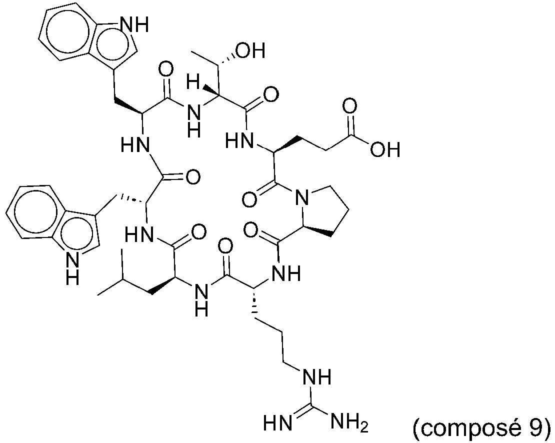 ou un sel pharmaceutiquement acceptable de celui-ci ;
le composé 10 a la structure suivante : 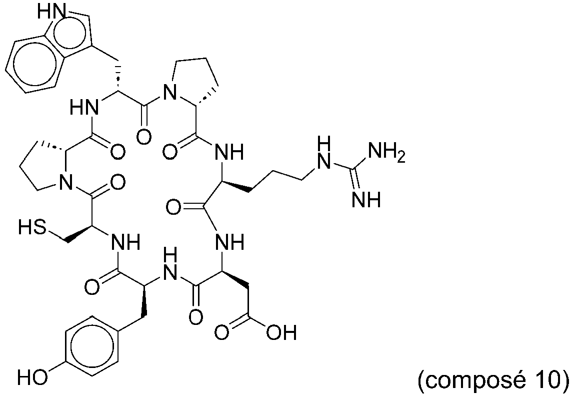 ou un sel pharmaceutiquement acceptable de celui-ci ; et
le composé 11 a la structure suivante : 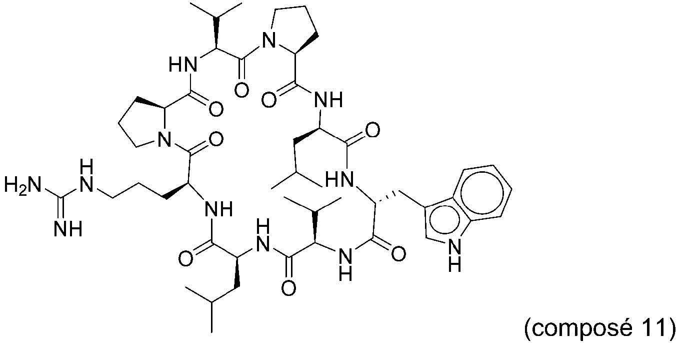 ou un sel pharmaceutiquement acceptable de celui-ci.

3. Oligopeptide cyclique selon la revendication 1 ou 2, dans lequel l'oligopeptide cyclique a la structure de l'un quelconque des composés 1 à 11, dans lequel
le composé 1 a la structure suivante : 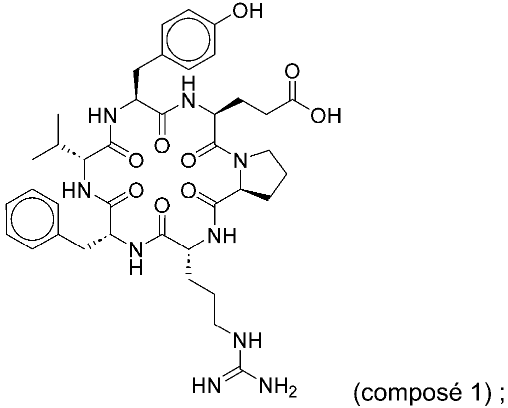
le composé 2 a la structure suivante : 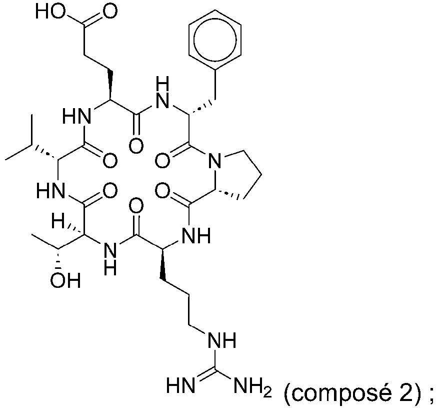
le composé 3 a la structure suivante : 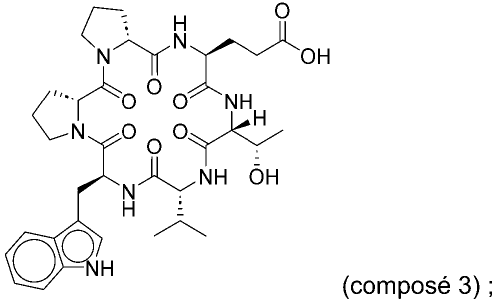
le composé 4 a la structure suivante : 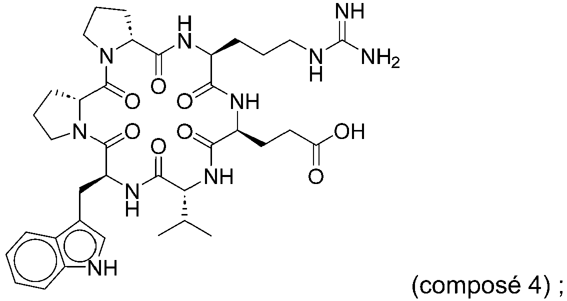
le composé 5 a la structure suivante : 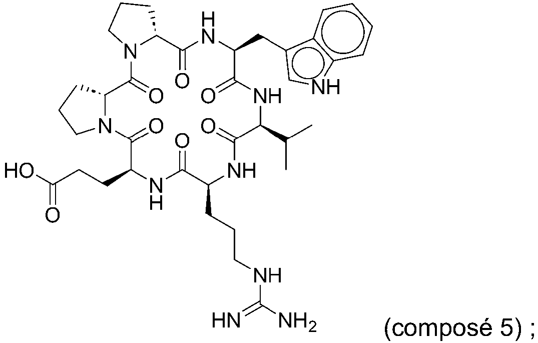
le composé 6 a la structure suivante : 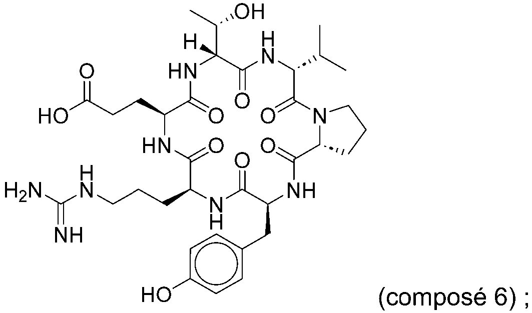
le composé 7 a la structure suivante : 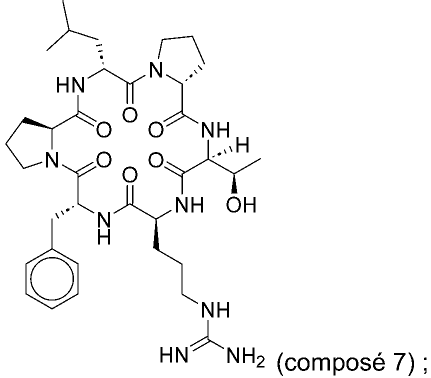
le composé 8 a la structure suivante : 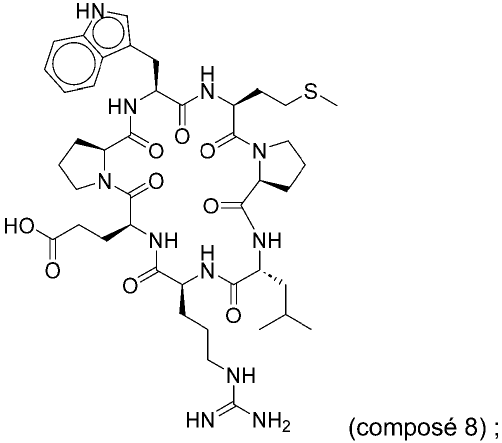
le composé 9 a la structure suivante : 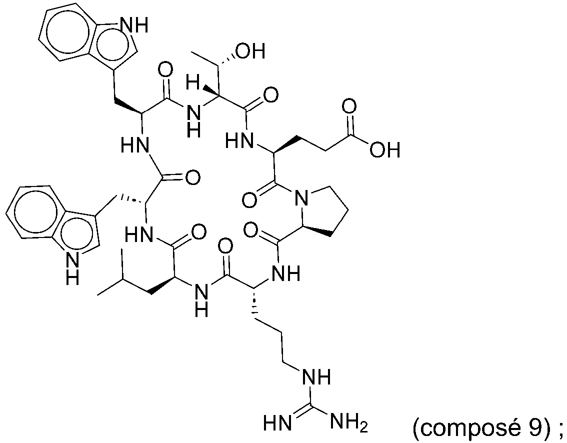
le composé 10 a la structure suivante : 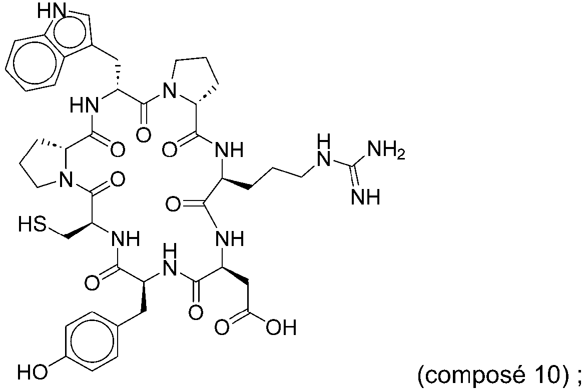 et
le composé 11 a la structure suivante :

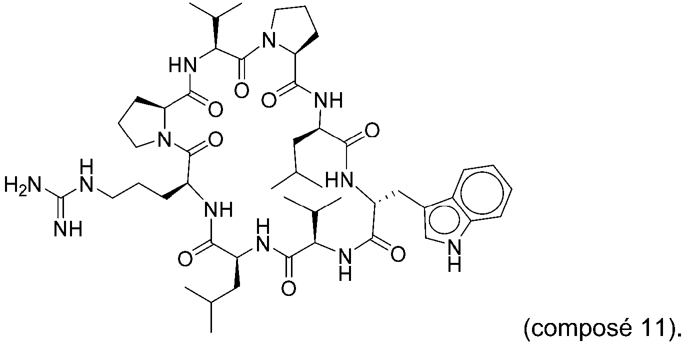

4. Formulation pharmaceutique ou cosmétique comprenant un oligopeptide cyclique selon l'une quelconque des revendications 1 à 3 et un support pharmaceutiquement ou cosmétiquement acceptable.

5. Oligopeptide cyclique selon l'une quelconque des revendications 1 à 3, ou formulation pharmaceutique ou cosmétique selon la revendication 4, destiné à être utilisé dans un procédé de traitement ou de prévention d'un trouble ou d'une affection cutané associé à l'IL-13, dans lequel le trouble ou l'affection cutané associé à l'IL-13 est un trouble ou une affection inflammatoire, allergique ou auto-immun, dans lequel, éventuellement, le trouble ou l'affection cutané associé à l'IL-13 est choisi dans le groupe constitué de : dermatite atopique, dermatite de contact allergique, urticaire, eczéma, eczéma chronique des mains, maladies bulleuses (pemphigoïde bulleuse), alopécie areata, prurigo et molluscum contagiosum, chez un sujet qui en a besoin.

6. Oligopeptide cyclique destiné à être utilisé selon la revendication 5, dans lequel l'oligopeptide cyclique ou la combinaison de celui-ci est formulé pour être administré par voie topique, transdermique, sous-cutanée ou intraveineuse.

7. Procédé pour la production de l'oligopeptide cyclique selon l'une quelconque des revendications 1 à 3, le procédé comprenant les étapes consistant à :
(1) mettre en contact une résine FMOC (9-fluorénylméthyloxycarbonyl)-D-Pro-2cl ou une résine FMOC-Pro-2cl avec un premier résidu d'acide aminé dans des conditions appropriées pour former une liaison peptidique entre le premier résidu d'acide aminé et le D-Pro ou Pro lié à la résine pour former un oligopeptide lié à la résine ;
(2) laver l'oligopeptide lié à la résine pour éliminer tout résidu d'acide aminé non lié ;
(3) mettre en contact l'oligopeptide lié à la résine avec un résidu d'acide aminé supplémentaire dans des conditions appropriées pour former une liaison peptidique entre le résidu d'acide aminé supplémentaire et l'oligopeptide lié à la résine ;
(4) laver l'oligopeptide lié à la résine pour éliminer tout résidu d'acide aminé non lié ;
(5) éliminer la résine de l'oligopeptide ; et
(6) cycliser l'oligopeptide.

8. Procédé selon la revendication 7, dans lequel :
a) les conditions appropriées pour former une liaison peptidique aux étapes (1) et (3) comportent l'ajout de O-(benzotriazol-1-yl)-N, N, N', N'-tétraméthyluronium tétrafluoroborate (TBTU) ;
b) les conditions appropriées pour former une liaison peptidique aux étapes (1) et (3) comportent en outre l'ajout de N,N-diméthylformamide (DMF) et de N,N-diisopropyléthylamine (DIEA) ;
c) les étapes (3) et (4) sont répétées entre 1 et 20 fois avant que la résine ne soit éliminée de l'oligopeptide ;
d) l'étape de cyclisation comprend le groupe carboxyle du résidu D-Pro ou Pro formant une liaison covalente avec un ou plusieurs groupes amino de l'oligopeptide linéaire ; et
e) l'étape de cyclisation comprend l'ajout de DMF, de PyBOP (hexafluorophosphate de benzotriazol-1-yl-oxytripyrrolidinophosphonium), de HOBT (1-hydroxybenzotriazole) et de DIEA à l'oligopeptide linéaire et l'incubation du mélange de ceux-ci à température ambiante.
